# EUROPEAN PATENT APPLICATION

(11) **EP 4 725 501 A1**
(43) Date of publication of application: **15.04.2026**
(21) Application number: 24818731.2
(22) Date of filing: 06.06.2024
(51) Int. Cl.: A61K 39/395, A61P 7/06, C07K 16/40, A61K 45/06, C12N 15/13, C07K 16/24, C07K 16/28

(54) **ANTIBODY SPECIFICALLY BINDING TO MASP3, AND MULTI-SPECIFIC ANTIBODY SPECIFICALLY BINDING TO MASP3 AND MASP2**

(30) Priority: 09.06.2023 WO PCT/CN2023/099286
(71) Applicant: Staidson (Beijing) Biopharmaceuticals Co., Ltd., Beijing 100176 (CN)
(72) Inventor: FU, Wenyan, Beijing 100176 (CN); ZHANG, Chao, Beijing 100176 (CN); LI, Zhong, Beijing 100176 (CN); FAN, Jundie, Beijing 100176 (CN)
(74) Representative: HGF
(86) International application number: PCT/CN2024/097754
(87) International publication number: WO 2024/251199

(57) **Abstract**

The present invention relates to the field of biomedicine, and specifically relates to an antibody that specifically recognizes MASP3 and the use thereof. The antibody has a strong affinity to human MASP3 and has an alternative pathway inhibitory activity. In addition, the present invention also relates to a multi-specific antibody that specifically binds to MASP3 and MASP2, and a pharmaceutical composition comprising the antibody specifically binding to MASP3 and an antibody specifically binding to MASP2 and/or a multi-specific antibody. The above-described antibody or composition is used for preparing a drug for treating autoimmune diseases, transplantation-related diseases, inflammatory diseases, hematological diseases, coagulation diseases, angiogenesis-dependent diseases and/or viral infectious diseases.

## Description

### CROSS-REFERENCE TO RELATED APPLICATION

This application claims priority to the PCT application NO. PCT/CN2023/099286, filed on 2023.06.09, entitled "ANTIBODIES SPECIFICALLY BINDING TO MASP3 AND MULTI-SPECIFIC ANTIBODIES SPECIFICALLY BINDING TO MASP3 AND MASP2", the contents of which are incorporated herein by reference in its entirety.

### REFERENCE TO AN ELECTRONIC SEQUENCE LISTING

The contents of the electronic sequence listing (file name: M23-2.xml, date recorded: June 9, 2023, size: 76KB) is herein incorporated by reference in its entirety.

### FIELD

This application pertains to antibodies that specifically bind to MASP3, multi-specific antibodies that specifically bind to MASP3 and MASP2, and pharmaceutical compositions comprising antibodies that specifically bind to MASP3 and antibodies that specifically bind to MASP2 and/or multi-specific antibodies, as well as methods of manufacture and uses thereof, including methods of preventing and treating complement dysregulation.

### BACKGROUND

The complement system plays a crucial role in both innate and adaptive immune responses. Complement clears immune complexes and cellular debris, and rapidly generates highly efficient and tightly regulated inflammatory and cytolytic immune responses against infectious organisms, including bacteria, viruses, and protozoan parasites (Dunkelberger, Jason R, and Wen-Chao Song. Cell research vol. 20,1 (2010): 34-50.). Complement activation leads to a proteolytic cascade reaction, ultimately resulting in the recruitment, phagocytosis, and cell lysis of inflammatory cells (Kjaer, Troels R et al. Molecular immunology vol. 56,4 (2013): 413-22.). It also produces allergenic toxins (C3a, C4a, C5a) that can effectively promote inflammatory responses, as well as modulators (C3b and C4b) that cover the surface of pathogens and mediate phagocytosis. The endpoint of the cascade reaction is the assembly of membrane attack complexes (MAC) on the cell membrane, forming pores that lead to cell lysis (Dunkelberger, Jason R, and Wen-Chao Song. Cell research vol. 20,1 (2010): 34-50.).

The complement system can be activated through three pathways: classical pathway (CP), alternative pathway (AP), and lectin pathway (LP). The classical pathway begins with C1q, which undergoes conformational changes when it recognizes some target molecules (such as immune complexes or surface bound pentamers), allowing C1q to interact with C1r and C1s to form activated molecules C1qr2s2(Carroll, M C.Annual review of immunology vol. 16 (1998): 545-68.). The enzyme active site is located on C1s. Subsequently, activated C1qr2s2 cleaves C4 and subsequent C2, generating the classical pathway C3 convertase C4b2a. C4b2a can induce proteolytic activity and activate common terminal pathways (Sim, R B, and S A Tsiftsoglou. Biochemical Society transactions vol. 32,Pt 1 (2004): 21-7.).

The lectin pathway can be triggered by circulating pattern recognition receptors (PRRs), which can recognize carbohydrates on the surface of microorganisms, including mannose binding lectin (MBL), collagen lectin, and fibronectin. Ficolin is a type of PRRs with a fibrin-like domain, such as M-ficolin (ficolin-1), L-ficolin (ficolin-2), and H-ficolin (ficolin-3). MBL-associated serine proteases (MASP-1 and MASP-2) are evolutionarily related to C1r and C1s and function similarly. MBL forms a complex with MASPs, which then cleaves C4 and C2 to form C3 convertase in the lectin pathway C4b2a(Garred, Peter et al. Immunological reviews vol. 274,1 (2016): 74-97.).

When C3b appears on the surface of the activator, alternative pathways can be activated (Lachmann, Peter J. Immunology vol. 223,8-9 (2018): 519-523.). After capturing FB with C3b, FB bound to C3b can be cleaved by FD into Ba and Bb. In the blood, MASP3 can cleave pro-PD, continuously providing PD for alternative pathways, even before any activation signal appears (Oroszlán, Gábor et al. Journal of immunology (Baltimore, Md. : 1950) vol. 196,2 (2016): 857-65.;Dobó, József et al. Scientific reports vol. 6 31877. 18 Aug. 2016). C3b and Bb form the C3 convertase C3bRb in alternative pathways, which can cleave more C3 and produce more C3 convertase complexes. This positive feedback mechanism can amplify complement activation initiated by classical or alternative pathways (Harboe, M et al. Clinical and experimental immunology vol. 138,3 (2004): 439-46.). Alternative pathways can also initiate themselves through the so-called 'slow transport' mechanism (Pangburn, M K et al. The Journal of experimental medicine vol. 154,3 (1981): 856-67). C3 slowly hydrolyzes in the cycle, producing C3 (H₂O) similar to C3b. C3 (H₂O) is cleaved by FD after binding to FB. C3 (H₂O) Bb is a liquid C3 convertase that can generate C3b near any surface. On the surface of its own cells, it can be protected from complement mediated damage by different complement inhibitors. On unprotected surfaces (such as bacterial surfaces), deposited C3b can initiate amplification loops in alternative pathways, leading to complete activation of complement.

When the density of deposited C3b reaches a certain point, C3b and C3 convertase (C4b2a or C3bBb) form C5 convertase (C4b2aC3b or C3bPbC3b) (Mannes, Marco et al. Blood vol. 137,4 (2021): 443-455.; Roumenina, Lubka T. Blood vol. 137,4 (2021): 431-432.). From this point on, the three activation pathways enter a common terminal pathway (TP). C5 convertase cleaves C5 into C5a and C5b, where C5a is a highly effective anaphylatoxin, while C5b binds to C6 and C7 to form C5b67, which expose membrane binding sites and binds non-specifically to nearby cell membranes, and capture C8 and multiple C9 molecules to form C5b6789n complex, also known as membrane attack complex (MAC). MAC inserted into the cell membrane can form hydrophilic pores that penetrate the membrane, leading to the lysis and destruction of target cells (Tegla, Cosmin A et al. Immunologic research vol. 51,1 (2011): 45-60.).

The complement system is a highly effective mechanism for cell killing and inflammation. To prevent excessive activation, the complement system is strictly controlled by different inhibitory mechanisms. The delicate balance between activation and inhibition is necessary to maintain the homeostasis of inflammation in the human body. When this balance is disrupted for any reason, self-organization will be disrupted and serious illnesses will occur. Many clinical diseases involve uncontrolled (or sometimes insufficient) complement activation. Usually, the etiology of these diseases is complex, and unnecessary complement activation is just one of the pathological factors. However, evidence obtained through various disease models suggests that preventing or inhibiting pathological complement activation may be a promising therapeutic approach (Dobó, József et al. Frontiers in immunology vol. 9 1851).

Inappropriate or uncontrolled activation of the complement system has been confirmed in many diseases. Some of these are rare diseases mainly mediated by complement, such as paroxysmal nocturnal hemoglobinuria, C3 glomerulonephritis, and atypical hemolytic uremic syndrome. Complement also plays a role in various multifactorial diseases that affect millions of people worldwide, such as ischemia-reperfusion injury (myocardial infarction, stroke) (Hart, Melanie L et al. "Journal of Immunology (Baltimore, Md.: 1950) vol. 174,10 (2005): 6373-80.), age-related macular degeneration (Geerlings, Maartje J et al. Molecular Immunology vol. 84 (2017): 65-76.), and several neurodegenerative diseases (Hong, Soyon et al. Science (New York, NY) vol. 3526286 (2016): 712-716.); Sekar, Aswin et al. Nature vol. 530,7589 (2016): 177-83.) _{∘}

Recently, in addition to AP, LP has also played an important role as a trigger for complement activation in the pathogenesis of IRI and AMD (Hart, Melanie L et al. Journal of immunology (Baltimore, Md. : 1950) vol. 174,10 (2005): 6373-80.; Jordan, J E et al. Circulation vol. 104,12 (2001): 1413-8.; Rohrer, Bärbel et al. Molecular immunology vol. 48,6-7 (2011): e1-8.; Joseph, Kusumam et al. The Journal of biological chemistry vol. 288,18 (2013): 12753-65.). In IRI and AMD, the mechanism of complement activation is as follows: first, natural IgM binds to self-antigens and leads to LP activation, followed by AP amplification reaction and tissue/organ damage (Joseph, Kusumam et al. The Journal of biological chemistry vol. 288,18 (2013): 12753-65.; Holers, V Michael et al. Seminars in immunology vol. 28,3 (2016): 260-7.). Obviously, without the expansion loop provided by AP, CP and LP will not be able to work effectively. In addition, both LP and AP are involved in IgA nephropathy (Daha, Mohamed R, and Cees van Kooten. Journal of Nephrology vol. 29,1 (2016): 1-4). Another study showed that the AP and CP/LP pathways are simultaneously activated in autoimmune glomerulonephritis (Genest, Dominique S et al. Kidney international reports vol. 7, 5 1027-1036, 14 Feb.2022). The imbalance of LP and AP is considered to play important pathophysiological roles in these diseases.

Therefore, a drug or drug combination that inhibits terminal pathways or specifically targets upstream molecules of multiple complement pathways has become a new research and development direction. Although targeting downstream terminal pathways may be more effective in controlling excessive complement activation, it can also result in the loss of important complement system functions. These measures include combating infections, connecting innate and adaptive immune responses, clearing debris, apoptotic cells, *etc.* (Dobó, József et al. Frontiers in immunology vol. 9 1851. 8 Aug. 2018, ). Therefore, targeting upstream molecules of the AP and/or LP signaling pathways can maintain the immune system's clearance ability, maintain a certain degree of host defense against pathogens, and specifically inhibit the complement pathway.

Currently, there have been reports of antibodies against MASP3 or MASP2, such as the Chinese patent CN103687620B which discloses the anti-MASP2 antibody OMS721 (used as a control antibody in the examples of this application) and the uses thereof; The Chinese application CN109715209A discloses the anti-MASP3 antibody OMS906 (used as a control antibody in the examples of the present application) and the uses thereof. However, there are currently no monoclonal antibodies targeting MASP3 or multi-specific antibodies (such as bispecific antibodies) targeting both MASP3 and MASP2 on the market. Therefore, there is an urgent need to develop monoclonal antibodies targeting MASP3 and multi-specific antibodies targeting both MASP3 and MASP2.

The disclosures of all publications, patents, patent applications and published patent applications referred to herein are hereby incorporated herein by reference in their entirety.

### BRIEF SUMMARY OF THE APPLICATION

In one aspect, the present application provides an isolated antibody or antigen-binding fragment specifically binding to MASP3. In some embodiments, the present application provides an isolated antibody or antigen-binding fragment specifically binding to MASP3, comprising: a heavy chain variable domain (V_{H}) comprising a heavy chain complementarity determining region (HC-CDR) 1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a light chain variable domain (V_{L}) comprising a light chain complementarity determining region (LC-CDR) 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

In some embodiments, the present application provides an isolated antibody or antigen-binding fragment specifically binding to MASP3, comprising: (i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7; or (ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

In some embodiments, there is provided an isolated antibody or antigen-binding fragment specifically binding to MASP3, comprising: (i) a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 16, or a variant thereof having at least about 80% sequence identity to the amino acid sequence of SEQ ID NO: 16; (ii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 18, or a variant thereof having at least about 80% sequence identity to the amino acid sequence of SEQ ID NO: 18; (iv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 18, or a variant thereof having at least about 80% sequence identity to the amino acid sequence of SEQ ID NO: 18; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (vii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 18, or a variant thereof having at least about 80% sequence identity to the amino acid sequence of SEQ ID NO: 18; (viii) a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 19, or a variant thereof having at least about 80% sequence identity to the amino acid sequence of SEQ ID NO: 19; (ix) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 20, or a variant thereof having at least about 80% sequence identity to the amino acid sequence of SEQ ID NO: 20; (x) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 20, or a variant thereof having at least about 80% sequence identity to the amino acid sequence of SEQ ID NO: 20; (xi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (xii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (xiii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 22, or a variant thereof having at least about 80% sequence identity to the amino acid sequence of SEQ ID NO: 22; (xiv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; (xv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (xvi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 21, or a variant thereof having at least about 80% sequence identity to the amino acid sequence of SEQ ID NO: 21; or (xvii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, there is provided an isolated antibody or antigen-binding fragment specifically binding to MASP3, which binds to MASP3 competitively with any one of the isolated antibodies or antigen-binding fragment described above. In some embodiments, there is provided an isolated antibody or antigen-binding fragment specifically binding to MASP3, which binds to the same epitope as any one of the isolated antibodies or antigen-binding fragment described above.

In some embodiments, according to any one of the isolated antibodies or antigen-binding fragments specifically binding to MASP3 described above, comprising an Fc region. In some embodiments, the isolated antibody or antigen-binding fragment specifically binding to MASP3 is a full-length IgG antibody. In some embodiments, the isolated antibody or antigen-binding fragment specifically binding to MASP3 is a full-length IgG1, IgG2, IgG3, or IgG4 antibody. In some embodiments, the isolated antibody or antigen-binding fragment specifically binding to MASP3 is chimeric, human, or humanized. In some embodiments, the isolated antibody or antigen-binding fragment specifically binding to MASP3, wherein the antigen-binding fragment is selected from Fab, Fab', F(ab)'2, Fab'-SH, single-chain Fv (scFv), Fv fragment, dAb, Fd, nanobody, diabody or linear antibody.

In some embodiments, there is provided an isolated nucleic acid molecule that encodes any one of the antibodies or antigen-binding fragments specifically binding to MASP3 described above. In some embodiments, there is provided a vector comprising any one of the nucleic acid molecules described above. In some embodiments, there is provided a host cell comprising any one of the antibodies or antigen-binding fragments specifically binding to MASP3 described above, any one of the nucleic acid molecules described above, or any one of the vectors described above. In some embodiments, there is provided a method of producing an antibody or antigen-binding fragment specifically binding to MASP3, comprising: a) culturing any one of the host cells described above under conditions effective to express the antibody or antigen-binding fragment specifically binding to MASP3; and b) obtaining the expressed antibody from the host cell.

In some embodiments, there are provided pharmaceutical compositions, kits and articles of manufacture comprising any one of the antibodies or antigen-binding fragments specifically binding to MASP3 described above.

In some embodiments, there is provided a method of treating a disease or condition in an individual in need thereof, comprising administering to the individual an effective amount of any one of the antibodies or antigen-binding fragments specifically binding to MASP3 described above or a pharmaceutical composition containing thereof. In some embodiments, there is provided the use of any one of the antibodies or antigen-binding fragments specifically binding to MASP3 described above in the manufacture of pharmaceutical compositions for treating a disease or condition in an individual in need. In some embodiments, there is provided the use of any one of the antibodies or antigen-binding fragments specifically binding to MASP3 described above or a pharmaceutical composition containing thereof in the manufacture of a medicament for treating a disease or condition in an individual in need. In some embodiments, the disease or condition comprises the disease or condition related to complement dysregulation. In some embodiments, the disease or condition comprises ischemia reperfusion injury, atherosclerosis, mesangial proliferative glomerulonephritis, membranous glomerulonephritis, membranoproliferative glomerulonephritis, acute post infection glomerulonephritis, cryoglobulinemic glomerulonephritis, lupus nephritis, systemic lupus erythematosus (SLE), Henoch Schonlein purpura nephritis, IgA nephropathy, ischemic shock, hemolytic anemia, autoimmune thrombotic thrombocytopenic purpura (TTP), hemolytic uremic syndrome (HUS), atypical hemolytic uremic syndrome (aHUS), paroxysmal nocturnal hemoglobinuria (PNH), transplant-associated TMA, Upshaw-Schulman syndrome, arthritis, traumatic brain injury, aspiration pneumonia, neuromyelitis optica, multiple sclerosis, amyotrophic lateral sclerosis (ALS), chronic obstructive pulmonary disease (COPD), C3 glomerulopathy, transplant rejection, graft-versus-host disease (GVHD), sepsis, systemic inflammatory response syndrome (SIRS) Acute respiratory distress syndrome (ARDS), ANCA vasculitis, antiphospholipid syndrome, myasthenia gravis, Degos disease, disseminated intravascular coagulation (DIC), Angiogenesis dependent cancer, age-related macular degeneration, retinopathy, proliferative diabetes, retinopathy secondary vitreous hemorrhage, neovascular glaucoma, corneal neovascularization, retinopathy of prematurity and respiratory distress syndrome or pneumonia caused by coronavirus infection.

In another aspect, the present application provides multi-specific antibodies (e.g., bispecific antibodies) specifically binding to MASP3 and MASP2 and pharmaceutical compositions containing the multi-specific antibodies (*e.g*., bispecific antibodies) specifically binding to MASP3 and MASP2. In other embodiments, the present application also provides the methods of using the multi-specific antibodies (*e.g*., bispecific antibodies) described above or pharmaceutical compositions thereof for treating the disease related to complement dysregulation. In other embodiments, the present application also provides the use of the multi-specific antibodies (*e.g*., bispecific antibodies) described above or pharmaceutical compositions thereof in the manufacture of a medicament for treating the disease related to complement dysregulation.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising an HC-CDR1 comprising GKWIE (SEQ ID NO: 1), an HC-CDR2 comprising EILPGSGSTDYNERFKD (SEQ ID NO: 2), and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T, an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P. In some embodiments, the second antigen-binding domain comprises: a V_{H} comprising an HC-CDR1 comprising SDYAWN (SEQ ID NO: 23), an HC-CDR2 comprising YISYSGRTSYNPSLKS (SEQ ID NO: 24), and an HC-CDR3 comprising HYGDY (SEQ ID NO: 25); and a V_{L} comprising an LC-CDR 1 comprising KASQNVGTNVA (SEQ ID NO: 26), an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, the present application provides a multi-specific antibody comprising a first antigen-binding domain specifically binding to MASP3, and a second antigen-binding domain specifically binding to MASP2, wherein the second antigen-binding domain comprises: a V_{H} comprising an HC-CDR1 comprising SDYAWN (SEQ ID NO: 23), an HC-CDR2 comprising YISYSGRTSYNPSLKS (SEQ ID NO: 24), and an HC-CDR3 comprising HYGDY (SEQ ID NO: 25); and a V_{L} comprising an LC-CDR 1 comprising KASQNVGTNVA (SEQ ID NO: 26), an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, according to any one of the multi-specific antibodies described herein, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7. In some embodiments, the first antigen-binding domain comprising: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

In some embodiments, according to any one of the multi-specific antibodies described herein, wherein the second antigen-binding domain comprises: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 28. In some embodiments, the second antigen-binding domain comprising: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 29. In some embodiments, the second antigen-binding domain comprising: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 30. In some embodiments, the second antigen-binding domain comprising: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 31. In some embodiments, the second antigen-binding domain comprising: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 32.

In some embodiments, according to any one of the multi-specific antibodies described herein, wherein the first antigen-binding domain comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8; and the second antigen-binding domain comprising: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27, and an LC-CDR3 comprising the amino acid sequence of SEQ ID NO: 29.

In some embodiments, according to any one of the multi-specific antibodies described herein, wherein the first antigen-binding domain comprises: (a) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (b) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (c) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (d) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (e) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or (f) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, according to any one of the multi-specific antibodies described herein, wherein the second antigen-binding domain comprises: (a) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36; (b) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; (c) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38; (d) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39; (e) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or (f) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

In some embodiments, according to any one of the multi-specific antibodies described herein, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37. In some embodiments, according to any one of the multi-specific antibodies described herein, wherein the first antigen-binding domain comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37. In some embodiments, according to any one of the multi-specific antibodies described herein, wherein the first antigen-binding domain comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37. In some embodiments, according to any one of the multi-specific antibodies described herein, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the multi-specific antibody comprises a Fc region, wherein the Fc region is selected from the Fc of IgG1, IgG2, IgG3, IgG4, IgA, IgM, IgE, or IgD. In some embodiments, the Fc region comprises a variant Fc region. In some embodiments, the Fc region is aglycosylated. In some embodiments, the Fc region is deglycosylated. In some embodiments, the Fc region has reduced fucosylation or is afucosylated. In some embodiments, the variant Fc region comprises a substitution at position 297. In some embodiments, the substitution at position 297 is 297Q. In some embodiments, the variant Fc region comprises a substitution at one or more of positions 239, 282, 289, 297, 312, 324, 330, 335, 337, 339, 356, 359, 361, 383, 384, 398, 400, 440, 422, and 442, as numbered by the EU index.

In some embodiments, the multi-specific antibodies (*e.g*., bispecific antibodies) has the formats selected from the group consisting of DVD-Ig, Bs4Ab, Hetero H, CrossMab, CrossMab2+1, IgG-(scFv)₂ and scFv-Fab IgG.

In some embodiments, the multi-specific antibodies (*e.g*., bispecific antibodies) adopts DVD-Ig format. In some embodiments, the multi-specific antibody comprises four polypeptide chains: wherein,
two of the polypeptide chains comprise V_{H}1-L-V_{H}2-C_{H}1 structure from N- terminus to C-terminus, wherein V_{H}1 is a heavy chain variable domain specifically binding to MASP3; V_{H}2 is a heavy chain variable domain specifically binding to MASP2; L is a peptide linker; C_{H}1 is a heavy chain constant domain 1; wherein the polypeptide chain further comprises an Fc comprising C_{H}2 and C_{H}3 domains;
the other two of the polypeptide chains comprise V_{L}1-L-V_{L}2-C_{L} structure from N- terminus to C-terminus, wherein V_{L}1 is a light chain variable domain specifically binding to MASP3; V_{L}2 is a light chain variable domain specifically binding to MASP2; L is a peptide linker; C_{L} is a light chain constant domain;
wherein the V_{H}1 and V_{L}1 form the antigen-binding domain (Fv) specifically binding to MASP3; the V_{H}2-C_{H}1 and V_{L}2-C_{L} form the antigen-binding domain (Fab) specifically binding to MASP2.

In other embodiments, the multi-specific antibody comprises four polypeptide chains: wherein,
two of the polypeptide chains comprise V_{H}1-L-V_{H}2-C_{H}1 structure from N- terminus to C-terminus, wherein V_{H}1 is a heavy chain variable domain specifically binding to MASP2; V_{H}2 is a heavy chain variable domain specifically binding to MASP3; L is a peptide linker; C_{H}1 is a heavy chain constant domain 1; wherein the polypeptide chain further comprises an Fc comprising C_{H}2 and C_{H}3 domains;
the other two of the polypeptide chains comprise V_{L}1-L-V_{L}2-C_{L} structure from N- terminus to C-terminus, wherein V_{L}1 is a light chain variable domain specifically binding to MASP2; V_{L}2 is a light chain variable domain specifically binding to MASP3; L is a peptide linker; C_{L} is a light chain constant domain;
wherein the V_{H}1 and V_{L}1 form the antigen-binding domain (Fv) specifically binding to MASP2; the V_{H}2-C_{H}1 and V_{L}2-C_{L} form the antigen-binding domain (Fab) specifically binding to MASP3.

In other embodiments, the multi-specific antibodies (*e.g*., bispecific antibodies) described herein adopts Bs4Ab format. In some embodiments, the multi-specific antibody comprises four polypeptide chains: wherein,
two of the polypeptide chains comprise V_{H}1-C_{H}1-L1-V_{H}2-L3-V_{L}2 structure or V_{H}1-C_{H}1-L1-V_{L}2-L3-V_{H}2 structure from N- terminus to C-terminus, wherein V_{H}1 is a heavy chain variable domain specifically binding to MASP3; V_{H}2 is a heavy chain variable domain specifically binding to MASP2; V_{L}2 is a light chain variable domain specifically binding to MASP2; L1 and L3 are peptide linkers; C_{H}1 is a heavy chain constant domain 1; wherein the polypeptide chain further comprises an Fc comprising C_{H}2 and C_{H}3 domains; and
the other two of the polypeptide chains comprise V_{L}1-C_{L} structure from N- terminus to C-terminus, wherein V_{L}1 is a light chain variable domain specifically binding to MASP3, C_{L} is a light chain constant domain;
wherein the V_{H}1-C_{H}1 and V_{L}1-C_{L} form the antigen-binding domain (Fab) specifically binding to MASP3; the V_{H}2-L3-V_{L}2 or V_{L}2-L3-V_{H}2 form the antigen-binding domain (scFv) specifically binding to MASP2.

In some embodiments, the multi-specific antibody comprises four polypeptide chains: wherein,
two of the polypeptide chains comprise V_{H}1-C_{H}1-L1-V_{H}2-L3-V_{L}2 structure or V_{H}1-C_{H}1-L1-V_{L}2-L3-V_{H}2 structure from N- terminus to C-terminus, wherein V_{H}1 is a heavy chain variable domain specifically binding to MASP2; V_{H}2 is a heavy chain variable domain specifically binding to MASP3; V_{L}2 is a light chain variable domain specifically binding to MASP3; L1 and L3 are peptide linkers; C_{H}1 is a heavy chain constant domain 1; wherein the polypeptide chain further comprises an Fc comprising C_{H}2 and C_{H}3 domains; and
the other two of the polypeptide chains comprise V_{L}1-C_{L} structure from N- terminus to C-terminus, wherein V_{L}1 is a light chain variable domain specifically binding to MASP2, C_{L} is a light chain constant domain;
wherein the V_{H}1-C_{H}1 and V_{L}1-C_{L} form the antigen-binding domain (Fab) specifically binding to MASP2; the V_{H}2-L3-V_{L}2 or V_{L}2-L3-V_{H}2 form the antigen-binding domain (scFv) specifically binding to MASP3.

In some embodiments, the multi-specific antibodies (*e.g*., bispecific antibodies) described herein adopts IgG-(scFv)₂ format. In some embodiments, the multi-specific antibody comprises four polypeptide chains: wherein,
two of the polypeptide chains comprise V_{H}1-C_{H}1-C_{H}2-C_{H}3-L-V_{H}2-L3-V_{L}2 structure from N- terminus to C-terminus, wherein V_{H}1 is a heavy chain variable domain specifically binding to MASP3; V_{H}2 is a heavy chain variable domain specifically binding to MASP2; V_{L}2 is a light chain variable domain specifically binding to MASP2; L and L3 are peptide linkers; C_{H}1 is a heavy chain constant domain 1; C_{H}2 is a heavy chain constant domain 2; C_{H}3 is a heavy chain constant domain 3; and
the other two of the polypeptide chains comprise V_{L}1-C_{L} structure from N- terminus to C-terminus, wherein V_{L}1 is a light chain variable domain specifically binding to MASP3, C_{L} is a light chain constant domain;
wherein the V_{H}1-C_{H}1 and V_{L}1-C_{L} form the antigen-binding domain (Fab) specifically binding to MASP3; the V_{H}2-L3-V_{L}2 form the antigen-binding domain (scFv) specifically binding to MASP2.

In some embodiments, the multi-specific antibody comprises four polypeptide chains: wherein,
two of the polypeptide chains comprise V_{H}1-C_{H}1-C_{H}2-C_{H}3-L-V_{H}2-L3-V_{L}2 structure from N- terminus to C-terminus, wherein V_{H}1 is a heavy chain variable domain specifically binding to MASP2; V_{H}2 is a heavy chain variable domain specifically binding to MASP3; V_{L}2 is a light chain variable domain specifically binding to MASP3; L and L3 are peptide linkers; C_{H}1 is a heavy chain constant domain 1; C_{H}2 is a heavy chain constant domain 2; C_{H}3 is a heavy chain constant domain 3; and
the other two of the polypeptide chains comprise V_{L}1-C_{L} structure from N- terminus to C-terminus, wherein V_{L}1 is a light chain variable domain specifically binding to MASP2, C_{L} is a light chain constant domain;
wherein the V_{H}1-C_{H}1 and V_{L}1-C_{L} form the antigen-binding domain (Fab) specifically binding to MASP2; the V_{H}2-L3-V_{L}2 form the antigen-binding domain (scFv) specifically binding to MASP3.

In some embodiments, the multi-specific antibodies (*e.g*., bispecific antibodies) described herein adopts scFv-Fab IgG format. In some embodiments, the multi-specific antibody comprises three polypeptide chains: wherein,
one of the polypeptide chains comprises V_{H}1-C_{H}1 structure from N- terminus to C-terminus, wherein V_{H}1 is a heavy chain variable domain specifically binding to MASP3; C_{H}1 is a heavy chain constant domain 1; wherein the polypeptide chain further comprises an Fc comprising C_{H}2 and C_{H}3 domains; and
one of the polypeptide chains comprises V_{L}1-C_{L} from N- terminus to C-terminus, wherein V_{L}1 is a light chain variable domain specifically binding to MASP3; C_{L} is a light chain constant domain; and
one of the polypeptide chains comprises V_{H}2-L3-V_{L}2 from N- terminus to C-terminus, wherein V_{H}2 is a heavy chain variable domain specifically binding to MASP2; V_{L}2 is a light chain variable domain specifically binding to MASP2; L3 is a peptide linker; wherein the polypeptide chain further comprises an Fc comprising C_{H}2 and C_{H}3 domains;
wherein the V_{H}1-C_{H}1 and V_{L}1-C_{L} form the antigen-binding domain (Fab) specifically binding to MASP3; the V_{H}2-L3-V_{L}2 form the antigen-binding domain (scFv) specifically binding to MASP2.

In some embodiments, the multi-specific antibody comprises three polypeptide chains: wherein,
one of the polypeptide chains comprises V_{H}1-C_{H}1 structure from N- terminus to C-terminus, wherein V_{H}1 is a heavy chain variable domain specifically binding to MASP2; C_{H}1 is a heavy chain constant domain 1; wherein the polypeptide chain further comprises an Fc comprising C_{H}2 and C_{H}3 domains; and
one of the polypeptide chains comprises V_{L}1-C_{L} from N- terminus to C-terminus, wherein V_{L}1 is a light chain variable domain specifically binding to MASP2; C_{L} is a light chain constant domain; and
one of the polypeptide chains comprises V_{H}2-L3-V_{L}2 from N- terminus to C-terminus, wherein V_{H}2 is a heavy chain variable domain specifically binding to MASP3; V_{L}2 is a light chain variable domain specifically binding to MASP3; L3 is a peptide linker; wherein the polypeptide chain further comprises an Fc comprising C_{H}2 and C_{H}3 domains;
wherein the V_{H}1-C_{H}1 and V_{L}1-C_{L} form the antigen-binding domain (Fab) specifically binding to MASP2; the V_{H}2-L3-V_{L}2 form the antigen-binding domain (scFv) specifically binding to MASP3.

In some embodiments, the multi-specific antibodies (*e.g*., bispecific antibodies) described herein adopts Hetero H, CrossMab 2+1 format. In some embodiments, the multi-specific antibody comprises five polypeptide chains: wherein,
one of the polypeptide chains comprises V_{H}1-C_{H}1 structure from N- terminus to C-terminus, wherein V_{H}1 is a heavy chain variable domain specifically binding to MASP3, C_{H}1 is a heavy chain constant domain 1; wherein the polypeptide chain further comprises an Fc comprising C_{H}2 and C_{H}3 domains; and
two of the polypeptide chains comprise V_{L}1-C_{L} from N- terminus to C-terminus, wherein V_{L}1 is a light chain variable domain specifically binding to MASP3; C_{L} is a light chain constant domain; and
one of the polypeptide chains comprises V_{H}1-C_{H}1-V_{H}2-C_{L} from N- terminus to C-terminus, wherein V_{H}1 is a heavy chain variable domain specifically binding to MASP3, C_{H}1 is a heavy chain constant domain 1, V_{H}2 is a heavy chain variable domain specifically binding to MASP2, C_{L} is a light chain constant domain, wherein the polypeptide chain further comprises an Fc comprising C_{H}2 and C_{H}3 domains; and
one of the polypeptide chains comprises V_{L}2-C_{H}1 from N- terminus to C-terminus, wherein V_{L}2 is the first light chain variable domain specifically binding to MASP2, C_{H}1 is a heavy chain constant domain 1.
wherein the V_{H}1-C_{H}1 and V_{L}1-C_{L} form the bivalent antigen-binding domain (Fab) specifically binding to MASP3, the V_{H}2-C_{L} and V_{L}2-C_{H}1 form the antigen-binding domain (Fab) specifically binding to MASP2.

In some embodiments, the multi-specific antibody comprises five polypeptide chains: wherein,
one of the polypeptide chains comprises V_{H}1-C_{H}1 structure from N- terminus to C-terminus, wherein V_{H}1 is a heavy chain variable domain specifically binding to MASP2, C_{H}1 is a heavy chain constant domain 1; wherein the polypeptide chain further comprises an Fc comprising C_{H}2 and C_{H}3 domains; and
two of the polypeptide chains comprise V_{L}1-C_{L} from N- terminus to C-terminus, wherein V_{L}1 is a light chain variable domain specifically binding to MASP2; C_{L} is a light chain constant domain; and
one of the polypeptide chains comprises V_{H}1-C_{H}1-V_{H}2-C_{L} from N- terminus to C-terminus, wherein V_{H}1 is a heavy chain variable domain specifically binding to MASP2, C_{H}1 is a heavy chain constant domain 1, V_{H}2 is a heavy chain variable domain specifically binding to MASP3, C_{L} is a light chain constant domain, wherein the polypeptide chain further comprises an Fc comprising C_{H}2 and C_{H}3 domains; and
one of the polypeptide chains comprises V_{L}2-C_{H}1 from N- terminus to C-terminus, wherein V_{L}2 is the first light chain variable domain specifically binding to MASP3, C_{H}1 is a heavy chain constant domain 1.
wherein the V_{H}1-C_{H}1 and V_{L}1-C_{L} form the bivalent antigen-binding domain (Fab) specifically binding to MASP2, the V_{H}2-C_{L} and V_{L}2-C_{H}1 form the antigen-binding domain (Fab) specifically binding to MASP3.

In some embodiments, the multi-specific antibody comprises five polypeptide chains: wherein,
one of the polypeptide chains comprises V_{H}1-mC_{H}1 structure from N- terminus to C-terminus, wherein V_{H}1 is a heavy chain variable domain specifically binding to MASP3, mC_{H}1 is a heavy chain constant domain 1 containing charged amino acids with opposite charges introduced at specific positions (such as glutamic acid (E) at position 148 and glutamic acid (E) at position 214 of the C_{H}1 domain, which are independently substituted (numbered according to Kabat) (see patent CN106661120B)), wherein the polypeptide chain further comprises an Fc comprising C_{H}2 and C_{H}3 domains; and
two of the polypeptide chains comprise V_{L}1-mC_{L} from N- terminus to C-terminus, wherein V_{L}1 is a light chain variable domain specifically binding to MASP3, mC_{L} is a light chain constant domain containing charged amino acids with opposite charges introduced at specific positions (such as arginine (R) at position 128 and lysine (K) at position 129 of the C_{L} domain, which are independently substituted (numbered according to Kabat) (see patent CN106661120B)); and
one of the polypeptide chains comprises V_{H}1-mC_{H}1-V_{H}2-C_{L} from N- terminus to C-terminus, wherein V_{H}1 is a heavy chain variable domain specifically binding to MASP3, mC_{H}1 is a heavy chain constant domain 1 containing charged amino acids with opposite charges introduced at specific positions (such as glutamic acid (E) at position 148 and glutamic acid (E) at position 214 of the C_{H}1 domain, which are independently substituted (numbered according to Kabat) (see patent CN106661120B)), V_{H}2 is a heavy chain variable domain specifically binding to MASP2, C_{L} is a light chain constant domain, wherein the polypeptide chain further comprises an Fc comprising C_{H}2 and C_{H}3 domains; and
one of the polypeptide chains comprises V_{L}2-C_{H}1 from N- terminus to C-terminus, wherein V_{L}2 is the first light chain variable domain specifically binding to MASP2, C_{H}1 is a heavy chain constant domain 1.
wherein the V_{H}1-mC_{H}1 and V_{L}1-mC_{L} form the bivalent antigen-binding domain (Fab) specifically binding to MASP3, the V_{H}2-C_{L} and V_{L}2-C_{H}1 form the antigen-binding domain (Fab) specifically binding to MASP2.

In some embodiments, the multi-specific antibody comprises five polypeptide chains: wherein,
one of the polypeptide chains comprises V_{H}1-mC_{H}1 structure from N- terminus to C-terminus, wherein V_{H}1 is a heavy chain variable domain specifically binding to MASP2; mC_{H}1 is a heavy chain constant domain 1 containing charged amino acids with opposite charges introduced at specific positions (such as glutamic acid (E) at position 148 and glutamic acid (E) at position 214 of the C_{H}1 domain, which are independently substituted (numbered according to Kabat) (see patent CN106661120B)), wherein the polypeptide chain further comprises an Fc comprising C_{H}2 and C_{H}3 domains; and
two of the polypeptide chains comprise V_{L}1-mC_{L} from N- terminus to C-terminus, wherein V_{L}1 is a light chain variable domain specifically binding to MASP2, mC_{L} is a light chain constant domain containing charged amino acids with opposite charges introduced at specific positions (such as arginine (R) at position 128 and lysine (K) at position 129 of the C_{L} domain, which are independently substituted (numbered according to Kabat) (see patent CN106661120B)); and
one of the polypeptide chains comprises V_{H}1-mC_{H}1-V_{H}2-C_{L} from N- terminus to C-terminus, wherein V_{H}1 is a heavy chain variable domain specifically binding to MASP2, mC_{H}1 is a heavy chain constant domain 1 containing charged amino acids with opposite charges introduced at specific positions (such as glutamic acid (E) at position 148 and glutamic acid (E) at position 214 of the C_{H}1 domain, which are independently substituted (numbered according to Kabat) (see patent CN106661120B)), V_{H}2 is a heavy chain variable domain specifically binding to MASP3, C_{L} is a light chain constant domain, wherein the polypeptide chain further comprises an Fc comprising C_{H}2 and C_{H}3 domains; and
one of the polypeptide chains comprises V_{L}2-C_{H}1 from N- terminus to C-terminus, wherein V_{L}2 is the first light chain variable domain specifically binding to MASP3, C_{H}1 is a heavy chain constant domain 1.
wherein the V_{H}1-mC_{H}1 and V_{L}1-mC_{L} form the bivalent antigen-binding domain (Fab) specifically binding to MASP2, the V_{H}2-C_{L} and V_{L}2-C_{H}1 form the antigen-binding domain (Fab) specifically binding to MASP3.

In some embodiments, the multi-specific antibody adopts Hetero H, CrossMab format. In some embodiments, the multi-specific antibody comprises four polypeptide chains: wherein,
one of the polypeptide chains comprises V_{H}1-C_{H}1 structure from N- terminus to C-terminus, wherein V_{H}1 is a heavy chain variable domain specifically binding to MASP3; C_{H}1 is a heavy chain constant domain 1; wherein the polypeptide chain further comprises an Fc comprising C_{H}2 and C_{H}3 domains; and
one of the polypeptide chains comprises V_{L}1-C_{L} structure from N- terminus to C-terminus, wherein V_{L}1 is a light chain variable domain specifically binding to MASP3, C_{L} is a light chain constant domain; and
one of the polypeptide chains comprises V_{H}2-C_{L} structure from N- terminus to C-terminus, wherein V_{H}2 is a heavy chain variable domain specifically binding to MASP2; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus, wherein V_{L}2 is a light chain variable domain specifically binding to MASP2, C_{H}1 is a heavy chain constant domain 1;
wherein the V_{H}1-C_{H}1 and V_{L}1-C_{L} form the antigen-binding domain (Fab) specifically binding to MASP3; V_{H}2-C_{L} and V_{L}2-C_{H}1 form the antigen-binding domain (Fab) specifically binding to MASP2.

In some embodiments, the multi-specific antibody comprises four polypeptide chains: wherein,
one of the polypeptide chains comprises V_{H}1-C_{H}1 structure from N- terminus to C-terminus, wherein V_{H}1 is a heavy chain variable domain specifically binding to MASP2; C_{H}1 is a heavy chain constant domain 1; wherein the polypeptide chain further comprises an Fc comprising C_{H}2 and C_{H}3 domains; and
one of the polypeptide chains comprises V_{L}1-C_{L} structure from N- terminus to C-terminus, wherein V_{L}1 is a light chain variable domain specifically binding to MASP2, C_{L} is a light chain constant domain; and
one of the polypeptide chains comprises V_{H}2-C_{L} structure from N- terminus to C-terminus, wherein V_{H}2 is a heavy chain variable domain specifically binding to MASP3; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus, wherein V_{L}2 is a light chain variable domain specifically binding to MASP3, C_{H}1 is a heavy chain constant domain 1;
wherein the V_{H}1-C_{H}1 and V_{L}1-C_{L} form the antigen-binding domain (Fab) specifically binding to MASP2; V_{H}2-C_{L} and V_{L}2-C_{H}1 form the antigen-binding domain (Fab) specifically binding to MASP3.

In some embodiments, the multi-specific antibody described herein comprises: (a) the amino acid sequence of SEQ ID NO: 53, or a variant thereof having at least about 80% sequence identity to the amino acid sequence of SEQ ID NO: 53; and/or the amino acid sequence of SEQ ID NO: 54, or a variant thereof having at least about 80% sequence identity to the amino acid sequence of SEQ ID NO: 54; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52; (b) the amino acid sequence of SEQ ID NO: 55, or a variant thereof having at least about 80% sequence identity to the amino acid sequence of SEQ ID NO: 55; and/or the amino acid sequence of SEQ ID NO: 56, or a variant thereof having at least about 80% sequence identity to the amino acid sequence of SEQ ID NO: 56; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52; (c) the amino acid sequence of SEQ ID NO: 57, or a variant thereof having at least about 80% sequence identity to the amino acid sequence of SEQ ID NO: 57; and/or the amino acid sequence of SEQ ID NO: 56, or a variant thereof having at least about 80% sequence identity to the amino acid sequence of SEQ ID NO: 56; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52; or (d) the amino acid sequence of SEQ ID NO: 53, or a variant thereof having at least about 80% sequence identity to the amino acid sequence of SEQ ID NO: 53; and/or the amino acid sequence of SEQ ID NO: 58, or a variant thereof having at least about 80% sequence identity to the amino acid sequence of SEQ ID NO: 58; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52.

In some embodiments, the multi-specific antibody described herein comprises: (a) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 54, or a variant thereof having at least about 80% sequence identity to the amino acid sequence of SEQ ID NO: 54; and/or the amino acid sequence of SEQ ID NO: 60, or a variant thereof having at least about 80% sequence identity to the amino acid sequence of SEQ ID NO: 60; (b) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 56, or a variant thereof having at least about 80% sequence identity to the amino acid sequence of SEQ ID NO: 56; and/or the amino acid sequence of SEQ ID NO: 61, or a variant thereof having at least about 80% sequence identity to the amino acid sequence of SEQ ID NO: 61; (c) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 56, or a variant thereof having at least about 80% sequence identity to the amino acid sequence of SEQ ID NO: 56; and/or the amino acid sequence of SEQ ID NO: 62, or a variant thereof having at least about 80% sequence identity to the amino acid sequence of SEQ ID NO: 62; or (d) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 58, or a variant thereof having at least about 80% sequence identity to the amino acid sequence of SEQ ID NO: 58; and/or the amino acid sequence of SEQ ID NO: 60, or a variant thereof having at least about 80% sequence identity to the amino acid sequence of SEQ ID NO: 60.

In some embodiments, there is provided a method of treating a disease or condition in an individual in need thereof, comprising administering to the individual an effective amount of any one of the multi-specific antibodies (*e.g*., bispecific antibodies) described herein and/or pharmaceutical compositions thereof. In some embodiments, there is provided the use of any one of the multi-specific antibodies (*e.g*., bispecific antibodies) described herein in the manufacture of a pharmaceutical composition for treating a disease or condition in an individual in need thereof. In some embodiments, there is provided the use of any one of the multi-specific antibodies (*e.g*., bispecific antibodies) described herein and/or pharmaceutical compositions thereof in the manufacture of a medicament for treating a disease or condition. In some embodiments, the disease or condition is related to complement dysregulation, comprises autoimmune disease, transplantation-related disease, inflammatory disease, hemopathy, coagulation disease, angiogenesis-dependent diseases and/or viral infectious disease. In some embodiments, the disease or condition comprises ischemia reperfusion injury, atherosclerosis, mesangial proliferative glomerulonephritis, membranous glomerulonephritis, membranoproliferative glomerulonephritis, acute post infection glomerulonephritis, cryoglobulinemic glomerulonephritis, lupus nephritis, systemic lupus erythematosus (SLE), Henoch Schonlein purpura nephritis, IgA nephropathy, ischemic shock, hemolytic anemia, autoimmune thrombotic thrombocytopenic purpura (TTP), hemolytic uremic syndrome (HUS), atypical hemolytic uremic syndrome (aHUS), paroxysmal nocturnal hemoglobinuria (PNH), transplant-associated TMA, Upshaw-Schulman syndrome, arthritis, traumatic brain injury, aspiration pneumonia, neuromyelitis optica, multiple sclerosis, amyotrophic lateral sclerosis (ALS), chronic obstructive pulmonary disease (COPD), C3 glomerulopathy, transplant rejection, graft-versus-host disease (GVHD), sepsis, systemic inflammatory response syndrome (SIRS) Acute respiratory distress syndrome (ARDS), ANCA vasculitis, antiphospholipid syndrome, myasthenia gravis, Degos disease, disseminated intravascular coagulation (DIC), Angiogenesis dependent cancer, age-related macular degeneration, retinopathy, proliferative diabetes, retinopathy secondary vitreous hemorrhage, neovascular glaucoma, corneal neovascularization, retinopathy of prematurity and respiratory distress syndrome or pneumonia caused by coronavirus infection.

In some embodiments, there is provided an isolated nucleic acid molecule that encodes any one of the multi-specific antibodies (*e.g*., bispecific antibodies) described above. In some embodiments, there is provided a vector comprising any one of the nucleic acid molecules described above. In some embodiments, there is provided a host cell comprising any one of the multi-specific antibodies (*e.g*., bispecific antibodies), any one of the nucleic acid molecules or any one of the vectors described above. In some embodiments, there is provided a method of producing a multi-specific antibody (*e.g*., bispecific antibody) specifically binding to MASP3 and MASP2, comprising: a) culturing any one of the host cells described above under conditions effective to express the multi-specific antibody (*e.g*., bispecific antibody) specifically binding to MASP3 and MASP2; and b) obtaining the expressed multi-specific antibody (*e.g*., bispecific antibody) from the host cell.

Also provided are pharmaceutical compositions, kits and articles of manufacture comprising any one of the multi-specific antibodies (*e.g*., bispecific antibodies), nucleic acids, vectors or host cells described above.

In one aspect, the present application provides a pharmaceutical composition comprises: (i) antibody or antigen-binding fragment specifically binding to MASP3 and (ii) antibody or antigen-binding fragment specifically binding to MASP2.

In one aspect, the present application provides a method of treating a disease or condition in an individual in need thereof, comprising administering to the individual an effective amount of (i) antibody or antigen-binding fragment specifically binding to MASP3 and (ii) antibody or antigen-binding fragment specifically binding to MASP2, or the pharmaceutical composition comprising the antibody or antigen-binding fragment specifically binding to MASP3 and the antibody or antigen-binding fragment specifically binding to MASP2.

In another aspect, the present application provides the use of the antibody or antigen-binding fragment specifically binding to MASP3 and the antibody or antigen-binding fragment specifically binding to MASP2 in the manufacture of a pharmaceutical composition for treating a disease or condition in an individual in need thereof. In another aspect, the present application provides the use of the antibody or antigen-binding fragment specifically binding to MASP3 and the antibody or antigen-binding fragment specifically binding to MASP2, or the pharmaceutical composition comprising the antibody or antigen-binding fragment specifically binding to MASP3 and the antibody or antigen-binding fragment specifically binding to MASP2 in the manufacture of a medicament for treating a disease or condition.

In some embodiments, according to the pharmaceutical compositions, methods or uses described herein, the antibody or antigen-binding fragment specifically binding to MASP3 comprises: (a) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

In some embodiments, according to the pharmaceutical compositions, methods or uses described herein, the antibody or antigen-binding fragment specifically binding to MASP2 comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, according to the pharmaceutical compositions, methods or uses described herein, the antibody or antigen-binding fragment specifically binding to MASP3 comprises: (i) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 4; an LC-CDR2 comprising the amino acid sequence of SEQ ID NO: 6; and an LC-CDR3 comprising the amino acid sequence of SEQ ID NO: 7; or (ii) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8.

In some embodiments, according to the pharmaceutical compositions, methods or uses described herein, the antibody or antigen-binding fragment specifically binding to MASP2 comprises: (i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28; (ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29; (iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30; (iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or (v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

In some embodiments, according to the pharmaceutical compositions, methods or uses described herein, the antibody or antigen-binding fragment specifically binding to MASP3 comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, according to the pharmaceutical compositions, methods or uses described herein, the antibody or antigen-binding fragment specifically binding to MASP2 comprises: (i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36; (ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; (iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38; (iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39; (v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or (vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

In some embodiments, according to the pharmaceutical compositions, methods or uses described herein, the antibody or antigen-binding fragment specifically binding to MASP3 comprises: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8; and wherein the antibody or antigen-binding fragment specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29.

In some embodiments, according to the pharmaceutical compositions, methods or uses described herein, the antibody or antigen-binding fragment specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the pharmaceutical compositions, methods or uses described herein, the antibody or antigen-binding fragment specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the pharmaceutical compositions, methods or uses described herein, the antibody or antigen-binding fragment specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the pharmaceutical compositions, methods or uses described herein, the antibody or antigen-binding fragment specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the methods or uses described herein, the antibody or antigen-binding fragment specifically binding to MASP3 and the antibody or antigen-binding fragment specifically binding to MASP2 are administered concurrently. In other embodiments, the antibody or antigen-binding fragment specifically binding to MASP3 and the antibody or antigen-binding fragment specifically binding to MASP2 are administered consecutively.

In some embodiments, according to the pharmaceutical compositions, methods or uses described herein, the molar ratio of the antibody or antigen-binding fragment specifically binding to MASP3 and the antibody or antigen-binding fragment specifically binding to MASP2 is any one of about 5:1, 4:1, 3:1, 2:1, 1:1, 1:2, 1:3, 1:4 or 1:5. In some embodiments, the molar ratio of the antibody or antigen-binding fragment specifically binding to MASP3 and the antibody or antigen-binding fragment specifically binding to MASP2 is about 2:1 or 1:1.

In one aspect, the present application provides a method of treating and/or preventing a disease or condition in an individual in need thereof, comprising administering to the individual an effective amount of any one of the antibodies or antigen-binding fragments and/or multi-specific antibodies and/or any one of the pharmaceutical compositions described herein.

In some embodiments, according to any one of the methods provided herein, the disease or condition comprises one or more symptoms caused by complement dysregulation. In some embodiments, the disease or condition comprises autoimmune disease, transplantation-related disease, inflammatory disease, hemopathy, coagulation disease, angiogenesis-dependent diseases and/or viral infectious disease. In some embodiments, the disease or condition comprises ischemia reperfusion injury, atherosclerosis, mesangial proliferative glomerulonephritis, membranous glomerulonephritis, membranoproliferative glomerulonephritis, acute post infection glomerulonephritis, cryoglobulinemic glomerulonephritis, lupus nephritis, systemic lupus erythematosus (SLE), Henoch Schonlein purpura nephritis, IgA nephropathy, ischemic shock, hemolytic anemia, autoimmune thrombotic thrombocytopenic purpura (TTP), hemolytic uremic syndrome (HUS), atypical hemolytic uremic syndrome (aHUS), paroxysmal nocturnal hemoglobinuria (PNH), transplant-associated TMA, Upshaw-Schulman syndrome, arthritis, traumatic brain injury, aspiration pneumonia, neuromyelitis optica, multiple sclerosis, amyotrophic lateral sclerosis (ALS), chronic obstructive pulmonary disease (COPD), C3 glomerulopathy, transplant rejection, graft-versus-host disease (GVHD), sepsis, systemic inflammatory response syndrome (SIRS) Acute respiratory distress syndrome (ARDS), ANCA vasculitis, antiphospholipid syndrome, myasthenia gravis, Degos disease, disseminated intravascular coagulation (DIC), Angiogenesis dependent cancer, age-related macular degeneration, retinopathy, proliferative diabetes, retinopathy secondary vitreous hemorrhage, neovascular glaucoma, corneal neovascularization, retinopathy of prematurity and respiratory distress syndrome or pneumonia caused by coronavirus infection.

### BRIEF DESCRIPTION OF THE DRAWINGS

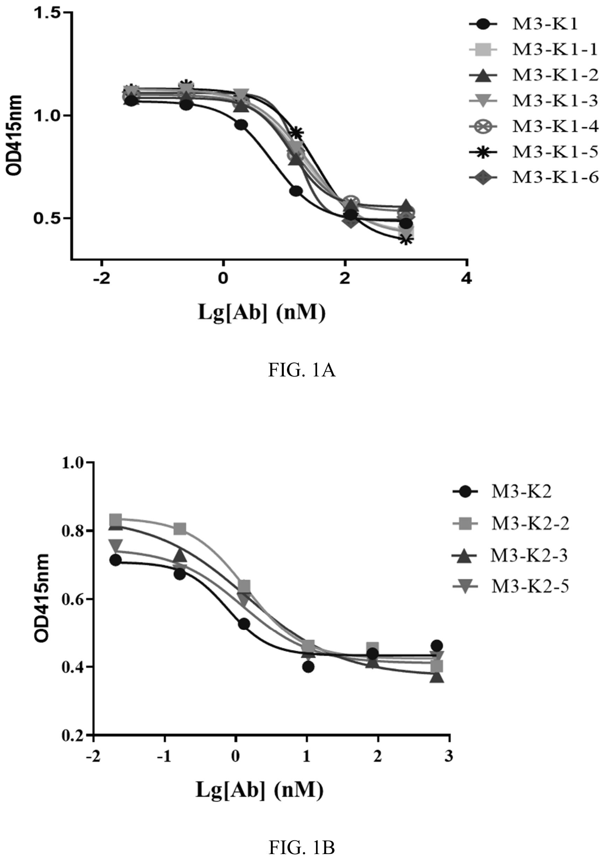

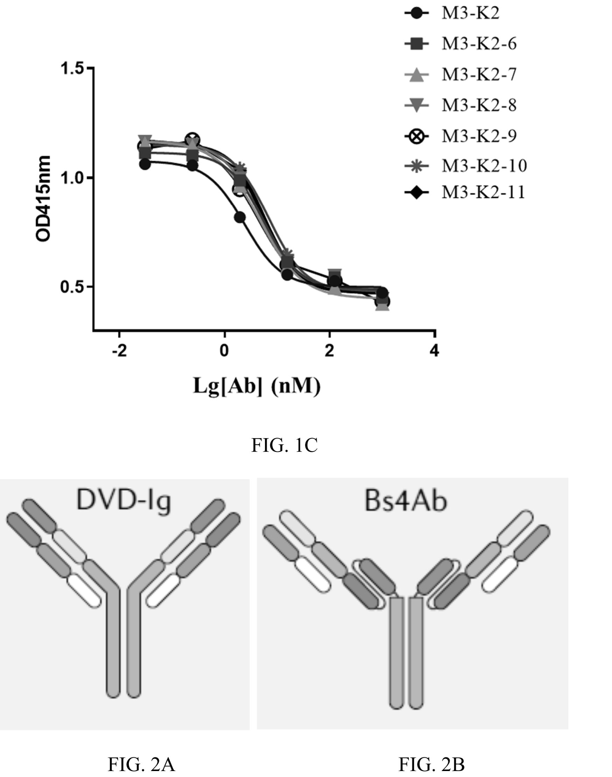

FIGs. 1A-1C show the inhibitory activity of humanized M3-K1 and M3-K2 antibodies in hemolysis mediated by the complement substitution pathway. All the humanized antibodies M3-K1-1~ M3-K1-6 (FIG. 1A), M3-K2-2, M3-K2-3, M3-K2-5(FIG. 1B), and M3-K2-6~ M3-K2-11 can all inhibit hemolytic activity mediated by the complement alternative pathway.

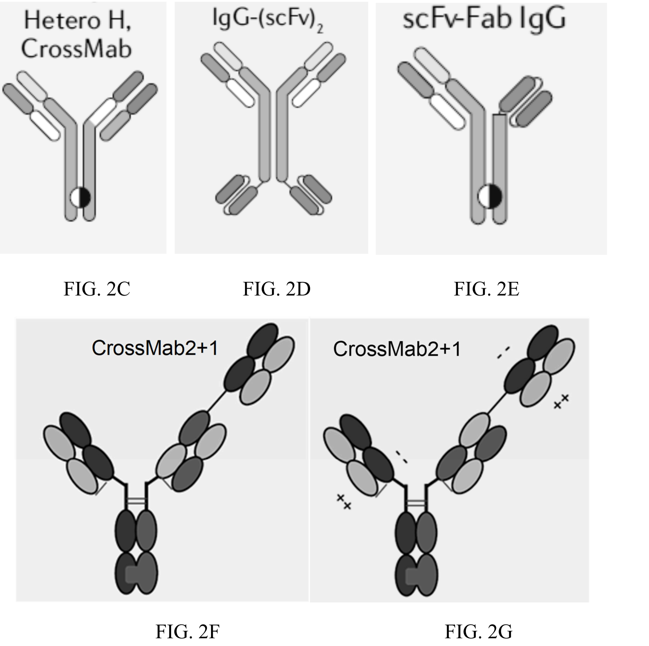

FIG. 2A shows the schematic diagram of DVD-Ig (Dual-variable domain-Ig) format multi-specific antibody; FIG. 2B shows the schematic diagram of Bs4Ab format multi-specific antibody; FIG. 2C shows the schematic diagram of Hetero H, CrossMab format multi-specific antibody; FIG. 2D shows the schematic diagram of IgG-(scFv)₂ format multi-specific antibody; FIG. 2E shows the schematic diagram of scFv-Fab IgG format multi-specific antibody; FIG. 2F shows the schematic diagram of Hetero H, CrossMab2+1 format multi-specific antibody. FIG. 2G shows the schematic diagram of Hetero H, CrossMab2+1 format multi-specific antibody with opposite charged amino acid mutations in the non exchange Fab region.

### DETAILED DESCRIPTION OF THE APPLICATION

The present application in one aspect provides antibodies or antigen-binding fragments specifically binding to MASP3. In another aspect provides multi-specific antibodies specifically binding to MASP3 and MASP2. In another aspect provides pharmaceutical compositions comprising antibodies or antigen-binding fragments specifically binding to MASP3 and antibodies or antigen-binding fragments specifically binding to MASP2, pharmaceutical compositions comprising multi-specific antibodies specifically binding to MASP3 and MASP2. In another aspect provides a method of treating and/or preventing a disease or condition in an individual in need thereof, comprising administering to the individual an effective amount of antibodies specifically binding to MASP3 or pharmaceutical compositions thereof, multi-specific antibodies specifically binding to MASP3 and MASP2 or pharmaceutical compositions thereof, or antibodies or antigen-binding fragments specifically binding to MASP3 and antibodies or antigen-binding fragments specifically binding to MASP2 or pharmaceutical compositions thereof. In another aspect provides the use of antibodies specifically binding to MASP3 or pharmaceutical compositions thereof, multi-specific antibodies specifically binding to MASP3 and MASP2 or pharmaceutical compositions thereof, or antibodies or antigen-binding fragments specifically binding to MASP3 and antibodies or antigen-binding fragments specifically binding to MASP2 or pharmaceutical compositions thereof in the manufacture of a medicament for treating and/or preventing a disease or condition in an individual in need thereof.

By using a combination of selections on scFv phage libraries, antibody humanization and appropriately designed biochemical and biological assays, we have identified the antibodies or antigen-binding fragments specifically binding to MASP3 and the antibodies or antigen-binding fragments specifically binding to MASP2. The multi-specific antibodies specifically binding to MASP3 and MASP2 were also produced. The results described herein indicate that, the combination of these antibody binding moieties or in the form of (i) a pharmaceutical composition, or (ii) a multi-specific antibody, or (iii) a combination, inhibit the complement lectin pathway and alternative pathway simultaneously, for use in treating and/or preventing a disease/condition related to complement dysregulation, compared to administering the antibody molecule each alone.

The present application also provided are nucleic acids encoding the antibodies or antigen-binding fragments specifically binding to MASP3, or the multi-specific antibodies specifically binding to MASP3 and MASP2, multi-specific antibodies, compositions comprising the antibodies or antigen-binding fragments specifically binding to MASP3 and antibodies or antigen-binding fragments specifically binding to MASP2, and methods of making and using the antibodies specifically binding to MASP3, multi-specific antibodies specifically binding to MASP3 and MASP2, and the pharmaceutical compositions comprising any one of the antibodies or antigen binding fragments.

### Definitions

As used herein, "treatment" or "treating" is an approach for obtaining beneficial or desired results, including clinical results. For purposes of this application, beneficial or desired clinical results include, but are not limited to, one or more of the following: alleviating one or more symptoms resulting from the disease, diminishing the extent of the disease, stabilizing the disease (*e.g*., preventing or delaying the worsening of the disease), preventing or delaying the spread (*e.g*., transfer) of the disease, preventing or delaying the recurrence of the disease, delaying or slowing the progression of the disease, ameliorating the disease state, providing a remission (partial or total) of the disease, decreasing the dose of one or more other medications required to treat the disease, delaying the progression of the disease, increasing or improving the quality of life, increasing weight gain, and/or prolonging survival. Also encompassed by "treatment" is a reduction of pathological consequence of disease (such as, for example, reduced deposition of immune complexes and inhibition of thrombus formation). The methods of the application contemplate any one or more of these aspects of treatment.

The term "prevent," and similar words such as "prevented," "preventing," "prevention" or "prophylactic"*etc*., indicate an approach for preventing, inhibiting, or reducing the likelihood of the occurrence or recurrence of, a disease or condition. It also refers to delaying the occurrence or recurrence of a disease or condition, or delaying the occurrence or recurrence of the symptoms of a disease or condition. As used herein, "prevention" and similar words also includes reducing the intensity, effect, symptoms and/or burden of a disease or condition prior to occurrence or recurrence of the disease or condition. As used herein, "prevention" and similar words also includes reducing the risk and susceptibility to occurrence or recurrence of the disease or condition.

The term "antibody" is used in the broadest sense and encompasses a variety of antibody structures, including, but not limited to, monoclonal antibodies, polyclonal antibodies, monospecific, multi-specific antibodies (*e.g*., multi-specific antibodies), full-length antibodies and antigen-binding fragments thereof, so long as they exhibit the desired antigen binding activity. A full-length antibody comprises two heavy chains and two light chains. The variable domains of the light and heavy chains are responsible for antigen binding. The variable domains in both chains generally contain three highly variable loops called the complementarity determining regions (CDRs), light chain (LC) CDRs including LC-CDR1, LC-CDR2, and LC-CDR3, heavy chain (HC) CDRs including HC-CDR1, HC-CDR2, and HC-CDR3. CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µheavy chains, respectively. Several of the major antibody classes are divided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain).

The term "antigen-binding fragment" as used herein includes an antibody fragment including, for example, a diabody, a Fab, a Fab', a F(ab')2, an Fv fragment, a disulfide stabilized Fv fragment (dsFv), a (dsFv)₂, a bispecific dsFv (dsFv-dsFv'), a disulfide stabilized diabody (ds diabody), a single-chain Fv (scFv), an scFv dimer (bivalent diabody), a multi-specific antibody formed from a portion of an antibody comprising one or more CDRs, a camelized single domain antibody, a nanobody, a domain antibody, a bivalent domain antibody, or any other antibody fragment that binds to an antigen but does not comprise a complete antibody structure. Wherein the Fab (fragment antigen-binding) as used herein is monovalent fragment containing the V_{L} domain, V_{H} domain, C_{L} domain, and C_{H}1 domain of antibodies. An antigen-binding fragment also includes a fusion protein comprising the antibody fragment described above. An antigen-binding fragment is capable of binding to the same antigen to which the parent antibody or a parent antibody fragment (*e.g*., a parent scFv) binds. In some embodiments, an antigen-binding fragment may comprise one or more CDRs from a particular human antibody grafted to a framework region from one or more different human antibodies.

The term "multi-specific antibody" as used herein refers to an antibody molecule (bispecific antibody) having binding specificity to at least two different antigen or epitopes in one molecule. Preferably, the multi-specific antibody is a bispecific antibody. The term "bispecific antibody" as used herein refers to an antibody molecule having binding specificity to two different antigen or epitopes in one molecule. Multi-specific antibody (*e.g*., bispecific antibody) is produced through a process that involves design of the intact molecule, synthesis and cloning of the nucleotide sequences for each domain, expression in mammalian cells and purification of the final product. Exemplary multi-specific antibodies (*e.g*., bispecific antibodies) formats include those known in the art, *e.g*., DVD-Ig format, Bs4Ab format, Hetero H, CrossMab format, CrossMab2+1 format, IgG- (scFv)₂ format or scFv-Fab IgG format, *etc.* (see, *e.g.,* Labrijn AF, et al. Nat Rev Drug Discov. 2019 Aug;18(8):585-608).

The DVD-Ig (Dual-variable domain-Ig) multi-specific antibody, the format of which is connecting the V_{L} and V_{H} domain of another antibody respectively to the N-terminus of the light chain and heavy chains of a full-length IgG antibody, and forming an antigen-binding domain (Fv) by the interaction between V_{H} and V_{L}, which can simultaneously bind to the corresponding antigen to achieve bispecific binding. Exemplary DVD-Ig format multi-specific antibody is described in Wu C, et al. Molecular construction and optimization of anti-human IL-1alpha/beta dual variable region immunoglobulin (DVD-Ig) molecules. MAbs. 2009 Jul-Aug;1(4):339-47. The Bs4Ab format multi-specific antibody is a tetravalent bispecific antibody comprising a full length IgG1 with another binding unit scFv inserted into the hinge domain to achieve bispecific. The Bs4Ab format multi-specific antibody is described in document Bezabeh B, et al. Insertion of scFv into the hinge domain of full-length IgG1 monoclonal antibody results in tetravalent bispecific molecule with robust properties. MAbs. 2017 Feb/Mar;9(2):240-256. The Hetero H, crossMab format multi-specific antibody, *i.e.,* the knob-in-hole structure (KIH) is designed in the Fc region, introduces two Cys residue mutations that form stable disulfide bridges (S354C on the "knob" side and Y349C on the "hole" side). CrossMab technology was applied simultaneously to ensure correct pairing between the light and heavy chains of the antibody. CrossMAb technology is based on the exchange of antibody domains in one Fab arm of bispecific IgG antibodies, either as an exchange of intact Fab domains (CrossMAb Fab), or as an exchange of only variable domains (CrossMAb V_{H}-V_{L}) or only constant domains (CrossMAb C_{H}1-Cᵣ.) in the Fab domain. The Hetero H, CrossMab format bispecific antibody is described in Klein C, et al. The use of CrossMAb technology for the generation of bi- and multi-specific antibodies. MAbs. 2016 Aug-Sep;8(6):1010-20. The CrossMab2+1 multi-specific antibody, the format of which adds a Fab portion of one antigen binding domain on the basis of the CrossMab structure, forming a monovalent binding of one antigen binding domain and a divalent binding of the other antigen binding domain. In addition, by introducing charged amino acids with opposite charges at specific amino acid positions in the C_{H}1 and C_{L} domains, the ratio of desired multi-specific antibodies to unwanted byproducts can be increased, for example, in the constant domain C_{L} of one Fab molecule, the amino acid at position 128 is independently replaced with arginine (R), and the amino acid at position 129 is independently replaced with lysine (K) (numbered according to Kabat); and in the constant domain C_{H}1 of the corresponding Fab molecule, the amino acid at position 148 is independently replaced with glutamic acid (E), and the amino acid at position 214 is independently replaced with glutamic acid (E) (numbering according to Kabat) (see patent CN10666110B). CrossMab 2+1 format is described in CN103748114B, CN106661120B, Klein C, et al. Engineering therapeutic bispecific antibodies using CrossMab technology. Methods. 2019 Feb 1;154:21-31. The IgG- (scFv)₂ format multi-specific antibody is achieved by connecting the scFv fragment of another antibody to the Fc-terminus of two heavy chains of an IgG antibody. The IgG- (scFv)₂ format multi-specific antibody is described in Coloma MJ, Morrison SL. Design and production of novel tetravalent bispecific antibodies. Nat Biotechnol. 1997 Feb;15(2):159-63. The scFv-Fab IgG format multi-specific antibody is a heterodimeric antibody, with IgG antibody structure, wherein one Fab arm is replaced with an scFv structure, wherein the first monomer comprises scFv and IgG Fc, the scFv is linked to IgG Fc C_{H}2 domain by a peptide linker and the second monomer comprises Fab and IgG Fc.

The term "antigen-binding domain" as used herein refers to the portion of an antigen binding molecule that specifically binds to an antigen. More specifically, the term "antigen-binding domain" refers to a portion of an antibody that comprises a region that specifically binds to and is complementary to a portion or all of an antigen. In the case of large antigens, the antigen binding molecule may bind only a specific part of the antigen, which part is called an epitope. The antigen-binding fragment may be provided by, for example, one or more variable domains (also referred to as variable regions). Preferably, the antigen-binding fragment comprises an antibody light chain variable domain (V_{L}) and an antibody heavy chain variable domain (V_{H}). In one aspect, the antigen-binding fragment is capable of binding its antigen and blocking or partially blocking the function of said antigen. antigen-binding fragments that specifically bind MASP3 or MASP2 include antibodies and fragments thereof as further defined herein.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

As used herein, a first antibody "competes" for binding to a target MASP3 with a second antibody when the first antibody inhibits target MASP3 binding of the second antibody by at least about 50% (such as at least about any of 55%, 60%, 65%, 70%, 75%, 80%, 85%, 90%, 95%, 98% or 99%) in the presence of an equimolar concentration of the first antibody, or *vice versa.* A high throughput process for "binning" antibodies based upon their cross-competition is described in PCT Publication No. WO 03/48731.

As used herein, the term "specifically binds," "specifically recognizing," or "is specific for" refers to measurable and reproducible interactions, such as binding between a target and an antibody, that is determinative of the presence of the target in the presence of a heterogeneous population of molecules, including biological molecules. For example, an antibody that specifically recognizes a target (which can be an epitope) is an antibody that binds to this target with greater affinity, avidity, more readily, and/or with greater duration than its bindings to other targets. In some embodiments, an antibody that specifically recognizes an antigen reacts with one or more antigenic determinants of the antigen with a binding affinity that is at least about 10 times its binding affinity for other targets.

An "isolated" antibody as used herein refers to an antibody that (1) is not associated with proteins found in nature, (2) is free of other proteins from the same source, (3) is expressed by a cell from a different species, or, (4) does not occur in nature.

The term "isolated nucleic acid" as used herein is intended to mean a nucleic acid of genomic, cDNA, or synthetic origin or some combination thereof, which by virtue of its origin the "isolated nucleic acid" (1) is not associated with all or a portion of a polynucleotide in which the "isolated nucleic acid" is found in nature, (2) is operably linked to a polynucleotide which it is not linked to in nature, or (3) does not occur in nature as part of a larger sequence.

As used herein, the term "CDR" or "complementarity determining region" is intended to mean the non-contiguous antigen combining sites found within the variable domain of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**TABLE 1: CDR DEFINITIONS**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat *et al., supra* ²Residue numbering follows the nomenclature of Chothia *et al., supra* ³Residue numbering follows the nomenclature of MacCallum *et al., supra* ⁴Residue numbering follows the nomenclature of Lefranc *et al., supra* ⁵Residue numbering follows the nomenclature of Honegger and Plückthun, *supra* | | | | | |

The term "chimeric antibodies" refer to antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit a biological activity of this application (*see* U.S. Patent No. 4,816,567; and Morrison et al., Proc. Natl. Acad. Sci. USA, 81:6851-6855 (1984)).

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the heavy and light chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv," also abbreviated as "sFv" or "scFv," are antibody fragments that comprise the V_{H} and V_{L} antibody domains connected into a single polypeptide chain. In some embodiments, the scFv polypeptide further comprises a peptide linker between the V_{H} and V_{L} domains which enables the scFv to form the desired structure for antigen binding. For a review of scFv, *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments prepared by constructing scFv fragments (see preceding paragraph) typically with short linkers (such as about 5 to about 10 residues) between the V_{H} and V_{L} domains such that inter-chain but not intra-chain pairing of the V domains is achieved, resulting in a bivalent fragment, *i.e.,* fragment having two antigen-binding sites. Multi-specific diabodies are heterodimers of two "crossover" scFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region (HVR) of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies can comprise residues that are not found in the recipient antibody or in the donor antibody. These modifications are made to further refine antibody performance. In general, the humanized antibody will comprise substantially at least one, and typically two, variable regions, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skilled in the art, for instance, using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program MUSCLE (Edgar, R.C., Nucleic Acids Research 32(5):1792-1797, 2004; Edgar, R.C., BMC Bioinformatics 5(1):113, 2004).

The terms "Fc (fragment crystallizable)" or "Fc region" refers to polypeptides comprising the complete antibody constant region, excluding the CH₁ domain, and in some cases comprising a partial hinge region, whether in monomeric form or multimeric form. The primary immunoglobulin source of the natural Fc is preferably of human origin and can be any immunoglobulin, such as IgG1, IgG2, IgG3, or IgG4. Natural Fc is composed of monomeric peptides, which can be covalently (*i.e*., disulfide bonds) and non-covalently linked into dimeric or multimeric forms. The Fc region of immunoglobulins generally comprises the CH₂ and CH₃ domains of the heavy chain constant region, and can optionally comprise the CH₄ domain.

In some embodiments, each of the two Fc monomers in the Fc dimer comprises an amino acid substitution that promotes heterodimerization of the two monomers. In some embodiments, heterodimerization of the Fc monomers may be facilitated by introducing different but compatible substitutions in the two Fc monomers, such as "knob-into-hole" residue pairs. The "knob-into-hole" technology is also disclosed in U.S. patent No. 8,216,805. In some embodiments, one Fc monomer comprises the knob mutation T366W and the other Fc monomer comprises the hole mutations T366S, L358A and Y407V. In some embodiments, two Cys residues were introduced to form a stabilized disulfide bridge (S354C in the "knob" side and Y349C in the "hole" side).

The terms "Fc receptor" or "FcR" are used to describe a receptor that binds to the Fc region of an antibody. In some embodiments, an FcR of this application is one that binds an IgG antibody (a γ receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain (*see* review M. in Daëron, Annu. Rev. Immunol. 15:203-234 (1997)). The term includes allotypes, such as FcγRIIIA allotypes: FcγRIIIA-Phe158, FcγRIIIA-Val158, FcγRIIA-R131 and/or FcγRIIA-H131. FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The term "FcRn" refers to the neonatal Fc receptor (FcRn). FcRn is structurally similar to major histocompatibility complex (MHC) and consists of an α-chain noncovalently bound to β2-microglobulin. The multiple functions of the neonatal Fc receptor FcRn are reviewed in Ghetie and Ward (2000) Annu. Rev. Immunol. 18, 739-766. FcRn plays a role in the passive delivery of immunoglobulin IgGs from mother to young and the regulation of serum IgG levels. FcRn can act as a salvage receptor, binding and transporting pinocytosed IgGs in intact form both within and across cells, and rescuing them from a default degradative pathway.

The "CH1 domain" of a human IgG heavy chain constant region usually extends from about amino acid 118 to about amino acid 215 (EU numbering system).

"Hinge region" is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "CH2 domain" of a human IgG Fc region usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec Immunol. 22:161-206 (1985).

The "CH3 domain" comprises the stretch of residues C-terminal to a CH2 domain in an Fc region (*i.e.,* from about amino acid residue 341 to the C-terminal end of an antibody sequence, typically at amino acid residue 446 or 447 of an IgG).

A "functional Fc fragment" possesses an "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g.* B cell receptor; BCR), *etc.* Such effector functions generally require the Fc region to be combined with a binding domain (e.g. an antibody variable domain) and can be assessed using various assays known in the art.

An antibody with a variant IgG Fc with "altered" FcR binding affinity or ADCC activity is one which has either enhanced or diminished FcR binding activity (*e.g*., FcyR or FcRn) and/or ADCC activity compared to a parent polypeptide or to a polypeptide comprising a native sequence Fc region. The variant Fc which "exhibits increased binding" to an FcR binds at least one FcR with higher affinity (*e.g*., lower apparent Kd or IC₅₀ value) than the parent polypeptide or a native sequence IgG Fc. According to some embodiments, the improvement in binding compared to a parent polypeptide is about 3-fold, such as about any of 5, 10, 25, 50, 60, 100, 150, 200, or up to 500-fold, or about 25% to 1000% improvement in binding. The polypeptide variant which "exhibits decreased binding" to an FcR, binds at least one FcR with lower affinity (*e.g*., higher apparent Kd or higher IC₅₀ value) than a parent polypeptide. The decrease in binding compared to a parent polypeptide may be about 40% or more decrease in binding.

"Antibody-dependent cell-mediated cytotoxicity" or "ADCC" refers to a form of cytotoxicity in which secreted Ig bound to Fc receptors (FcRs) present on certain cytotoxic cells *(e.g.,* Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. PNAS (USA) 95:652-656 (1998).

The polypeptide comprising a variant Fc region which "exhibits increased ADCC" or mediates ADCC in the presence of human effector cells more effectively than a polypeptide having wild type IgG Fc or a parent polypeptide is one which *in vitro* or *in vivo* is substantially more effective at mediating ADCC, when the amounts of polypeptide with variant Fc region and the polypeptide with wild type Fc region (or the parent polypeptide) in the assay are essentially the same. Generally, such variants will be identified using any *in vitro* ADCC assay known in the art, such as assays or methods for determining ADCC activity, *e.g.,* in an animal model *etc.* In some embodiments, the variant is from about 5-fold to about 100-fold, *e.g.* from about 25 to about 50-fold, more effective at mediating ADCC than the wild type Fc (or parent polypeptide).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed. Polypeptide variants with altered Fc region amino acid sequences and increased or decreased C1q binding capability are described in US patent No. 6,194,551B1 and WO99/51642. The contents of those patent publications are specifically incorporated herein by reference. *See* also, Idusogie et al. J. Immunol. 164: 4178-4184 (2000).

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Homologous" refers to the sequence similarity or sequence identity between two polypeptides or between two nucleic acid molecules. When a position in both of the two compared sequences is occupied by the same base or amino acid monomer subunit, *e.g*., if a position in each of two DNA molecules is occupied by adenine, then the molecules are homologous at that position. The percent of homology between two sequences is a function of the number of matching or homologous positions shared by the two sequences divided by the number of positions compared times 100. For example, if 6 of 10 of the positions in two sequences are matched or homologous then the two sequences are 60% homologous. By way of example, the DNA sequences ATTGCC and TATGGC share 50% homology. Generally, a comparison is made when two sequences are aligned to give maximum homology.

An "effective amount" of an antibody (including multi-specific antibody) or composition as disclosed herein, is an amount sufficient to carry out a specifically stated purpose. An "effective amount" can be determined empirically and by known methods relating to the stated purpose.

The term "therapeutically effective amount" refers to an amount of an antibody (including multi-specific antibody) or composition as disclosed herein, refers to the effective amount to treat a disease or disorder in an individual. The amount sufficient to alleviate or improve the severity and/or duration of the disease or one or more of its symptoms. The amount to prevent the development of diseases, cause the regression of symptoms, prevent the recurrence, development, onset, or progression of one or more symptoms related to the disease, detect the disease, or enhance/improve the preventive or therapeutic effect of another therapy (such as prophylactic or therapeutic agents). In some embodiments, the therapeutically effective amount is an amount that can prolong the patient survival. In some embodiments, the therapeutically effective amount is an amount that can improve the progression free survival of a patient.

As used herein, by "pharmaceutically acceptable" or "pharmacologically compatible" is meant a material that is not biologically or otherwise undesirable, *e.g*., the material may be incorporated into a pharmaceutical composition administered to a patient without causing any significant undesirable biological effects or interacting in a deleterious manner with any of the other components of the composition in which it is contained. Pharmaceutically acceptable carriers or excipients have preferably met the required standards of toxicological and manufacturing testing and/or are included on the Inactive Ingredient Guide prepared by the U.S. Food and Drug Administration.

It is understood that embodiments of the application described herein include "consisting of" and/or "consisting essentially of" embodiments.

Reference to "about" a value or parameter herein includes (and describes) variations that are directed to that value or parameter per se. For example, description referring to "about X" includes description of "X".

As used herein, reference to "not" a value or parameter generally means and describes "other than" a value or parameter. For example, the method is not used to treat infection of type X means the method is used to treat infection of types other than X.

As used herein and in the appended claims, the singular forms "a," "or," and "the" include plural referents unless the context clearly dictates otherwise.

### Antibodies specifically binding to MASP3

In one aspect, the present application provides antibodies or antigen-binding fragments specifically binding to MASP3 including, but not limited to, humanized antibodies, chimeric antibodies, mouse antibodies, human antibodies, and antibodies comprising the heavy chain and/or light chain CDRs discussed herein. In one aspect, the antibodies or antigen-binding fragments are isolated antibodies that bind to MASP3. Contemplated antibodies or antigen-binding fragments specifically binding to MASP3 can include the entire or a fragment of full-length antibodies specifically binding to MASP3 (*e.g*., full-length IgG1, IgG2 or IgG4), scFvs specifically binding to MASP3, multi-specific (such as bispecific) antibodies specifically binding to MASP3, immunoconjugates specifically binding to MASP3, and the like. In some embodiments, the antibody or antigen-binding fragment specifically binding to MASP3 is a Fab, a Fab', a F(ab)'2, a Fab'-SH, a single-chain Fv (scFv), an Fv fragment, a dAb, a Fd, a nanobody, a diabody or a linear antibody. In some embodiments, the antibody or antigen-binding fragment specifically binding to MASP3 means that the antibody or antigen-binding fragment binds to MASP3 with an affinity that is at least about 10 times (including for example at least about any of 10, 10², 10³, 10⁴, 10⁵, 10⁶, or 10⁷ times) of its binding affinity for non-target. In some embodiments, the non-target is an antigen that is not MASP3.

Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although the antibodies or antigen-binding fragments specifically binding to MASP3 and containing human sequences (*e.g*., human heavy and light chain variable region sequences comprising human CDR sequences) are extensively discussed herein, non-human antibodies are also contemplated. In some embodiments, non-human antibodies comprise human CDR sequences from an antibody or antigen-binding fragment specifically binding to MASP3 as described herein and non-human framework sequences. Non-human framework sequences include, in some embodiments, any sequence that can be used for generating synthetic heavy and/or light chain variable regions using one or more human CDR sequences as described herein, including, *e.g.,* mammals, *e.g.,* mouse, rat, rabbit, pig, bovine (*e.g.,* cow, bull, buffalo), deer, sheep, goat, chicken, cat, dog, ferret, primate (*e.g.,* marmoset, rhesus monkey), *etc.* In some embodiments, a non-human antibody or antigen-binding fragment specifically binding to MASP3 includes an antibody or antigen-binding fragment specifically binding to MASP3 generated by grafting one or more human CDR sequences as described herein onto a non-human framework sequence (*e.g*., a mouse or chicken framework sequence).

In some embodiments, the antibody or antigen-binding fragment specifically binding to MASP3 binds to the epitope of human MASP3. In some embodiments, the antibody or antigen-binding fragment specifically binding to MASP3 is completely specific for MASP3 and does not exhibit species cross-reactivity or other types of non- MASP3 cross-reactivity. In some embodiments, the antibody or antigen-binding fragment specifically binding to MASP3 exhibits MASP3 antigen of species other than human cross-reactivity.

In some embodiments, the antibody or antigen-binding fragment specifically binding to MASP3, comprising: a heavy chain variable domain (V_{H}) comprising a heavy chain complementarity determining region (HC-CDR) 1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a light chain variable domain (V_{L}) comprising a light chain complementarity determining region (LC-CDR) 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

In some embodiments, the antibody or antigen-binding fragment specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of any one of SEQ ID NOs: 4-5, or a variant thereof comprising up to about 3 amino acid substitutions, an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions.

In some embodiments, the antibody or antigen-binding fragment specifically binding to MASP3 comprises: a V_{H} comprising an HC-CDR1, an HC-CDR2, and an HC-CDR3 of a V_{H} comprising the amino acid sequence of any one of SEQ ID NOs: 11-15; and a V_{L} comprising an LC-CDR1, an LC-CDR2, and an LC-CDR3 of a V_{L} comprising the amino acid sequence of any one of SEQ ID NOs: 16-22.

In some embodiments, the antibody or antigen-binding fragment specifically binding to MASP3 comprises: (i) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 16; or (ii) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19.

In some embodiments, the antibody or antigen-binding fragment specifically binding to MASP3 comprises: (i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3, or a variant thereof comprising up to about 5 amino acid substitutions in the HC-CDRs; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7, or a variant thereof comprising up to about 5 amino acid substitutions in the LC-CDRs; or (ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3, or a variant thereof comprising up to about 5 amino acid substitutions in the HC-CDRs; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8, or a variant thereof comprising up to about 5 amino acid substitutions in the LC-CDRs.

In some embodiments, the antibody or antigen-binding fragment specifically binding to MASP3 comprises: a V_{H} comprising the amino acid sequence of any one of SEQ ID NOs: 11-15, or a variant thereof having at least about 80% sequence identity to the amino acid sequence of any one of SEQ ID NOs: 11-15; and a V_{L} comprising the amino acid sequence of any one of SEQ ID NOs: 16-22, or a variant thereof having at least about 80% sequence identity to the amino acid sequence of any one of SEQ ID NOs: 16-22.

In some embodiments, the antibody or antigen-binding fragment specifically binding to MASP3 comprises: (i) a V_{H} comprising the amino acid sequence of SEQ ID NO: 11, or a variant thereof having at least about 80% (*e.g.,* at least 80%, 85%, 90%, 95%, 96%, 97%, 98%, or 99%) sequence identity to the amino acid sequence of SEQ ID NO: 11; and a V_{L} comprising the amino acid sequence of SEQ ID NO: 16, or a variant thereof having at least about 80% (*e.g.,* at least 80%, 85%, 90%, 95%, 96%, 97%, 98%, or 99%) sequence identity to the amino acid sequence of SEQ ID NO: 16; (ii) a V_{H} comprising the amino acid sequence of SEQ ID NO: 12, or a variant thereof having at least about 80% (*e.g.,* at least 80%, 85%, 90%, 95%, 96%, 97%, 98%, or 99%) sequence identity to the amino acid sequence of SEQ ID NO: 12; and a V_{L} comprising the amino acid sequence of SEQ ID NO: 17, or a variant thereof having at least about 80% (*e.g.,* at least 80%, 85%, 90%, 95%, 96%, 97%, 98%, or 99%) sequence identity to the amino acid sequence of SEQ ID NO: 17; (iii) a V_{H} comprising the amino acid sequence of SEQ ID NO: 12, or a variant thereof having at least about 80% (*e.g.,* at least 80%, 85%, 90%, 95%, 96%, 97%, 98%, or 99%) sequence identity to the amino acid sequence of SEQ ID NO: 12; and a V_{L} comprising the amino acid sequence of SEQ ID NO: 18, or a variant thereof having at least about 80% (*e.g.,* at least 80%, 85%, 90%, 95%, 96%, 97%, 98%, or 99%) sequence identity to the amino acid sequence of SEQ ID NO: 18; (iv) a V_{H} comprising the amino acid sequence of SEQ ID NO: 13, or a variant thereof having at least about 80% (*e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 17, or a variant thereof having at least about 80% *(e.g.,* at least 80%, 85%, 90%, 95%, 96%, 97%, 98%, or 99%) sequence identity to the amino acid sequence of SEQ ID NO: 17; (v) a V_{H} comprising the amino acid sequence of SEQ ID NO: 13, or a variant thereof having at least about 80% (*e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 18, or a variant thereof having at least about 80% (*e.g.,* at least 80%, 85%, 90%, 95%, 96%, 97%, 98%, or 99%) sequence identity to the amino acid sequence of SEQ ID NO: 18; (vi) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 17, or a variant thereof having at least about 80% (*e.g.,* at least 80%, 85%, 90%, 95%, 96%, 97%, 98%, or 99%) sequence identity to the amino acid sequence of SEQ ID NO: 17; (vii) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 18, or a variant thereof having at least about 80% (*e.g.,* at least 80%, 85%, 90%, 95%, 96%, 97%, 98%, or 99%) sequence identity to the amino acid sequence of SEQ ID NO: 18; (viii) a V_{H} comprising the amino acid sequence of SEQ ID NO: 11, or a variant thereof having at least about 80% (*e.g.,* at least 80%, 85%, 90%, 95%, 96%, 97%, 98%, or 99%) sequence identity to the amino acid sequence of SEQ ID NO: 11; and a V_{L} comprising the amino acid sequence of SEQ ID NO: 19, or a variant thereof having at least about 80% (*e.g.,* at least 80%, 85%, 90%, 95%, 96%, 97%, 98%, or 99%) sequence identity to the amino acid sequence of SEQ ID NO: 19; (ix) a V_{H} comprising the amino acid sequence of SEQ ID NO: 13, or a variant thereof having at least about 80% (*e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 20, or a variant thereof having at least about 80% (*e.g.,* at least 80%, 85%, 90%, 95%, 96%, 97%, 98%, or 99%) sequence identity to the amino acid sequence of SEQ ID NO: 20; (x) a V_{H} comprising the amino acid sequence of SEQ ID NO: 15, or a variant thereof having at least about 80% (*e.g.,* at least 80%, 85%, 90%, 95%, 96%, 97%, 98%, or 99%) sequence identity to the amino acid sequence of SEQ ID NO: 15; and a V_{L} comprising the amino acid sequence of SEQ ID NO: 20, or a variant thereof having at least about 80% (*e.g.,* at least 80%, 85%, 90%, 95%, 96%, 97%, 98%, or 99%) sequence identity to the amino acid sequence of SEQ ID NO: 20; (xi) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 20, or a variant thereof having at least about 80% (*e.g.,* at least 80%, 85%, 90%, 95%, 96%, 97%, 98%, or 99%) sequence identity to the amino acid sequence of SEQ ID NO: 20; (xii) a V_{H} comprising the amino acid sequence of SEQ ID NO: 12, or a variant thereof having at least about 80% (*e.g.,* at least 80%, 85%, 90%, 95%, 96%, 97%, 98%, or 99%) sequence identity to the amino acid sequence of SEQ ID NO: 12; and a V_{L} comprising the amino acid sequence of SEQ ID NO: 21, or a variant thereof having at least about 80% *(e.g.,* at least 80%, 85%, 90%, 95%, 96%, 97%, 98%, or 99%) sequence identity to the amino acid sequence of SEQ ID NO: 21; (xiii) a V_{H} comprising the amino acid sequence of SEQ ID NO: 12, or a variant thereof having at least about 80% *(e.g.,* at least 80%, 85%, 90%, 95%, 96%, 97%, 98%, or 99%) sequence identity to the amino acid sequence of SEQ ID NO: 12; and a V_{L} comprising the amino acid sequence of SEQ ID NO: 22, or a variant thereof having at least about 80% *(e.g.,* at least 80%, 85%, 90%, 95%, 96%, 97%, 98%, or 99%) sequence identity to the amino acid sequence of SEQ ID NO: 22; (xiv) a V_{H} comprising the amino acid sequence of SEQ ID NO: 13, or a variant thereof having at least about 80% *(e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 21, or a variant thereof having at least about 80% (*e.g.,* at least 80%, 85%, 90%, 95%, 96%, 97%, 98%, or 99%) sequence identity to the amino acid sequence of SEQ ID NO: 21; (xv) a V_{H} comprising the amino acid sequence of SEQ ID NO: 13, or a variant thereof having at least about 80% (*e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 22, or a variant thereof having at least about 80% (*e.g.,* at least 80%, 85%, 90%, 95%, 96%, 97%, 98%, or 99%) sequence identity to the amino acid sequence of SEQ ID NO: 22; (xvi) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 21, or a variant thereof having at least about 80% (*e.g.,* at least 80%, 85%, 90%, 95%, 96%, 97%, 98%, or 99%) sequence identity to the amino acid sequence of SEQ ID NO: 21; or (xvii) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 22, or a variant thereof having at least about 80% (*e.g.,* at least 80%, 85%, 90%, 95%, 96%, 97%, 98%, or 99%) sequence identity to the amino acid sequence of SEQ ID NO: 22.

In some embodiments, the amino acid substitutions described herein are limited to "exemplary substitutions" shown in Table 10 of this application. In some embodiments, the amino acid substitutions are limited to "preferred substitutions" shown in Table 10 of this application.

In some embodiments, the application provides antibodies specifically binding to MASP3, which bind to MASP3 competitively with any one of the isolated antibodies described above. In some embodiments, the application provides antibodies, which binds to the same epitope as any one of the antibodies specifically binding to MASP3 described above.

In some embodiments, competition assays may be used to identify a monoclonal antibody that competes with the antibodies specifically binding to MASP3 described herein for binding to MASP3. Competition assays can be used to determine whether two antibodies bind to the same epitope by recognizing identical or sterically overlapping epitopes or one antibody competitively inhibits binding of another antibody to the antigen. In certain embodiments, such a competing antibody binds to the same epitope that is bound by an antibody described herein. Exemplary competition assays include, but are not limited to, routine assays such as those provided in Harlow and Lane (1988) Antibodies: A Laboratory Manual ch.14 (Cold Spring Harbor Laboratory, Cold Spring Harbor, N.Y.). Detailed exemplary methods for mapping an epitope to which an antibody binds are provided in Morris (1996) "Epitope Mapping Protocols", in Methods in Molecular Biology vol. 66 (Humana Press, Totowa, N.J.). In some embodiments, two antibodies are said to bind to the same epitope if each blocks binding of the other by 50% or more. In some embodiments, the antibodies that compete with the antibodies specifically binding to MASP3 described herein are chimeric, humanized or human antibodies.

Exemplary antibodies specifically binding to MASP3 sequences are shown in Tables 2 and Table 3, wherein the CDR numbering is according to the Kabat index. Those skilled in the art will recognize that many algorithms (Kabat index) are known for prediction of CDR positions and for delimitation of antibody heavy chain and light chain variable domains. Antibodies specifically binding to MASP3 comprising CDRs, V_{H} and/or V_{L} sequences from antibodies described herein, but based on prediction algorithms other than those exemplified in the tables below, are within the scope of this invention.

### Full-length antibodies specifically binding to MASP3

In some embodiments, the antibody specifically binding to MASP3 is a full-length antibody. In some embodiments, the full-length antibody specifically binding to MASP3 is an IgA, IgD, IgE, IgG, or IgM. In some embodiments, the antibody or antigen-binding fragment specifically binding to MASP3 comprises antibody heavy chain constant region and antibody light chain constant region. In some embodiments, the full-length antibody specifically binding to MASP3 comprises IgG constant regions, such as constant regions of any one of IgG1, IgG2, IgG3, and IgG4 including variants thereof. In some embodiments, the antibody or antigen-binding fragment specifically binding to MASP3 comprises IgG1 heavy chain constant region. In some embodiments, the antibody or antigen-binding fragment specifically binding to MASP3 comprises IgG2 heavy chain constant region. In some embodiments, the antibody or antigen-binding fragment specifically binding to MASP3 comprises IgG3 heavy chain constant region. In some embodiments, the antibody or antigen-binding fragment specifically binding to MASP3 comprises IgG4 heavy chain constant region. In some embodiments, the IgG1 refers to human IgG. In some embodiments, the heavy chain constant region comprises or consists of the amino acid sequence of SEQ ID NO: 43. In some embodiments, the heavy chain constant region comprises or consists of the amino acid sequence of SEQ ID NO: 44. In some embodiments, the antibody or antigen-binding fragment specifically binding to MASP3 comprises a kappa light chain constant region. In some embodiments, the light chain constant region comprises or consists of the amino acid sequence of SEQ ID NO: 45. In some embodiments, the antibody or antigen-binding fragment specifically binding to MASP3 comprises a lambda light chain constant region. In some embodiments, the light chain constant region comprises or consists of the amino acid sequence of SEQ ID NO: 46. In some embodiments, the antibody or antigen-binding fragment specifically binding to MASP3 comprises antibody heavy chain variable domain and antibody light chain variable domain. In some embodiments, the full-length antibody specifically binding to MASP3 comprises an altered or otherwise altered Fc sequence, resulting in enhanced antibody-dependent cell-mediated cytotoxicity (ADCC) and complement dependent cytotoxicity (CDC) effector functions.

In some embodiments, there is provided a full-length antibody specifically binding to MASP3 comprising IgG1, IgG2, IgG3, or IgG4 constant regions, wherein the antibody specifically binding to MASP3 comprises: a) a heavy chain variable domain, the heavy chain variable domain comprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and b) a light chain variable domain, the light chain variable domain comprises an LC-CDR1 comprising the amino acid sequence of any one of SEQ ID NOs: 4-5, or a variant thereof comprising up to about 3 amino acid substitutions (*e.g*., 1, 2, or 3), an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions (*e.g*., 1, 2, or 3). In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG2 is human IgG2. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the IgG3 is human IgG3. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a) a heavy chain variable domain, the heavy chain variable domain comprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and b) a light chain variable domain, the light chain variable domain comprises an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a) a heavy chain variable domain, the heavy chain variable domain comprises: an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and b) a light chain variable domain, the light chain variable domain comprises an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1, IgG2, IgG3, or IgG4 constant regions, wherein the antibody specifically binding to MASP3 comprises: a V_{H} comprising the amino acid sequence of any one of SEQ ID NOs: 11-15, or a variant thereof having at least about 80% (*e.g.,* at least 80%, 85%, 90%, 95%, 96%, 97%, 98%, or 99%) sequence identity to the amino acid sequence of any one of SEQ ID NOs: 11-15; and a V_{L} comprising the amino acid sequence of any one of SEQ ID NOs: 16-22, or a variant thereof having at least about 80% (*e.g.,* at least 80%, 85%, 90%, 95%, 96%, 97%, 98%, or 99%) sequence identity to the amino acid sequence of any one of SEQ ID NOs: 16-22. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG2 is human IgG2. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the IgG3 is human IgG3. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 16, or a variant thereof having at least about 80% sequence identity to the amino acid sequence of SEQ ID NO: 16. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 17, or a variant thereof having at least about 80% sequence identity to the amino acid sequence of SEQ ID NO: 17. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 18, or a variant thereof having at least about 80% sequence identity to the amino acid sequence of SEQ ID NO: 18. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 18, or a variant thereof having at least about 80% sequence identity to the amino acid sequence of SEQ ID NO: 18. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 18, or a variant thereof having at least about 80% sequence identity to the amino acid sequence of SEQ ID NO: 18. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 19, or a variant thereof having at least about 80% sequence identity to the amino acid sequence of SEQ ID NO: 19. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 20, or a variant thereof having at least about 80% sequence identity to the amino acid sequence of SEQ ID NO: 20. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 20, or a variant thereof having at least about 80% sequence identity to the amino acid sequence of SEQ ID NO: 20. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 22, or a variant thereof having at least about 80% sequence identity to the amino acid sequence of SEQ ID NO: 22. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 21, or a variant thereof having at least about 80% sequence identity to the amino acid sequence of SEQ ID NO: 21. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

In some embodiments, there is provided a full-length antibody specifically binding to MASP3 comprising IgG1 or IgG4 constant regions, wherein the antibody specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22. In some embodiments, the IgG1 is human IgG1. In some embodiments, the heavy chain constant region comprises or consists of the amino acid sequence of SEQ ID NO: 43. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 43 and the light chain constant region comprises or consists of the amino acid sequence of SEQ ID NO: 45. In some embodiments, the heavy chain constant region comprises or consists of the amino acid sequence of SEQ ID NO: 43 and the light chain constant region comprises or consists of the amino acid sequence of SEQ ID NO: 46. In some embodiments, the IgG4 is human IgG4. In some embodiments, the heavy chain constant region comprises or consists of the amino acid sequence of SEQ ID NO: 44. In some embodiments, the light chain constant region comprises or consists of the amino acid sequence of SEQ ID NO: 45. In some embodiments, the light chain constant region comprises or consists of the amino acid sequence of SEQ ID NO: 46. In some embodiments, the heavy chain constant region comprises or consists of the amino acid sequence of SEQ ID NO: 44 and the light chain constant region comprises or consists of the amino acid sequence of SEQ ID NO: 45. In some embodiments, the heavy chain constant region comprises or consists of the amino acid sequence of SEQ ID NO: 44 and the light chain constant region comprises or consists of the amino acid sequence of SEQ ID NO: 46.

### Antibodies specifically binding to MASP2

In some embodiments, the antibody or antigen-binding fragment specifically binding to MASP2 includes, but is not limited to, humanized antibodies, chimeric antibodies, mouse antibodies, human antibodies, and antibodies comprising the heavy chain and/or light chain CDRs discussed herein. In one aspect, the antibodies or antigen-binding fragments are isolated antibodies that bind to MASP2. Contemplated antibodies or antigen-binding fragments specifically binding to MASP2 can include the entire or a fragment of full-length antibodies specifically binding to MASP2 (*e*.*g*., full-length IgG1, IgG2 or IgG4), scFvs specifically binding to MASP2, multi-specific (such as bispecific) antibodies specifically binding to MASP2, immunoconjugates specifically binding to MASP2, and the like. In some embodiments, the antibody or antigen-binding fragment specifically binding to MASP2 is a Fab, a Fab', a F(ab)'2, a Fab'-SH, a single-chain Fv (scFv), an Fv fragment, a dAb, a Fd, a nanobody, a diabody, or a linear antibody. In some embodiments, the antibody or antigen-binding fragment specifically binding to MASP2 means that the antibody or antigen-binding fragment binds to MASP2 with an affinity that is at least about 10 times (including for example at least about any of 10, 10², 10³, 10⁴, 10⁵, 10⁶, or 10⁷ times) of its binding affinity for non-target. In some embodiments, the non-target is an antigen that is not MASP2.

Binding affinity can be determined by methods known in the art, such as ELISA, fluorescence activated cell sorting (FACS) analysis, or radioimmunoprecipitation assay (RIA). Kd can be determined by methods known in the art, such as surface plasmon resonance (SPR) assay or biolayer interferometry (BLI).

Although the antibodies or antigen-binding fragments specifically binding to MASP2 and containing human sequences (*e*.*g*., human heavy and light chain variable domain sequences comprising human CDR sequences) are extensively discussed herein, non-human antibodies are also contemplated. In some embodiments, non-human antibodies comprise human CDR sequences from an antibody or antigen-binding fragment specifically binding to MASP2 described herein and non-human framework sequences. In some embodiments, non-human framework sequences include any sequence that can be used for generating synthetic heavy and/or light chain variable domains using one or more human CDR sequences as described herein, including, *e.g.,* mammals, *e.g.,* mouse, rat, rabbit, pig, bovine *(e.g.,* cow, bull, buffalo), deer, sheep, goat, chicken, cat, dog, ferret, primate *(e.g.,* marmoset, rhesus monkey), *etc.* In some embodiments, a non-human antibody or antigen-binding fragment specifically binding to MASP2 includes an antibody or antigen-binding fragment specifically binding to MASP2 generated by grafting one or more human CDR sequences as described herein onto a non-human framework sequence (*e*.*g*., a mouse or chicken framework sequence).

In some embodiments, the antibody or antigen-binding fragment specifically binding to MASP2 described herein binds specifically to the epitope of human MASP2. In some embodiments, the antibody or antigen-binding fragment specifically binding to MASP2 is completely specific for MASP2 and does not exhibit species cross-reactivity or other types of non-MASP2 cross-reactivity. In some embodiments, the antibody or antigen-binding fragment specifically binding to MASP2 exhibits MASP2 antigen of species other than human cross-reactivity.

In some embodiments, the antibody or antigen-binding fragment specifically binding to MASP2 comprises antibody heavy chain constant region and antibody light chain constant region. In some embodiments, the antibody or antigen-binding fragment specifically binding to MASP2 comprises IgG1 heavy chain constant region. In some embodiments, the antibody or antigen-binding fragment specifically binding to MASP2 comprises IgG2 heavy chain constant region. In some embodiments, the antibody or antigen-binding fragment specifically binding to MASP2 comprises IgG3 heavy chain constant region. In some embodiments, the antibody or antigen-binding fragment specifically binding to MASP2 comprises IgG4 heavy chain constant region. In some embodiments, the IgG1 refers to human IgG. In some embodiments, the heavy chain constant region comprises or consists of the amino acid sequence of SEQ ID NO: 43. In some embodiments, the heavy chain constant region comprises or consists of the amino acid sequence of SEQ ID NO: 44. In some embodiments, the antibody or antigen-binding fragment specifically binding to MASP2 comprises a kappa light chain constant region. In some embodiments, the light chain constant region comprises or consists of the amino acid sequence of SEQ ID NO: 45. In some embodiments, the antibody or antigen-binding fragment specifically binding to MASP2 comprises a lambda light chain constant region. In some embodiments, the light chain constant region comprises or consists of the amino acid sequence of SEQ ID NO: 46. In some embodiments, the antibody or antigen-binding fragment specifically binding to MASP2 comprises antibody heavy chain variable domain and antibody light chain variable domain. In some embodiments, the full-length antibody specifically binding to MASP2 comprises an altered or otherwise altered Fc sequence, resulting in enhanced antibody-dependent cell-mediated cytotoxicity (ADCC) and complement dependent cytotoxicity (CDC) effector functions.

### Multi-specific antibodies specifically binding to MASP3 and MASP2

In one aspect, the present application provides a multi-specific antibody comprising a first antigen-binding domain specifically binding to MASP3, and a second antigen-binding domain specifically binding to MASP2. In some embodiments, multi-specific antibody can bind to MASP3 or MASP2. In other preferred embodiments, the multi-specific antibody can bind to both MASP3 and MASP2. In other preferred embodiments, the multi-specific antibody can bind to MASP3 and MASP2 simultaneously.

In some embodiments, the first antigen-binding domain specifically binding to MASP3 comprises: (a) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); or (b) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a light chain variable domain (V_{L}) comprising a light chain complementarity determining region (LC-CDR) 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

In some embodiments, the second antigen-binding domain specifically binding to MASP2 comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); or (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, the first antigen-binding domain specifically binding to MASP3 comprises: a V_{H} comprises an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprises an LC-CDR1 comprising the amino acid sequence of any one of SEQ ID NOs: 4-5, or a variant thereof comprising up to about 3 amino acid substitutions (*e*.*g*., 1, 2, or 3) , an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions (*e*.*g*., 1, 2, or 3).

In some embodiments, the second antigen-binding domain specifically binding to MASP2 comprises: a V_{H} comprises an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprises an LC-CDR1 comprising the amino acid sequence of any one of SEQ ID NO: 26, an LC-CDR2 comprising the amino acid sequence of SEQ ID NO: 27, and an LC-CDR3 comprising the amino acid sequence of any one of SEQ ID NOs: 28-32, or a variant thereof comprising up to about 3 amino acid substitutions (*e*.*g*., 1, 2, or 3).

In some embodiments, the first antigen-binding domain specifically binding to MASP3 comprises: a V_{H} comprising an HC-CDR1, an HC-CDR2, and an HC-CDR3 of a V_{H} comprising the amino acid sequence of any one of SEQ ID NOs: 11-15; and a V_{L} comprising an LC-CDR1, an LC-CDR2, and an LC-CDR3 of a V_{L} comprising the amino acid sequence of any one of SEQ ID NOs: 16-22.

In some embodiments, the second antigen-binding domain specifically binding to MASP2 comprises: a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of any one of SEQ ID NOs: 36-41.

In some embodiments, the first antigen-binding domain specifically binding to MASP3 comprises: (a) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 16; or (ii) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19.

In some embodiments, the second antigen-binding domain specifically binding to MASP2 comprises: (a) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 36; (b) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 37; (c) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 38; (d) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 39; (e) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 40; or (f) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 41.

In some embodiments, the first antigen-binding domain specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7. In some embodiments, the first antigen-binding domain specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

In some embodiments, the second antigen-binding domain specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28. In some embodiments, the second antigen-binding domain specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29. In some embodiments, the second antigen-binding domain specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30. In some embodiments, the second antigen-binding domain specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31. In some embodiments, the second antigen-binding domain specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

In some embodiments, the first antigen-binding domain specifically binding to MASP3 comprises: a V_{H} comprising the amino acid sequence of any one of SEQ ID NOs: 11-15, or a variant thereof having at least about 80% (*e.g.,* at least 80%, 85%, 90%, 95%, 96%, 97%, 98%, or 99%) sequence identity to the amino acid sequence of any one of SEQ ID NOs: 11-15; and a V_{L} comprising the amino acid sequence of any one of SEQ ID NOs: 16-22, or a variant thereof having at least about 80% (*e.g.,* at least 80%, 85%, 90%, 95%, 96%, 97%, 98%, or 99%) sequence identity to the amino acid sequence of any one of SEQ ID NOs: 16-22. In some embodiments, the first antigen-binding domain specifically binding to MASP3 comprises: a V_{H} comprising the amino acid sequence of any one of SEQ ID NOs: 11-15; and a V_{L} comprising the amino acid sequence of any one of SEQ ID NOs: 16-22.

In some embodiments, the first antigen-binding domain specifically binding to MASP3 comprises: (a) a V_{H} comprising the amino acid sequence of SEQ ID NO: 13, or a variant thereof having at least about 80% *(e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 17, or a variant thereof having at least about 80% (*e.g.,* at least 80%, 85%, 90%, 95%, 96%, 97%, 98%, or 99%) sequence identity to the amino acid sequence of SEQ ID NO: 17; (b) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 17, or a variant thereof having at least about 80% (*e.g.,* at least 80%, 85%, 90%, 95%, 96%, 97%, 98%, or 99%) sequence identity to the amino acid sequence of SEQ ID NO: 17; (c) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 20, or a variant thereof having at least about 80% (*e.g.,* at least 80%, 85%, 90%, 95%, 96%, 97%, 98%, or 99%) sequence identity to the amino acid sequence of SEQ ID NO: 20; (d) a V_{H} comprising the amino acid sequence of SEQ ID NO: 12, or a variant thereof having at least about 80% (*e.g.,* at least 80%, 85%, 90%, 95%, 96%, 97%, 98%, or 99%) sequence identity to the amino acid sequence of SEQ ID NO: 12; and a V_{L} comprising the amino acid sequence of SEQ ID NO: 21, or a variant thereof having at least about 80% (*e.g.,* at least 80%, 85%, 90%, 95%, 96%, 97%, 98%, or 99%) sequence identity to the amino acid sequence of SEQ ID NO: 21; (e) a V_{H} comprising the amino acid sequence of SEQ ID NO: 13, or a variant thereof having at least about 80% (*e.g.,* at least 80%, 85%, 90%, 95%, 96%, 97%, 98%, or 99%) sequence identity to the amino acid sequence of SEQ ID NO: 13; and a V_{L} comprising the amino acid sequence of SEQ ID NO: 21, or a variant thereof having at least about 80% (*e.g.,* at least 80%, 85%, 90%, 95%, 96%, 97%, 98%, or 99%) sequence identity to the amino acid sequence of SEQ ID NO: 21; or (f) a V_{H} comprising the amino acid sequence of SEQ ID NO: 14, or a variant thereof having at least about 80% (*e.g.,* at least 80%, 85%, 90%, 95%, 96%, 97%, 98%, or 99%) sequence identity to the amino acid sequence of SEQ ID NO: 14; and a V_{L} comprising the amino acid sequence of SEQ ID NO: 22, or a variant thereof having at least about 80% (*e.g.,* at least 80%, 85%, 90%, 95%, 96%, 97%, 98%, or 99%) sequence identity to the amino acid sequence of SEQ ID NO: 22.

In some embodiments, the second antigen-binding domain specifically binding to MASP2 comprises: a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of any one of SEQ ID NOs: 36-41, or a variant thereof having at least about 80% (*e.g.,* at least 80%, 85%, 90%, 95%, 96%, 97%, 98%, or 99%) sequence identity to the amino acid sequence of any one of SEQ ID NOs: 36-41. In some embodiments, the second antigen-binding domain specifically binding to MASP2 comprises: a V_{H} comprising the amino acid sequence of SEQ ID NO: 34; and a V_{L} comprising the amino acid sequence of any one of SEQ ID NOs: 36-41.

In some embodiments, the second antigen-binding domain specifically binding to MASP2 comprises: (a) a V_{H} comprising the amino acid sequence of SEQ ID NO: 34, or a variant thereof having at least about *80%* (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 36, or a variant thereof having at least about 80% (*e.g.,* at least 80%, 85%, 90%, 95%, 96%, 97%, 98%, or 99%) sequence identity to the amino acid sequence of SEQ ID NO: 36; (b) a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 37, or a variant thereof having at least about 80% *(e.g.,* at least 80%, 85%, 90%, 95%, 96%, 97%, 98%, or 99%) sequence identity to the amino acid sequence of SEQ ID NO: 37; (c) a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 38, or a variant thereof having at least about 80% (*e.g.,* at least 80%, 85%, 90%, 95%, 96%, 97%, 98%, or 99%) sequence identity to the amino acid sequence of SEQ ID NO: 38; (d) a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 39, or a variant thereof having at least about 80% (*e.g.,* at least 80%, 85%, 90%, 95%, 96%, 97%, 98%, or 99%) sequence identity to the amino acid sequence of SEQ ID NO: 39; (e) a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 40, or a variant thereof having at least about 80% (*e.g.,* at least 80%, 85%, 90%, 95%, 96%, 97%, 98%, or 99%) sequence identity to the amino acid sequence of SEQ ID NO: 40; or (f) a V_{H} comprising the amino acid sequence of SEQ ID NO: 34, or a variant thereof having at least about 80% (*e.g.,* at least 80%, 85%, 90%, 95%, 96%, 97%, 98%, or 99%) sequence identity to the amino acid sequence of SEQ ID NO: 34; and a V_{L} comprising the amino acid sequence of SEQ ID NO: 41, or a variant thereof having at least about 80% (*e.g.,* at least 80%, 85%, 90%, 95%, 96%, 97%, 98%, or 99%) sequence identity to the amino acid sequence of SEQ ID NO: 41.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 3; and a V_{L} comprising: an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8; and wherein the second antigen-binding domain comprises: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising: an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 29.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the present application provides a multi-specific antibody comprising a first antigen-binding domain specifically binding to MASP3, and a second antigen-binding domain specifically binding to MASP2, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

### DVD-Ig format multi-specific antibodies

In some embodiments, any one of the multi-specific antibody (*e*.*g*., bispecific antibody) described herein has a dual variable region immunoglobulin molecule (DVD-Ig) format, connecting the V_{L} and V_{H} domains of another antibody respectively to the N-terminus of the V_{L} and V_{H} of a normal IgG antibody, forming an antigen-binding domain by the interaction between V_{H} and V_{L} of two antibodies, which can simultaneously bind to the corresponding antigen to achieve bispecific binding. In some embodiments, the DVD-IgG format is a homodimeric structure, consisting of two identical monomers, each monomer comprising two antigen binding domains, one of which is Fv and the other of which is Fab. The two domains described above are connected in tandem through a peptide linker (L). In some embodiments, the DVD-Ig format further comprises an Fc. An exemplary schematic diagram of DVD-Ig format is shown in FIG. 2A.

In one embodiment of the application, one of the antigen-binding domains specifically binds to MASP2 and the other antigen-binding domain specifically binds to MASP3. In some embodiments, the multi-specific antibody can bind to MASP3 and MASP2 simultaneously.

In some embodiments, the multi-specific antibody consists of two identical monomers, each of which comprises two polypeptide chains, a heavy chain and a light chain, for a total of four polypeptide chains. Wherein, the heavy chain comprises V_{H}1-L-V_{H}2-C_{H}1 from N- terminus to C-terminus, the light chain comprises V_{L}1-L-V_{L}2-C_{L} from N- terminus to C-terminus. In some embodiments, the heavy chain further comprises an Fc comprising C_{H}2 and C_{H}3 domains. In other embodiments, the heavy chain comprises V_{H}1-L-V_{H}2-C_{H}1-C_{H}2-C_{H}3 from N- terminus to C-terminus, the light chain comprises V_{L}1-L-V_{L}2-C_{L} from N-terminus to C-terminus. Wherein the V_{H}1 and V_{L}1 are the heavy chain variable domain and light chain variable domain that specifically bind to one of the antigens, respectively; the VH₂ and VL₂ are the heavy chain variable domain and light chain variable domain that specifically bind to another antigen, respectively; L is a peptide linker; C_{H}1 is a heavy chain constant domain 1; C_{L} is a light chain constant domain. Wherein, the V_{H}1 and V_{L}1 form one of the antigen-binding domains (Fv) of the multi-specific antibody; the V_{H}2-C_{H}1 and V_{L}2-C_{L} form the other antigen-binding domain (Fab) of the multi-specific antibody.

In some embodiments, the V_{H}1 and V_{L}1 are the heavy chain variable domain and light chain variable domain that specifically bind to MASP2, respectively, *i.e.,* the V_{H}1 and V_{L}1 form the antigen-binding domain (Fv) specifically binding to MASP2; the V_{H}2 and V_{L}2 are the heavy chain variable domain and light chain variable domain that specifically bind to MASP3, respectively, *i.e.,* the V_{H}2-C_{H}1 and V_{L}2-C_{L} form the antigen-binding domain (Fab) specifically binding to MASP3. In some embodiments, the V_{H}1 and V_{L}1 are the heavy chain variable domain and light chain variable domain that specifically bind to MASP3, respectively, *i.e.,* the V_{H}1 and V_{L}1 form the antigen-binding domain (Fv) specifically binding to MASP3; the V_{H}2 and V_{L}2 are the heavy chain variable domain and light chain variable domain that specifically bind to MASP2, respectively, *i.e.,* the V_{H}2-C_{H}1 and V_{L}2-C_{L} form the antigen-binding domain (Fab) specifically binding to MASP2.

Peptide linker (or linker) sequence can be single amino acid or polypeptide sequence. In some embodiments, the peptide linker (or linker) comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer *(e.g.,* 1, 2, 3, 4, 5, 6, 7, 8, 9 or 10). A preferred Gly-Ser linker is (Gly₄Ser)₂, *i.e.,* GGGGSGGGGS(SEQ ID NO: 63), and (Gly₄Ser)₄, *i.e.,* GGGGSGGGGSGGGGSGGGGS(SEQ ID NO: 64). Another preferred Gly-Ser linker is (Gly₄Ser)₃, *i.e.,,* GGGGSGGGGSGGGGS (SEQ ID NO: 65). In other aspects, two or more Gly-Ser linker are incorporated in series in a peptide linker. In some aspects, the peptide linker comprises at least a portion of a hinge region (*e*.*g*., derived from an IgG1, IgG2, IgG3, or IgG4 molecule) and a series of Gly- Ser amino acid residues (*e*.*g*., a Gly-Ser linker such as (Gly₄Ser)ₙ). In some embodiments, the peptide linker can also be selected to comprise amino acid sequence ASTKGP (SEQ ID NO: 66) or amino acid sequence TVAAP(SEQ ID NO: 67).

### Bs4Ab format multi-specific antibodies

In some embodiments, the multi-specific antibody (*e*.*g*., bispecific antibody) described herein has Bs4Ab format, which consists of two identical monomers, each monomer comprising two antigen-binding domains, one of which is Fab and the other of which is scFv. In some embodiments, the Bs4Ab format bispecific antibody further comprises an Fc comprising C_{H}2 and C_{H}3 domains. The scFv connects to the Fab through the first peptide linker (L1) and the Fc through the second peptide linker (L2). An exemplary schematic diagram of Bs4Ab format is shown in FIG. 2B.

In some embodiments of the application, one of the antigen-binding domains (Fab or scFv) specifically binds to MASP2 and the other antigen-binding domain (scFv or Fab) specifically binds to MASP3. In some embodiments, the multi-specific antibody can bind to MASP3 and MASP2 simultaneously.

In some embodiments, the multi-specific antibody described herein consists of two identical monomers, each of which comprises two polypeptide chains, a heavy chain and a light chain, for a total of four polypeptide chains. In some embodiments, the heavy chain of the multi-specific antibody comprises V_{H}1-C_{H}1-L1-V_{H}2-L3-V_{L}2 structure from N- terminus to C-terminus. In other embodiments, the heavy chain of the multi-specific antibody comprises V_{H}1-C_{H}1-L1-V_{L}2-L3-V_{H}2 structure from N- terminus to C-terminus. In some embodiments, the heavy chain further comprises an Fc comprising C_{H}2 and C_{H}3 domains. In some embodiments, the heavy chain of the multi-specific antibody comprises V_{H}1-C_{H}1-L1-V_{H}2-L3-V_{L}2-L2-C_{H}2-C_{H}3 structure from N- terminus to C-terminus. In other embodiments, the heavy chain of the multi-specific antibody comprises V_{H}1-C_{H}1-L1-V_{L}2-L3-V_{H}2-L2-C_{H}2-C_{H}3 structure from N-terminus to C-terminus. In some embodiments, the light chain of the multi-specific antibody comprises V_{L}1-C_{L} structure from N- terminus to C-terminus. Wherein, the V_{H}1 and V_{L}1 are the heavy chain variable domain and light chain variable domain that specifically bind to one of the antigens, respectively; the V_{H}2 and V_{L}2 are the heavy chain variable domain and light chain variable domain that specifically bind to the other antigen, respectively; C_{H}1 is a heavy chain constant domain 1; C_{L} is a light chain constant domain; L1, L2 and L3 are peptide linkers. The V_{H}1-C_{H}1 and V_{L}1-C_{L} form one of the antigen-binding domains (Fab) of the multi-specific antibody; the V_{H}2-L3-V_{L}2 or V_{L}2-L3-V_{H}2 form the other antigen-binding domain (scFv) of the multi-specific antibody.

In some embodiments, the V_{H}1 and V_{L}1 are the heavy chain variable domain and light chain variable domain that specifically bind to MASP2, respectively, *i.e.,* the V_{H}1-C_{H}1 and V_{L}1-C_{L} form the antigen-binding domain (Fab) specifically binding to MASP2; the V_{H}2 and V_{L}2 are the heavy chain variable domain and light chain variable domain that specifically bind to MASP3, respectively, *i.e.,* the V_{H}2-L3-V_{L}2 or V_{L}2-L3-V_{H}2 form the antigen-binding domain (scFv) specifically binding to MASP3. In other embodiments, the V_{H}1 and V_{L}1 are the heavy chain variable domain and light chain variable domain that specifically bind to MASP3, respectively, *i.e.,* the V_{H}1-C_{H}1 and V_{L}1-C_{L} form the antigen-binding domain (Fab) specifically binding to MASP3; the V_{H}2 and V_{L}2 are the heavy chain variable domain and light chain variable domain that specifically bind to MASP2, respectively, *i.e.,* the V_{H}2-L3-V_{L}2 or V_{L}2-L3-V_{H}2 form the antigen-binding domain (scFv) specifically binding to MASP2. In some embodiments, the multi-specific antibody comprises C_{H}1 comprising the amino acid sequence of SEQ ID No: 49 or 50. In some embodiments, the multi-specific antibody comprises C_{H}2-C_{H}3 comprising the amino acid sequence of SEQ ID No: 47 or 48. In some embodiments, the multi-specific antibody comprises C_{H}2-C_{H}3 containing one or more substitutions at positions 22, 24, 26 relative to the amino acid sequence of SEQ ID No: 47 or 48. In some embodiments, the multi-specific antibody comprises C_{H}2-C_{H}3 containing one or more substitutions of M22Y, S24T, T26E relative to the amino acid sequence of SEQ ID No: 47 or 48. In some embodiments, the multi-specific antibody comprises C_{H}2-C_{H}3 containing one or more substitutions at positions 198, 204 relative to the amino acid sequence of SEQ ID No: 47 or 48. In some embodiments, the multi-specific antibody comprises C_{H}2-C_{H}3 containing one or more substitutions of M198L, N204S relative to the amino acid sequence of SEQ ID No: 47 or 48. In some embodiments, the multi-specific antibody comprises C_{H}2-C_{H}3 containing one or more substitutions at positions 4, 5 relative to the amino acid sequence of SEQ ID No: 47. In some embodiments, the multi-specific antibody comprises C_{H}2-C_{H}3 containing one or more substitutions of L4A, L5A relative to the amino acid sequence of SEQ ID No: 47.

In some embodiments, the antigen-binding domain scFv specifically binding to MASP3 or MASP2 comprises genetic engineering cysteine mutations. Multi-specific antibodies with disulfide bond stability are obtained through introducing two cysteine mutations at the V_{H} and V_{L} interfaces.

Peptide linker (or linker) may be used to join domains and/or regions of the chimeric heavy chain of the multi-specific antibody into a contiguous molecule. The multi-specific antibody may include additional linkers, such as a flexible linker interconnecting the variable heavy and light chains of an scFv. In some embodiments, the multi-specific antibody may include additional linkers, such as a flexible linker interconnecting the variable heavy and light chains of an scFv and other linkers that connect other binding units to the core structure of the multi-specific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (*e*.*g*., the same or different length and/or amino acid sequence).

In some aspects, the peptide linker comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (*e.g*., 1, 2, 3, 4, 5, 6, 7, 8, 9 or 10). A preferred Gly-Ser linker is (Gly₄Ser)₂, i.e., GGGGSGGGGS(SEQ ID NO: 63), and (Gly₄Ser)₄, i.e., GGGGSGGGGSGGGGSGGGGS(SEQ ID NO: 64). Another preferred Gly-Ser linker is (Gly₄Ser)₃, i.e.,, GGGGSGGGGSGGGGS (SEQ ID NO: 65). In other aspects, two or more Gly-Ser linker are incorporated in series in a peptide linker. In some aspects, the peptide linker comprises at least a portion of a hinge region (*e*.*g*., derived from an IgG1, IgG2, IgG3, or IgG4 molecule) and a series of Gly-Ser amino acid residues (*e*.*g*., a Gly-Ser linker such as (G₄S)ₙ).

In some embodiments, L1 and/or L2 include both a hinge portion and a linker portion, such as a linker portion comprising a Gly-Ser linker. In other aspects, L1 and/or L2 include only a hinge portion or only a linker portion, such as a Gly-Ser linker. In some embodiments, L1 and L2 include a Gly-Ser linker portion. In certain aspects, the Gly-Ser linker portion of L1 and L2 is the same length, whereas in other aspects, the Gly-Ser linker portion of LI and L2 are different lengths. When a multi-specific molecule comprises an scFv, the heavy and light chains of the scFv may be connected by a flexible linker. In some embodiments, this flexible linker generally does not include a hinge portion, but rather, is a Gly-Ser linker or other flexible linker. The length and amino acid sequence of a flexible linker interconnecting domains of an scFv may be selected and optimized.

In some embodiments, the peptide linker (for example L1 and/or L2) comprises a Gly-Ser or all Gly linker and a portion or modified portion of a hinge domain. In some aspects, the peptide linker (L1) connecting one of the antigen-binding domains (*e.g*. the Fab or scFv) to the other antigen-binding domain (*e.g.* scFv or Fab) of the multi-specific antibody comprises the amino acid sequence EPKSDKTGGGGSGGGGS (SEQ ID NO: 68) or EPKSCGKTGGGGSGGGGS (SEQ ID NO: 69) or EPKSCGGGGSGGGGS (SEQ ID NO: 70). In some aspects the peptide linker (L2) connecting the antigen-binding domain scFv to the Fc domain of the bispecific antibody comprises the amino acid sequence GGGGSGGGGSEPKSDKTHTCPPCP (SEQ ID NO: 71) or GGGGSGGGGSCPPCP (SEQ ID NO: 72) or GGGGSGGGGSDKTHTCPPCP (SEQ ID NO: 73).

In some embodiments, regardless of the peptide linker used to interconnect one antigen-binding domain to the other antigen-binding domain or one of the antigen-binding domains to Fc (*e.g.,* L1 and L2), the bispecific antibody may optionally comprise additional peptide linkers. The lengths and sequence of such additional peptide linkers are independently selected. For example, the bispecific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 64).

### Hetero H, CrossMab format multi-specific antibodies

In some embodiments, any one of the multi-specific antibodies (*e*.*g*., bispecific antibodies) described herein has Hetero H, CrossMab structure, which is a bivalent multi-specific antibody consisting of heterodimer, comprising two antigen-binding domains Fab. In other embodiments, the multi-specific antibody further comprises two Fc domains comprising C_{H}2 and C_{H}3 domains. In some embodiments, the amino acid residue in C_{H}3 domain of one of the Fc is substituted with a larger side chain volume amino acid residue to form a "knob", the amino acid residue in C_{H}3 domain of the other Fc is substituted with a smaller side chain volume amino acid residue to form a "hole", which can promote the binding of heterodimers. Wherein, in the Fab arm, the location of the light chain constant domain (C_{L}) and the heavy chain constant domain 1 (C_{H}1) can be exchanged with each other; or the location of the heavy chain variable domain (V_{H} ) and the light chain variable domain (V_{L}) can be exchanged with each other; or the light chain constant domain (C_{L}) and the heavy chain constant domain 1(C_{H}1) as well as the heavy chain variable domain (V_{H} ) and the light chain variable domain (V_{L}) can be exchanged with each other simultaneously. An exemplary schematic diagram of the Hetero H, CrossMab format is shown in FIG. 2C.

In some embodiments, the Fc is derived from wild-type human IgG1 Fc. In other embodiments, the C_{H}2 domain of the Fc comprises, but is not limited to the amino acid substitution described below: L234A, L235A, wherein the numbering is according to EU index of Kabat. In other embodiments, the C_{H}3 domain of the Fc comprises, but is not limited to the amino acid substitution described below: S354C, T366W, Y349C, T366S, L368A and Y407V, wherein the numbering is according to EU index of Kabat.

In other embodiments of the application, one of the antigen-binding domains specifically binds to MASP3 and the other antigen-binding domain specifically binds to MASP2. In some embodiments, the multi-specific antibody can bind to MASP3 and MASP2 simultaneously.

In some embodiments, the multi-specific antibody described herein has Hetero H, CrossMab format, which is a heterodimer consisting of two different monomers, each of which comprises two polypeptide chains. Wherein the first monomer comprises the first heavy chain and the first light chain specifically binding to one of the antigens, and the second monomer comprises the second heavy chain and the second light chain specifically binding to the other antigen. In some embodiments, the first heavy chain of the multi-specific antibody comprises V_{H}1-C_{H}1 structure from N- terminus to C-terminus; the first light chain of the multi-specific antibody comprises V_{L}1-C_{L} structure from N- terminus to C-terminus. In some embodiments, the first heavy chain further comprises an Fc comprising C_{H}2 and C_{H}3 domains. In some embodiments, the first heavy chain of the multi-specific antibody comprises V_{H}1-C_{H}1-C_{H}2-C_{H}3 structure from N- terminus to C-terminus; the first light chain of the multi-specific antibody comprises V_{L}1-C_{L} structure from N- terminus to C-terminus. The V_{H}1 and V_{L}1 are the heavy chain variable domain and light chain variable domain that specifically bind to one of the antigens, respectively, C_{H}1 is a heavy chain constant domain 1, C_{L} is a light chain constant domain. Wherein, the V_{H}1-C_{H}1 and V_{L}1-C_{L} form the antigen-binding domain (Fab) binding to one of the antigens. In some embodiments, the second heavy chain of the multi-specific antibody comprises V_{H}2-C_{L} structure from N- terminus to C-terminus; the second light chain of the multi-specific antibody comprises V_{L}2-C_{H}1 structure from N- terminus to C-terminus. In some embodiments, the second heavy chain further comprises an Fc comprising C_{H}2 and C_{H}3 domains. In some embodiments, the second heavy chain of the multi-specific antibody comprises V_{H}2-C_{L}-C_{H}2-C_{H}3 structure from N- terminus to C-terminus; the second light chain of the multi-specific antibody comprises V_{L}2-C_{H}1 structure from N- terminus to C-terminus. The V_{H}2 and V_{L}2 are the heavy chain variable domain and light chain variable domain that specifically bind to the other antigen, respectively, C_{H}1 is a heavy chain constant domain 1, C_{L} is a light chain constant domain. Wherein, the V_{H}2-C_{L} and V_{L}2-C_{H}1 form the antigen-binding domain (Fab) binding to the other antigen. In some embodiments, the location of C_{L} and C_{H}1 of the first monomer or the second monomer of the multi-specific antibody can be exchanged with each other. In some embodiments, the location of V_{H}1 and V_{L}1 can be exchanged with each other. In other embodiments, the location of V_{H}2 and V_{L}2 can be exchanged with each other. In some embodiments, the amino acid residue in C_{H}3 domain of the first heavy chain is substituted with a larger side chain volume amino acid residue to form a "knob", and the amino acid residue in C_{H}3 domain of the second heavy chain is substituted with a smaller side chain volume amino acid residue to form a "hole". In some embodiments, the amino acid residue in C_{H}3 domain of the second heavy chain is substituted with a larger side chain volume amino acid residue to form a "knob", and the amino acid residue in C_{H}3 domain of the first heavy chain is substituted with a smaller side chain volume amino acid residue to form a "hole". In some embodiments, the C_{H}3 domain comprises, but is not limited to the amino acid substitution described below: S354C, T366W, Y349C, T366S, L368A and Y407V, wherein the numbering is according to EU index of Kabat.

In some embodiments, the V_{H}1 and V_{L}1 are the heavy chain variable domain and light chain variable domain that specifically bind to MASP2, respectively, the V_{H}2 and V_{L}2 are the heavy chain variable domain and light chain variable domain that specifically bind to MASP3, respectively, *i.e.,* the V_{H}1-C_{H}1 and V_{L}1-C_{L} form the antigen-binding domain (Fab) specifically binding to MASP2, V_{H}2-C_{L} and V_{L}2-C_{H}1 form the antigen-binding domain (Fab) specifically binding to MASP3. In another embodiment, the V_{H}1 and V_{L}1 are the heavy chain variable domain and light chain variable domain that specifically bind to MASP3, respectively, the V_{H}2 and V_{L}2 are the heavy chain variable domain and light chain variable domain that specifically bind to MASP2, respectively, *i.e.,* the V_{H}1-C_{H}1 and V_{L}1-C_{L} form the antigen-binding domain (Fab) specifically binding to MASP3, V_{H}2-C_{L} and V_{L}2-C_{H}1 form the antigen-binding domain (Fab) specifically binding to MASP2.

In some embodiments, according to the multi-specific antibodies described herein comprises the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52.

In some embodiments, according to the multi-specific antibodies described herein comprises the amino acid sequence of SEQ ID NO: 53, or a variant thereof having at least about 80% (*e.g.,* at least 80%, 85%, 90%, 95%, 96%, 97%, 98%, or 99%) sequence identity to the amino acid sequence of SEQ ID NO: 53.

In some embodiments, according to the multi-specific antibodies described herein comprises the amino acid sequence of SEQ ID NO: 54, or a variant thereof having at least about 80% (*e.g.,* at least 80%, 85%, 90%, 95%, 96%, 97%, 98%, or 99%) sequence identity to the amino acid sequence of SEQ ID NO:54.

In some embodiments, according to the multi-specific antibodies described herein comprises the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52.

In some embodiments, according to the multi-specific antibodies described herein comprises the amino acid sequence of SEQ ID NO: 56, or a variant thereof having at least about 80% (*e.g.,* at least 80%, 85%, 90%, 95%, 96%, 97%, 98%, or 99%) sequence identity to the amino acid sequence of SEQ ID NO: 56.

In some embodiments, according to the multi-specific antibodies described herein comprises the amino acid sequence of SEQ ID NO: 55, or a variant thereof having at least about 80% (*e.g.,* at least 80%, 85%, 90%, 95%, 96%, 97%, 98%, or 99%) sequence identity to the amino acid sequence of SEQ ID NO: 55.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52.

In some embodiments, according to the multi-specific antibodies described herein comprises the amino acid sequence of SEQ ID NO: 56, or a variant thereof having at least about 80% (*e.g.,* at least 80%, 85%, 90%, 95%, 96%, 97%, 98%, or 99%) sequence identity to the amino acid sequence of SEQ ID NO: 56.

In some embodiments, according to the multi-specific antibodies described herein comprises the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51.

In some embodiments, according to the multi-specific antibodies described herein comprises the amino acid sequence of SEQ ID NO: 57, or a variant thereof having at least about 80% (*e.g.,* at least 80%, 85%, 90%, 95%, 96%, 97%, 98%, or 99%) sequence identity to the amino acid sequence of SEQ ID NO: 57.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52.

In some embodiments, according to the multi-specific antibodies described herein comprises the amino acid sequence of SEQ ID NO: 58, or a variant thereof having at least about 80% (*e.g.,* at least 80%, 85%, 90%, 95%, 96%, 97%, 98%, or 99%) sequence identity to the amino acid sequence of SEQ ID NO: 58.

In some embodiments, according to the multi-specific antibodies described herein comprises the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51.

In some embodiments, according to the multi-specific antibodies described herein comprises the amino acid sequence of SEQ ID NO: 53, or a variant thereof having at least about 80% (*e.g.,* at least 80%, 85%, 90%, 95%, 96%, 97%, 98%, or 99%) sequence identity to the amino acid sequence of SEQ ID NO: 53.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 54, or a variant thereof having at least about 80% (*e.g.,* at least 80%, 85%, 90%, 95%, 96%, 97%, 98%, or 99%) sequence identity to the amino acid sequence of SEQ ID NO: 54; and/or the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51; and /or the amino acid sequence of SEQ ID NO: 53, or a variant thereof having at least about 80% (*e.g.,* at least 80%, 85%, 90%, 95%, 96%, 97%, 98%, or 99%) sequence identity to the amino acid sequence of SEQ ID NO: 53.

In some embodiments, according to the multi-specific antibodies described herein comprises the amino acid sequence of SEQ ID NO: 52, or a variant thereof having at least about 80% *(e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 56, or a variant thereof having at least about 80% *(e.g.,* at least 80%, 85%, 90%, 95%, 96%, 97%, 98%, or 99%) sequence identity to the amino acid sequence of SEQ ID NO: 56; and/or the amino acid sequence of SEQ ID NO: 51, or a variant thereof having at least about 80% *(e.g.,* at least 80%, 85%, 90%, 95%, 96%, 97%, 98%, or 99%) sequence identity to the amino acid sequence of SEQ ID NO: 51; and /or the amino acid sequence of SEQ ID NO: 55, or a variant thereof having at least about 80% *(e.g.,* at least 80%, 85%, 90%, 95%, 96%, 97%, 98%, or 99%) sequence identity to the amino acid sequence of SEQ ID NO: 55.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 56, or a variant thereof having at least about 80% (*e.g.,* at least 80%, 85%, 90%, 95%, 96%, 97%, 98%, or 99%) sequence identity to the amino acid sequence of SEQ ID NO: 56; and/or the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51; and /or the amino acid sequence of SEQ ID NO: 57, or a variant thereof having at least about 80% (*e.g.,* at least 80%, 85%, 90%, 95%, 96%, 97%, 98%, or 99%) sequence identity to the amino acid sequence of SEQ ID NO: 57.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 58, or a variant thereof having at least about 80% (*e.g.,* at least 80%, 85%, 90%, 95%, 96%, 97%, 98%, or 99%) sequence identity to the amino acid sequence of SEQ ID NO: 58; and/or the amino acid sequence of SEQ ID NO: 51, or a variant thereof having at least about 80% (*e.g.,* at least 80%, 85%, 90%, 95%, 96%, 97%, 98%, or 99%) sequence identity to the amino acid sequence of SEQ ID NO: 51; and /or the amino acid sequence of SEQ ID NO: 53, or a variant thereof having at least about 80% (*e.g.,* at least 80%, 85%, 90%, 95%, 96%, 97%, 98%, or 99%) sequence identity to the amino acid sequence of SEQ ID NO: 53.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 54, or a variant thereof having at least about 80% (*e.g.,* at least 80%, 85%, 90%, 95%, 96%, 97%, 98%, or 99%) sequence identity to the amino acid sequence of SEQ ID NO: 54; and/or the amino acid sequence of SEQ ID NO: 59, or a variant thereof having at least about 80% (*e.g.,* at least 80%, 85%, 90%, 95%, 96%, 97%, 98%, or 99%) sequence identity to the amino acid sequence of SEQ ID NO: 59; and /or the amino acid sequence of SEQ ID NO: 60, or a variant thereof having at least about 80% *(e.g.,* at least 80%, 85%, 90%, 95%, 96%, 97%, 98%, or 99%) sequence identity to the amino acid sequence of SEQ ID NO: 60.

In some embodiments, according to the multi-specific antibodies described herein comprises the amino acid sequence of SEQ ID NO: 52, or a variant thereof having at least about 80% *(e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 56, or a variant thereof having at least about 80% *(e.g.,* at least 80%, 85%, 90%, 95%, 96%, 97%, 98%, or 99%) sequence identity to the amino acid sequence of SEQ ID NO: 56; and/or the amino acid sequence of SEQ ID NO: 59, or a variant thereof having at least about 80% *(e.g.,* at least 80%, 85%, 90%, 95%, 96%, 97%, 98%, or 99%) sequence identity to the amino acid sequence of SEQ ID NO: 59; and /or the amino acid sequence of SEQ ID NO: 61, or a variant thereof having at least about 80% (*e.g.,* at least 80%, 85%, 90%, 95%, 96%, 97%, 98%, or 99%) sequence identity to the amino acid sequence of SEQ ID NO: 61.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 56, or a variant thereof having at least about 80% (*e.g.,* at least 80%, 85%, 90%, 95%, 96%, 97%, 98%, or 99%) sequence identity to the amino acid sequence of SEQ ID NO: 56; and/or the amino acid sequence of SEQ ID NO: 59, or a variant thereof having at least about 80% (*e.g.,* at least 80%, 85%, 90%, 95%, 96%, 97%, 98%, or 99%) sequence identity to the amino acid sequence of SEQ ID NO: 59; and /or the amino acid sequence of SEQ ID NO: 62, or a variant thereof having at least about 80% (*e.g.,* at least 80%, 85%, 90%, 95%, 96%, 97%, 98%, or 99%) sequence identity to the amino acid sequence of SEQ ID NO: 62.

In some embodiments, according to the multi-specific antibodies described herein comprises the amino acid sequence of SEQ ID NO: 52, or a variant thereof having at least about 80% (*e.g.,* at least 80%, 85%, 90%, 95%, 96%, 97%, 98%, or 99%) sequence identity to the amino acid sequence of SEQ ID NO: 52; and/or the amino acid sequence of SEQ ID NO: 58, or a variant thereof having at least about 80% (*e.g.,* at least 80%, 85%, 90%, 95%, 96%, 97%, 98%, or 99%) sequence identity to the amino acid sequence of SEQ ID NO: 58; and/or the amino acid sequence of SEQ ID NO: 59, or a variant thereof having at least about 80% (*e.g.,* at least 80%, 85%, 90%, 95%, 96%, 97%, 98%, or 99%) sequence identity to the amino acid sequence of SEQ ID NO: 59; and /or the amino acid sequence of SEQ ID NO: 60, or a variant thereof having at least about 80% (*e.g.,* at least 80%, 85%, 90%, 95%, 96%, 97%, 98%, or 99%) sequence identity to the amino acid sequence of SEQ ID NO: 60.

### IgG-(scFv)₂ format multi-specific antibodies

In some embodiments, the multi-specific antibody described herein has IgG-(scFv)₂ format, which consists of two identical monomers, each monomer comprising two antigen-binding domains, one of which is Fab and the other of which is scFv. In some embodiments, the multi-specific antibody further comprises an Fc comprising C_{H}2 and C_{H}3 domains. The scFv connects to the carboxyl terminal of the Fc through the peptide linker (L). An exemplary schematic diagram of the IgG-(scFv)₂ format is shown in FIG. 2D.

In some embodiments of the application, one of the antigen-binding domains (Fab or scFv) specifically binds to MASP2 and the other antigen-binding domain (scFv or Fab) specifically binds to MASP3. In some embodiments, the multi-specific antibody can bind to MASP3 and MASP2 simultaneously.

In some embodiments, the multi-specific antibody disclosed herein consists of two identical monomers, each of which comprises a heavy chain and a light chain. In some embodiments, the heavy chain of the multi-specific antibody comprises V_{H}1-C_{H}1 structure from N- terminus to C-terminus. In some embodiments, the light chain of the multi-specific antibody comprises V_{L}1-C_{L} structure from N- terminus to C-terminus. In some embodiments, the heavy chain further comprises an Fc comprising C_{H}2 and C_{H}3 domains. In other embodiments, the heavy chain of the multi-specific antibody comprises V_{H}1-C_{H}1-C_{H}2-C_{H}3-L-V_{H}2-L3-V_{L}2 structure from N- terminus to C-terminus. In other embodiments, the heavy chain of the multi-specific antibody comprises V_{H}1-C_{H}1-C_{H}2-C_{H}3-L-V_{L}2-L3-V_{H}2 structure from N- terminus to C-terminus. In some embodiments, the light chain of the multi-specific antibody comprises V_{L}1-C_{L} structure from N- terminus to C-terminus. Wherein, the V_{H}1 and V_{L}1 are the heavy chain variable domain and light chain variable domain that specifically bind to one of the antigens, respectively; the V_{H}2 and V_{L}2 are the heavy chain variable domain and light chain variable domain that specifically bind to the other antigen, respectively; C_{H}1 is a heavy chain constant domain 1; C_{L} is a light chain constant domain; L and L3 are peptide linkers. The V_{H}1-C_{H}1 and V_{L}1-C_{L} form one of the antigen-binding domains (Fab) of the multi-specific antibody; the V_{H}2-L3-V_{L}2 or V_{L}2-L3-V_{H}2 form the other antigen-binding domain (scFv) of the multi-specific antibody.

In some embodiments, the V_{H}1 and V_{L}1 are the heavy chain variable domain and light chain variable domain that specifically bind to MASP2, respectively, *i.e.,* the V_{H}1-C_{H}1 and V_{L}1-C_{L} form the antigen-binding domain (Fab) specifically binding to MASP2; the V_{H}2 and V_{L}2 are the heavy chain variable domain and light chain variable domain that specifically bind to MASP3, respectively, *i.e.,* the V_{H}2-L3-V_{L}2 or V_{L}2-L3-V_{H}2 form the antigen-binding domain (scFv) specifically binding to MASP3. In some embodiments, the V_{H}1 and V_{L}1 are the heavy chain variable domain and light chain variable domain that specifically bind to MASP3, respectively, *i.e.,* the V_{H}1-C_{H}1 and V_{L}1-C_{L} form the antigen-binding domain (Fab) specifically binding to MASP3; the V_{H}2 and V_{L}2 are the heavy chain variable domain and light chain variable domain that specifically bind to MASP2, respectively, *i.e.,* the V_{H}2-L3-V_{L}2 or V_{L}2-L3-V_{H}2 form the antigen-binding domain (scFv) specifically binding to MASP2. In some embodiments, the multi-specific antibody comprises C_{H}1 comprising the amino acid sequence of SEQ ID No: 49 or 50. In some embodiments, the multi-specific antibody comprises C_{H}2-C_{H}3 comprising the amino acid sequence of SEQ ID No: 47 or 48.

Peptide linker (or linker) may be used to join domains and/or regions of the chimeric heavy chain of the multi-specific antibody into a contiguous molecule. In some embodiments, the multi-specific antibody may include additional linkers, such as a flexible linker interconnecting the variable heavy and light chains of an scFv. In some embodiments, the multi-specific antibody may include additional linkers, such as a flexible linker interconnecting the variable heavy and light chains of an scFv and other linkers that connect other binding units to the core structure of the multi-specific antibody.

An exemplary, non-limiting example of a linker is a polypeptide chain comprising at least 4 residues. Portions of such linkers may be flexible, hydrophilic and have little or no secondary structure of their own (linker portions or flexible linker portions). Linkers of at least 4 amino acids may be used to join domains and/or regions that are positioned near to one another after the molecule has assembled. Longer linkers may also be used. In some embodiments, linkers may be about any one of: 1, 2, 3, 4, 5, 6, 7, 8, 9, 10, 11, 12, 13, 14, 15, 16, 17, 18, 19, 20, 25, 30, 35, 40, 45, 50, 100, 125, 150, 175 or 200 residues. When multiple linkers are used to interconnect portions of the molecule, the linkers may be the same or different (*e*.*g*., the same or different length and/or amino acid sequence).

In some aspects, the peptide linker (linker) comprises or consists of a Gly-Ser linker. As described herein, the term "Gly-Ser linker" refers to a peptide that consists of glycine and serine residues. An exemplary Gly-Ser linker comprises an amino acid sequence of the formula (Gly₄Ser)ₙ, wherein n is a positive integer (*e.g.,* 1, 2, 3, 4, 5, 6, 7, 8, 9 or 10). A preferred Gly-Ser linker is (Gly₄Ser)₂, *i.e.,* GGGGSGGGGS(SEQ ID NO: 63), and (Gly₄Ser)₄, *i.e.,* GGGGSGGGGSGGGGSGGGGS(SEQ ID NO: 64). Another preferred Gly-Ser linker is (Gly₄Ser)₃, *i*.*e*.,, GGGGSGGGGSGGGGS (SEQ ID NO: 65). In yet other aspects, two or more Gly-Ser linker are incorporated in series in a peptide linker. In some aspects, the peptide linker comprises at least a portion of a hinge region (*e*.*g*., derived from an IgG1, IgG2, IgG3, or IgG4 molecule) and a series of Gly-Ser amino acid residues (*e*.*g*., a Gly-Ser linker such as (G₄S)ₙ).

In some embodiments, the peptide linker comprises a Gly-Ser or all Gly linker and a portion or modified portion of a hinge domain. In some embodiments, the peptide linker (*e.g.,* L) of the multi-specific antibody connecting the antigen-binding domain to the C_{H}3 of the Fc carboxyl terminal comprises the amino acid sequence GGGGSGGGGTGGGGS (SEQ ID NO: 75).

In some embodiments, regardless of the peptide linker used to interconnect the antigen-binding domain to Fc (*e.g.,* L), the multi-specific antibody may optionally comprise additional peptide linkers. The lengths and sequence of such additional peptide linkers are independently selected. For example, the multi-specific antibody may further comprise a flexible peptide linker (L3) interconnecting the variable heavy and light chains of a scFv (V_{HSCFV} and V_{LSCFV}). This flexible peptide linker may comprise a Gly-Ser linker. Generally, this linker does not include a hinge portion. In some embodiments, this flexible peptide linker (L3) interconnecting the variable heavy and light chains of the scFv comprises the sequence of GGGGSGGGGSGGGGSGGGGS (SEQ ID NO: 64).

### scFv-Fab IgG format multi-specific antibodies

In some embodiments, the multi-specific antibody described herein has scFv-Fab IgG format, which is in the form of heterodimer. The heterodimer comprises a first monomer and a second monomer, while the first monomer comprises the antigen-binding domain (Fab) binding to one of the antigens, the second monomer comprises the antigen-binding domain (scFv) binding to the other antigen. In some embodiments, the multi-specific antibody further comprises two Fc comprising C_{H}2 and C_{H}3 domains. The two Fc further comprise amino acid substitution, which can promote the binding of heterodimers. An exemplary schematic diagram of scFv-Fab IgG format is shown in FIG. 2E.

In some embodiments, one of the antigen-binding domains (Fab or scFv) specifically binds to MASP3 and the other antigen-binding domain (scFv or Fab) specifically binds to MASP2. In some embodiments, the bispecific antibody can bind to MASP3 and MASP2 simultaneously.

In some embodiments, the multi-specific antibody described herein has scFv-Fab IgG format, which is in the form of heterodimer. The heterodimer comprises a first monomer and a second monomer, while the first monomer comprises two polypeptide chains: the first heavy chain and light chain, wherein the first heavy chain comprises V_{H}1-C_{H}1 structure from N- terminus to C-terminus, the light chain comprises V_{L}1-C_{L} structure. In some embodiments, the first heavy chain further comprises an Fc comprising C_{H}2 and C_{H}3 domains. In some embodiments, the first heavy chain comprises V_{H}1-C_{H}1-C_{H}2-C_{H}3 structure from N-terminus to C-terminus, the light chain comprises V_{L}1-C_{L} structure. The V_{H}1 and V_{L}1 are the heavy chain variable domain and light chain variable domain that specifically bind to one of the antigens, respectively; C_{H}1 is a heavy chain constant domain 1; C_{L} is a light chain constant domain. The V_{H}1-C_{H}1 and V_{L}1-C_{L} form one of the antigen-binding domains (Fab). The second monomer comprises one polypeptide chain: the second heavy chain. The second heavy chain comprises V_{H}2-L3-V_{L}2 structure or V_{L}2-L3-V_{H}2 structure from N- terminus to C-terminus. In some embodiments, the second heavy chain further comprises an Fc comprising C_{H}2 and C_{H}3 domains. In some embodiments, the second heavy chain comprises V_{H}2-L3-V_{L}2-C_{H}2-C_{H}3 structure or V_{L}2-L3-V_{H}2-C_{H}2-C_{H}3 structure from N- terminus to C-terminus. The V_{H}2 and V_{L}2 are the heavy chain variable domain and light chain variable domain that specifically bind to the other antigen, respectively; L3 is a peptide linker. The V_{H}2-L3-V_{L}2 or V_{L}2-L3-V_{H}2 form the other antigen-binding domains (scFv).

In some embodiments, the V_{H}1 and V_{L}1 are the heavy chain variable domain and light chain variable domain that specifically bind to MASP2, respectively, *i.e.,* the V_{H}1-C_{H}1 and V_{L}1-C_{L} form the antigen-binding domain (Fab) specifically binding to MASP2; the V_{H}2 and V_{L}2 are the heavy chain variable domain and light chain variable domain that specifically bind to MASP3, respectively, *i.e.,* the V_{H}2-L3-V_{L}2 or V_{L}2-L3-V_{H}2 form the antigen-binding domain (scFv) specifically binding to MASP3. In some embodiments, the V_{H}1 and V_{L}1 are the heavy chain variable domain and light chain variable domain that specifically bind to MASP3, respectively, *i.e.,* the V_{H}1-C_{H}1 and V_{L}1-C_{L} form the antigen-binding domain (Fab) specifically binding to MASP3. The V_{H}2 and V_{L}2 are the heavy chain variable domain and light chain variable domain that specifically bind to MASP2, respectively, *i.e.,* the V_{H}2-L3-V_{L}2 or V_{L}2-L3-V_{H}2 form the antigen-binding domain (scFv) specifically binding to MASP2.

In some embodiments, the Fc is derived from human wild-type IgG1. In another embodiment, relative to human wild-type IgG1, the Fc in one monomer comprises, but is not limited to the amino acid substitution described below: E357Q and S364K; relative to human wild-type IgG1, the Fc in the other monomer comprises, but is not limited to the amino acid substitution described below: Q295E, L368D, K370S, N384D, Q418E and N421D, wherein the numbering is according to EU index of Kabat. In some embodiments, exemplary peptide linker *(e.g.,* L3) connecting V_{H}1 with V_{L}1 of scFv comprises GKPGSGKPGSGKPGSGKPGS (SEQ ID NO: 74).

### Hetero H, CrossMab 2+1 format multi-specific antibodies

In some embodiments, any one of the multi-specific antibodies (*e*.*g*., bispecific antibodies) described herein has Hetero H, CrossMab 2+1 structure, which is a trivalent multi-specific antibody consisting of heterodimer, comprising three antigen-binding domains Fab, wherein the first and the second antigen binding domain specifically bind to one of the antigens, and the third antigen binding domain specifically bind to the other antigen. In other embodiments, the multi-specific antibody further comprises two Fc domains comprising C_{H}2 and C_{H}3 domains. In some embodiments, the amino acid residue in C_{H}3 domain of one of the Fc is substituted with a larger side chain volume amino acid residue to form a "knob", the amino acid residue in C_{H}3 domain of the other Fc is substituted with a smaller side chain volume amino acid residue to form a "hole", which can promote the binding of heterodimers. Wherein, in the Fab arm of the third antigen binding domain, the location of the light chain constant domain (C_{L}) and the heavy chain constant domain 1 (C_{H}1) can be exchanged with each other; or the location of the heavy chain variable domain (V_{H} ) and the light chain variable domain (V_{L}) can be exchanged with each other; or the light chain constant domain (C_{L}) and the heavy chain constant domain 1(C_{H}1) as well as the heavy chain variable domain (V_{H} ) and the light chain variable domain (V_{L}) can be exchanged with each other simultaneously. An exemplary schematic diagram of the Hetero H, CrossMab 2+1 format is shown in FIG. 2F. An exemplary schematic diagram of the Hetero H, CrossMab 2+1 format with the amino acid mutations of opposite charges in the non-exchange Fab region is shown in FIG. 2G.

In some embodiments, the Fc is derived from wild-type human IgG1 Fc. In other embodiments, the C_{H}3 domain of the Fc comprises, but is not limited to the amino acid substitution described below: S354C, T366W, Y349C, T366S, L368A and Y407V, wherein the numbering is according to EU index of Kabat.

In other embodiments of the application, two of the antigen-binding domains specifically bind to MASP3 and one of the antigen-binding domains specifically binds to MASP2. In some embodiments, two of the antigen-binding domains specifically bind to MASP2 and one of the antigen-binding domains specifically binds to MASP3. In some embodiments, the multi-specific antibody can bind to MASP3 and MASP2 simultaneously.

In some embodiments, the multi-specific antibody described herein has Hetero H, CrossMab 2+1 format, which is a heterodimer consisting of two different monomers, the first monomer comprises two polypeptide chains, the second monomer comprises three polypeptide chains. Wherein the first monomer comprises the first heavy chain and the first light chain specifically binding to one of the antigens, and the second monomer comprises the second heavy chain, the second light chain, and the third light chain specifically binding to two antigens. In some embodiments, the first heavy chain of the multi-specific antibody comprises V_{H}1-C_{H}1 structure from N- terminus to C-terminus; the first light chain of the multi-specific antibody comprises V_{L}1-C_{L} structure from N- terminus to C-terminus. In some embodiments, the first heavy chain further comprises an Fc comprising C_{H}2 and C_{H}3 domains. In some embodiments, the first heavy chain of the multi-specific antibody comprises V_{H}1-C_{H}1-C_{H}2-C_{H}3 structure from N- terminus to C-terminus; the first light chain of the multi-specific antibody comprises V_{L}1-C_{L} structure from N- terminus to C-terminus. The V_{H}1 and V_{L}1 are the heavy chain variable domain and light chain variable domain that specifically bind to one of the antigens, respectively, C_{H}1 is a heavy chain constant domain 1, C_{L} is a light chain constant domain. Wherein, the V_{H}1-C_{H}1 and V_{L}1-C_{L} form the antigen-binding domain (Fab) binding to one of the antigens. In some embodiments, the second heavy chain of the multi-specific antibody comprises V_{H}1-C_{H}1-V_{H}2-C_{L}-C_{H}2-C_{H}3 structure from N- terminus to C-terminus; the second light chain of the multi-specific antibody comprises V_{L}1-C_{L} structure from N- terminus to C-terminus; the third light chain of the multi-specific antibody comprises V_{L}2-C_{H}1 structure from N- terminus to C-terminus. The V_{H}1 and V_{L}1 are the heavy chain variable domain and light chain variable domain that specifically bind to one antigen, respectively, the V_{H}2 and V_{L}2 are the heavy chain variable domain and light chain variable domain that specifically bind to the other antigen, respectively, C_{H}1 is a heavy chain constant domain 1, C_{L} is a light chain constant domain. Wherein, the V_{H}1-C_{H}1 and V_{L}1-C_{L} form the antigen-binding domain (Fab) binding to one of the antigens, the V_{H}2-C_{L} and V_{L}2-C_{H}1 form the antigen-binding domain (Fab) binding to the other antigen. In some embodiments, the location of C_{L} and C_{H}1 of the first monomer or the second monomer of the multi-specific antibody can be exchanged with each other. In some embodiments, the location of V_{H}1 and V_{L}1 can be exchanged with each other. In other embodiments, the location of V_{H}2 and V_{L}2 can be exchanged with each other. In some embodiments, the amino acid residue in C_{H}3 domain of the first heavy chain is substituted with a larger side chain volume amino acid residue to form a "knob", and the amino acid residue in C_{H}3 domain of the second heavy chain is substituted with a smaller side chain volume amino acid residue to form a "hole". In some embodiments, the amino acid residue in C_{H}3 domain of the second heavy chain is substituted with a larger side chain volume amino acid residue to form a "knob", and the amino acid residue in C_{H}3 domain of the first heavy chain is substituted with a smaller side chain volume amino acid residue to form a "hole". In some embodiments, the C_{H}3 domain comprises, but is not limited to the amino acid substitution described below: S354C, T366W, Y349C, T366S, L368A and Y407V, wherein the numbering is according to EU index of Kabat. In some embodiments, the amino acid residue in C_{H}1 domain of the first heavy chain is substituted with an amino acid with the negative charge, and the amino acid residue in C_{L} domain of the first light chain is substituted with an amino acid with the positive charge; and the amino acid residue in C_{H}1 domain of the second heavy chain is substituted with an amino acid with the negative charge, and the amino acid residue in C_{L} domain of the second light chain is substituted with an amino acid with the positive charge. In other embodiments, the amino acid residue in C_{H}1 domain of the first heavy chain is substituted with an amino acid with the positive charge, and the amino acid residue in C_{L} domain of the first light chain is substituted with an amino acid with the negative charge; and the amino acid residue in C_{H}1 domain of the second heavy chain is substituted with an amino acid with the positive charge, and the amino acid residue in C_{L} domain of the second light chain is substituted with an amino acid with the negative charge. In some embodiments, the C_{H}1 domain comprises, but is not limited to the amino acid substitution described below: K148E, K214E, wherein the numbering is according to EU index of Kabat; the C_{L} domain comprises, but is not limited to the amino acid substitution described below: E128R, Q129K, wherein the numbering is according to EU index of Kabat.

In some embodiments, the V_{H}1 and V_{L}1 are the heavy chain variable domain and light chain variable domain that specifically bind to MASP3, respectively, the V_{H}2 and V_{L}2 are the heavy chain variable domain and light chain variable domain that specifically bind to MASP2, respectively, *i.e.,* the V_{H}1-C_{H}1 and V_{L}1-C_{L} form the antigen-binding domain (Fab) specifically binding to MASP3, V_{H}2-C_{L} and V_{L}2-C_{H}1 form the antigen-binding domain (Fab) specifically binding to MASP2. In another embodiment, the V_{H}1 and V_{L}1 are the heavy chain variable domain and light chain variable domain that specifically bind to MASP2, respectively, the V_{H}2 and V_{L}2 are the heavy chain variable domain and light chain variable domain that specifically bind to MASP3, respectively, *i.e.,* the V_{H}1-C_{H}1 and V_{L}1-C_{L} form the antigen-binding domain (Fab) specifically binding to MASP2, V_{H}2-C_{L} and V_{L}2-C_{H}1 form the antigen-binding domain (Fab) specifically binding to MASP3.

Exemplary antibody sequences are shown in Tables 2-9, wherein the CDR numbering is according to the EU index of Kabat. Those skilled in the art will recognize that many algorithms are known for prediction of CDR positions and for delimitation of antibody heavy chain and light chain variable domains. CDRs, V_{H} and/or V_{L} sequences from the antibodies specifically binding to MASP3, the antibodies specifically binding to MASP2 or the multi-specific antibody described herein, but based on prediction algorithms other than those exemplified in the tables below, are within the scope of this invention.

**Table 2: Exemplary antibody CDR sequences specifically binding to MASP3**

| **Antibody name** | **HC-CDR1** | **HC-CDR2** | **HC-CDR3** |
|---|---|---|---|
| M3-K1 | GKWIE (SEQ ID NO: 1) | EILPGSGSTDYNERFKD (SEQ ID NO: 2) | SEDL (SEQ ID NO: 3) |
| M3-K1-1 | GKWIE (SEQ ID NO: 1) | EILPGSGSTDYNERFKD (SEQ ID NO: 2) | SEDL (SEQ ID NO: 3) |
| M3-K1-2 | GKWIE (SEQ ID NO: 1) | EILPGSGSTDYNERFKD (SEQ ID NO: 2) | SEDL (SEQ ID NO: 3) |
| M3-K1-3 | GKWIE (SEQ ID NO: 1) | EILPGSGSTDYNERFKD (SEQ ID NO: 2) | SEDL (SEQ ID NO: 3) |
| M3-K1-4 | GKWIE (SEQ ID NO: 1) | EILPGSGSTDYNERFKD (SEQ ID NO: 2) | SEDL (SEQ ID NO: 3) |
| M3-K1-5 | GKWIE (SEQ ID NO: 1) | EILPGSGSTDYNERFKD (SEQ ID NO: 2) | SEDL (SEQ ID NO: 3) |
| M3-K1-6 | GKWIE (SEQ ID NO: 1) | EILPGSGSTDYNERFKD (SEQ ID NO: 2) | SEDL (SEQ ID NO: 3) |
| M3-K2 | GKWIE (SEQ ID NO: 1) | EILPGSGSTDYNERFKD (SEQ ID NO: 2) | SEDL (SEQ ID NO: 3) |
| M3-K2-2 | GKWIE (SEQ ID NO: 1) | EILPGSGSTDYNERFKD (SEQ ID NO: 2) | SEDL (SEQ ID NO: 3) |
| M3-K2-3 | GKWIE (SEQ ID NO: 1) | EILPGSGSTDYNERFKD (SEQ ID NO: 2) | SEDL (SEQ ID NO: 3) |
| M3-K2-5 | GKWIE (SEQ ID NO: 1) | EILPGSGSTDYNERFKD (SEQ ID NO: 2) | SEDL (SEQ ID NO: 3) |
| M3-K2-6 | GKWIE (SEQ ID NO: 1) | EILPGSGSTDYNERFKD (SEQ ID NO: 2) | SEDL (SEQ ID NO: 3) |
| M3-K2-7 | GKWIE (SEQ ID NO: 1) | EILPGSGSTDYNERFKD (SEQ ID NO: 2) | SEDL (SEQ ID NO: 3) |
| M3-K2-8 | GKWIE (SEQ ID NO: 1) | EILPGSGSTDYNERFKD (SEQ ID NO: 2) | SEDL (SEQ ID NO: 3) |
| M3-K2-9 | GKWIE (SEQ ID NO: 1) | EILPGSGSTDYNERFKD (SEQ ID NO: 2) | SEDL (SEQ ID NO: 3) |
| M3-K2-10 | GKWIE (SEQ ID NO: 1) | EILPGSGSTDYNERFKD (SEQ ID NO: 2) | SEDL (SEQ ID NO: 3) |
| M3-K2-11 | GKWIE (SEQ ID NO: 1) | EILPGSGSTDYNERFKD (SEQ ID NO: 2) | SEDL (SEQ ID NO: 3) |
| Composite 1 | GKWIE (SEQ ID NO: 1) | EILPGSGSTDYNERFKD (SEQ ID NO: 2) | SEDL (SEQ ID NO: 3) |
| | | | |

| **Antibody name** | **LC-CDR1** | **LC-CDR2** | **LC-CDR3** |
|---|---|---|---|
| M3-K1 | KSSQSLLNSVTRKTYLA (SEQ ID NO: 4) | WASTRES (SEQ ID NO: 6) | KQSYTLPT (SEQ ID NO: 7) |
| M3-K1-1 | KSSQSLLNSVTRKTYLA (SEQ ID NO: 4) | WASTRES (SEQ ID NO: 6) | KQSYTLPT (SEQ ID NO: 7) |
| M3-K1-2 | KSSQSLLNSVTRKTYLA (SEQ ID NO: 4) | WASTRES (SEQ ID NO: 6) | KQSYTLPT (SEQ ID NO: 7) |
| M3-K1-3 | KSSQSLLNSVTRKTYLA (SEQ ID NO: 4) | WASTRES (SEQ ID NO: 6) | KQSYTLPT (SEQ ID NO: 7) |
| M3-K1-4 | KSSQSLLNSVTRKTYLA (SEQ ID NO: 4) | WASTRES (SEQ ID NO: 6) | KQSYTLPT (SEQ ID NO: 7) |
| M3-K1-5 | KSSQSLLNSVTRKTYLA (SEQ ID NO: 4) | WASTRES (SEQ ID NO: 6) | KQSYTLPT (SEQ ID NO: 7) |
| M3-K1-6 | KSSQSLLNSVTRKTYLA (SEQ ID NO: 4) | WASTRES (SEQ ID NO: 6) | KQSYTLPT (SEQ ID NO: 7) |
| M3-K2 | KSSQNLFNSRTRKNYLA (SEQ ID NO: 5) | WASTRES (SEQ ID NO: 6) | KQSYIPFT (SEQ ID NO: 8) |
| M3-K2-2 | KSSQNLFNSRTRKNYLA (SEQ ID NO: 5) | WASTRES (SEQ ID NO: 6) | KQSYIPFT (SEQ ID NO: 8) |
| M3-K2-3 | KSSQNLFNSRTRKNYLA (SEQ ID NO: 5) | WASTRES (SEQ ID NO: 6) | KQSYIPFT (SEQ ID NO: 8) |
| M3-K2-5 | KSSQNLFNSRTRKNYLA (SEQ ID NO: 5) | WASTRES (SEQ ID NO: 6) | KQSYIPFT (SEQ ID NO: 8) |
| M3-K2-6 | KSSQNLFNSRTRKNYLA (SEQ ID NO: 5) | WASTRES (SEQ ID NO: 6) | KQSYIPFT (SEQ ID NO: 8) |
| M3-K2-7 | KSSQNLFNSRTRKNYLA (SEQ ID NO: 5) | WASTRES (SEQ ID NO: 6) | KQSYIPFT (SEQ ID NO: 8) |
| M3-K2-8 | KSSQNLFNSRTRKNYLA (SEQ ID NO: 5) | WASTRES (SEQ ID NO: 6) | KQSYIPFT (SEQ ID NO: 8) |
| M3-K2-9 | KSSQNLFNSRTRKNYLA (SEQ ID NO: 5) | WASTRES (SEQ ID NO: 6) | KQSYIPFT (SEQ ID NO: 8) |
| M3-K2-10 | KSSQNLFNSRTRKNYLA (SEQ ID NO: 5) | WASTRES (SEQ ID NO: 6) | KQSYIPFT (SEQ ID NO: 8) |
| M3-K2-11 | KSSQNLFNSRTRKNYLA (SEQ ID NO: 5) | WASTRES (SEQ ID NO: 6) | KQSYIPFT (SEQ ID NO: 8) |
| Composite 1 | KSSQX₁LX₂NSX₃TRKX₄YLA (SEQ ID NO: 9) wherein, X₁ is selected from N or S, X₂ is selected from F or L, X₃ is selected from R or V, X₄ is selected from N or T | WASTRES (SEQ ID NO: 6) | KQSYX₁X₂X₃T (SEQ ID NO: 10) wherein, X₁ is selected from I or T , X₂ is selected from L or P, X₃ is selected from F or P |

**Table 3: Exemplary antibody V_{H}/V_{L} sequences specifically binding to MASP3**

| **SEQ ID NO:** | **Description** | **Sequence** |
|---|---|---|
| 11 | M3-K1 V_{H} | EVQLQQSGAELVKPGASVKISCKASGYTFTGKWIEWVKQRPGHGLE WIGEILPGSGSTDYNERFKDKATFTADTSSNTAYMQLTSLTPDDSAT YYCVRSEDLWGSGTTLTVSS |
| | M3-K2 V_{H} | |
| 12 | M3-K1-1 V_{H} | QVQLVQSGAEVKKPGASVKVSCKASGYTFTGKWIEWVRQAPGQGL EWIGEILPGSGSTDYNERFKDRATFTADTSTSTAYMELSSLRSEDTAV YYCVRSEDLWGQGTLVTVSS |
| | M3-K1-2 V_{H} | |
| | M3-K2-6 V_{H} | |
| | M3-K2-7 V_{H} | |
| 13 | M3-K1-3 V_{H} | QVQLVQSGAEVKKPGASVKVSCKASGYTFTGKWIEWVRQAPGQGL EWMGEILPGSGSTDYNERFKDRATFTADTSTSTAYMELSSLRSEDTA VYYCVRSEDLWGQGTLVTVSS |
| | M3-K1-4 V_{H} | |
| | M3-K2-2 V_{H} | |
| | M3-K2-8 V_{H} | |
| | M3-K2-9 V_{H} | |
| 14 | M3-K1-5 V_{H} | QVQLVQSGAEVKKPGASVKVSCKASGYTFTGKWIEWVRQAPGQGL EWMGEILPGSGSTDYNERFKDRVTFTADTSTSTVYMELSSLRSEDTA VYYCVRSEDLWGQGTLVTVSS |
| | M3-K1-6 V_{H} | |
| | M3-K2-5 V_{H} | |
| | M3-K2-10 V_{H} | |
| | M3-K2-11 V_{H} | |
| 15 | M3-K2-3 V_{H} | QVQLVQSGAEVKKPGASVKVSCKASGYTFTGKWIEWVRQAPGQGL EWIGEILPGSGSTDYNERFKDRATFTADTSTSTVYMELSSLRSEDTAV YYCVRSEDLWGQGTLVTVSS |
| | | |
| 16 | M3-K1 V_{L} | DVVMTQTPSSLAVSAGEKVTMSCKSSQSLLNSVTRKTYLAWYQQKP GQSPKLLIYWASTRESGVPDRFTGSGSGTDFTLTISSVQAEDLAIYYC KQSYTLPTFGGGTKLEIK |
| 17 | M3-K1-1 V_{L} | DIVMTQSPDSLAVSLGERATINCKSSQSLLNSVTRKTYLAWYQQKPG QPPKLLIYWASTRESGVPDRFSGSGSGTDFTLTISSLQAEDVAVYYCK QSYTLPTFGGGTKVEIK |
| | M3-K1-3 V_{L} | |
| | M3-K1-5 V_{L} | |
| 18 | M3-K1-2 V_{L} | DIVMTQSPDSLAVSLGERATINCKSSQSLLNSVTRKTYLAWYQQKPG QSPKLLIYWASTRESGVPDRFSGSGSGTDFTLTISSLQAEDVAVYYCK QSYTLPTFGGGTKVEIK |
| | M3-K1-4 V_{L} | |
| | M3-K1-6 V_{L} | |
| 19 | M3-K2 V_{L} | DIVLTQSPSSLAVSAGEKVTMTCKSSQNLFNSRTRKNYLAWFQQKPG QSPKLLIYWASTRESGVPDRFTGSGSGTDFTLTISSVQAEDLAVYYCK QSYIPFTFGGGTKLEIK |
| | M3-K2-2 V_{L} | EIVLTQSPATLSVSPGERATLSCKSSQNLFNSRTRKNYLAWFQQKPG QSPRLLIYWASTRESGIPARFSGSGSGTEFTLTISSLQSEDFAVYYCKQ SYIPFTFGGGTKVEIK |
| 20 | M3-K2-3 V_{L} | |
| | M3-K2-5 V_{L} | |
| | M3-K2-6 V_{L} | DIVLTQSPDSLAVSLGERATINCKSSQNLFNSRTRKNYLAWFQQKPG QSPKLLIYWASTRESGVPDRFSGSGSGTDFTLTISSLQAEDVAVYYCK QSYIPFTFGGGTKVEIK |
| 21 | M3-K2-8 V_{L} | |
| | M3-K2-10 V_{L} | |
| | M3-K2-7 V_{L} | DIVMTQSPDSLAVSLGERATINCKSSQNLFNSRTRKNYLAWFQQKPG QSPKLLIYWASTRESGVPDRFSGSGSGTDFTLTISSLQAEDVAVYYCK QSYIPFTFGGGTKVEIK |
| 22 | M3-K2-9 V_{L} | |
| | M3-K2-11 V_{L} | |

**Table 4: Exemplary antibody CDR sequences specifically binding to MASP2**

| **Antibody name** | **HC-CDR1** | **HC-CDR2** | **HC-CDR3** |
|---|---|---|---|
| humM2mc51-1 | SDYAWN (SEQ ID NO: 23) | YISYSGRTSYNPSLKS (SEQ ID NO: 24) | HYGDY (SEQ ID NO: 25) |
| humM2mc51-1-S93A | SDYAWN (SEQ ID NO: 23) | YISYSGRTSYNPSLKS (SEQ ID NO: 24) | HYGDY (SEQ ID NO: 25) |
| humM2mc51-1-S93E | SDYAWN (SEQ ID NO: 23) | YISYSGRTSYNPSLKS (SEQ ID NO: 24) | HYGDY (SEQ ID NO: 25) |
| humM2mc51-1-N92Q | SDYAWN (SEQ ID NO: 23) | YISYSGRTSYNPSLKS (SEQ ID NO: 24) | HYGDY (SEQ ID NO: 25) |
| humM2mc51-1-N92S | SDYAWN (SEQ ID NO: 23) | YISYSGRTSYNPSLKS (SEQ ID NO: 24) | HYGDY (SEQ ID NO: 25) |
| humM2mc51-1-ES93DA | SDYAWN (SEQ ID NO: 23) | YISYSGRTSYNPSLKS (SEQ ID NO: 24) | HYGDY (SEQ ID NO: 25) |
| M2mc51 | SDYAWN (SEQ ID NO: 23) | YISYSGRTSYNPSLKS (SEQ ID NO: 24) | HYGDY (SEQ ID NO: 25) |
| Composite 1 | SDYAWN (SEQ ID NO: 23) | YISYSGRTSYNPSLKS (SEQ ID NO: 24) | HYGDY (SEQ ID NO: 25) |
| | | | |

| **Antibody name** | **LC-CDR1** | **LC-CDR2** | **LC-CDR3** |
|---|---|---|---|
| humM2mc51-1 | KASQNVGTNVA (SEQ ID NO: 26) | SASYRYS (SEQ ID NO: 27) | HQYNSNPLT (SEQ ID NO: 28) |
| humM2mc51-1-S93A | KASQNVGTNVA (SEQ ID NO: 26) | SASYRYS (SEQ ID NO: 27) | HQYNANPLT (SEQ ID NO: 29) |
| humM2mc51-1-S93E | KASQNVGTNVA (SEQ ID NO: 26) | SASYRYS (SEQ ID NO: 27) | HQYNENPLT (SEQ ID NO: 30) |
| humM2mc51-1-N92Q | KASQNVGTNVA (SEQ ID NO: 26) | SASYRYS (SEQ ID NO: 27) | HQYQSNPLT (SEQ ID NO: 31) |
| humM2mc51-1-N92S | KASQNVGTNVA (SEQ ID NO: 26) | SASYRYS (SEQ ID NO: 27) | HQYSSNPLT (SEQ ID NO: 32) |
| humM2mc51-1-ES93DA | KASQNVGTNVA (SEQ ID NO: 26) | SASYRYS (SEQ ID NO: 27) | HQYNANPLT (SEQ ID NO: 29) |
| M2mc51 | KASQNVGTNVA (SEQ ID NO: 26) | SASYRYS (SEQ ID NO: 27) | HQYNSNPLT (SEQ ID NO: 28) |
| Composite 1 | KASQNVGTNVA (SEQ ID NO: 26) | SASYRYS (SEQ ID NO: 27) | HQYX₁X₂NPLT (SEQ ID NO: 33) wherein, X₁ is selected from N, Q, or S, X₂ is selected from A, E, or S |

**Table 5: Exemplary antibody V_{H}/V_{L} sequences specifically binding to MASP2**

| **SEQ ID NO:** | **Description** | **Sequence** |
|---|---|---|
| 34 | humM2mc51-1 V_{H} | QVQLQESGPGLVKPSETLSLTCTVSGYSITSDYAWNWIRQ PPGKKLEWMGYISYSGRTSYNPSLKSRITISRDTSKNQFSL KLSSVTAADTAVYYCSRHYGDYWGQGTLVTVSS |
| | humM2mc51-1-S93A V_{H} | |
| | humM2mc51-1-S93E V_{H} | |
| | humM2mc51-1-N92Q V_{H} | |
| | humM2mc51-1-N92S V_{H} | |
| | humM2mc51-1-ES93DA V_{H} | |
| 35 | M2mc51 V_{H} | EVKLQESGPGLVKPSQSLSLTCTVTGYSITSDYAWNWIRQ FPGNKLEWMGYISYSGRTSYNPSLKSRISITRDTSNNQFFL QLNSVTTEDTATYYCSRHYGDYWGQGTTLTVSS |
| | | |
| 36 | humM2mc51-1 V_{L} | EIVMTQSPATLSVSPGERATLSCKASQNVGTNVAWYQQK PGQSPRALIYSASYRYSGIPARFSGSGSGTEFTLTISSLQSE DFAVYFCHQYNSNPLTFGGGTKVEIK |
| 37 | humM2mc51-1-S93A V_{L} | EIVMTQSPATLSVSPGERATLSCKASQNVGTNVAWYQQK PGQSPRALIYSASYRYSGIPARFSGSGSGTEFTLTISSLQSE DFAVYFCHQYNANPLTFGGGTKVEIK |
| 38 | humM2mc51-1-S93E V_{L} | EIVMTQSPATLSVSPGERATLSCKASQNVGTNVAWYQQK PGQSPRALIYSASYRYSGIPARFSGSGSGTEFTLTISSLQSE DFAVYFCHQYNENPLTFGGGTKVEIK |
| 39 | humM2mc51-1-N92Q V_{L} | EIVMTQSPATLSVSPGERATLSCKASQNVGTNVAWYQQK PGQSPRALIYSASYRYSGIPARFSGSGSGTEFTLTISSLQSE DFAVYFCHQYQSNPLTFGGGTKVEIK |
| 40 | humM2mc51-1-N92S V_{L} | EIVMTQSPATLSVSPGERATLSCKASQNVGTNVAWYQQK PGQSPRALIYSASYRYSGIPARFSGSGSGTEFTLTISSLQSE DFAVYFCHQYSSNPLTFGGGTKVEIK |
| 41 | humM2mc51-1-ES93DA V_{L} | DIVMTQSPATLSVSPGERATLSCKASQNVGTNVAWYQQK PGQSPRALIYSASYRYSGIPARFSGSGSGTEFTLTISSLQSE DFAVYFCHQYNANPLTFGGGTKVEIK |
| 42 | M2mc51 V_{L} | DILMTQTPKFMSTSVGDRVSVTCKASQNVGTNVAWYQQ KPGQSPKALIYSASYRYSGVPDRFTGSGSGTDFTLTISNVQ SEDLAEYFCHQYNSNPLTFGAGTKLELK |

**Table 6: Exemplary antibody constant region sequences**

| **SEQ ID NO:** | **Description** | **Sequence** |
|---|---|---|
| 43 | IgG1 constant region | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK |
| 44 | IgG4 constant region | ASTKGPSVFPLAPCSRSTSESTAALGCLVKDYFPEPVTVSWNSGALTSGVH TFPAVLQSSGLYSLSSVVTVPSSSLGTKTYTCNVDHKPSNTKVDKRVESK YGPPCPSCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKC KVSNKGLPSSIEKTISKAKGQPREPQVYTLPPSQEEMTKNQVSLTCLVKGF YPSDIAVEWESNGQPENNYKTTPPVLDSDGSFFLYSRLTVDKSRWQEGNV FSCSVMHEALHNHYTQKSLSLSLGK |
| 45 | kappa | |
| 46 | lambda | GQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKTV APTECS |
| 47 | Exemplary IgG1 C_{H}2-C_{H}3 | APELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKAL PAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVM HEALHNHYTQKSLSLSPGK |
| 48 | Exemplary IgG4 CH2-CH3 | APEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMH EALHNHYTQKSLSLSLGK |
| 49 | Exemplary IgG1 C_{H}1 | ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKV |
| 50 | Exemplary IgG4 C_{H}1 | ASTKGPSVFPLAPCSRSTSESTAALGCLVKDYFPEPVTVSWNSGALTSGVH TFPAVLQSSGLYSLSSVVTVPSSSLGTKTYTCNVDHKPSNTKVDKRV |

**Table 7: Partial heavy chain and light chain sequences of exemplary Hetero H, CrossMab format multi-specific antibody specifically binding to MASP3 and MASP2**

| **SEQ ID NO:** | **Description** | **Sequence** |
|---|---|---|
| 51 | humM2mc51-1-S93A V_{H}-C_{L} | QVQLQESGPGLVKPSETLSLTCTVSGYSITSDYAWNWIRQPPGKKLE WMGYISYSGRTSYNPSLKSRITISRDTSKNQFSLKLSSVTAADTAVYY CSRHYGDYWGQGTLVTVSSASVAAPSVFIFPPSDEQLKSGTASVVCL LNNFYPREAKVQWKVDNALQSGNSQESVTEQDSKDSTYSLSSTLTLS KADYEKHKVYACEVTHQGLSSPVTKSFNRGEC |
| 52 | humM2mc51-1-S93A V_{L}-C_{H}1 | EIVMTQSPATLSVSPGERATLSCKASQNVGTNVAWYQQKPGQSPRA LIYSASYRYSGIPARFSGSGSGTEFTLTISSLQSEDFAVYFCHQYNANP LTFGGGTKVEIKSSASTKGPSVFPLAPSSKSTSGGTAALGCLVKDYFP EPVTVSWNSGALTSGVHTFPAVLQSSGLYSLSSVVTVPSSSLGTQTYI CNVNHKPSNTKVDKKVEPKSC |
| 53 | M3-K2-5 V_{H}-C_{H}1 | QVQLVQSGAEVKKPGASVKVSCKASGYTFTGKWIEWVRQAPGQGL EWMGEILPGSGSTDYNERFKDRVTFTADTSTSTVYMELSSLRSEDTA VYYCVRSEDLWGQGTLVTVSSASTKGPSVFPLAPSSKSTSGGTAALG CLVKDYFPEPVTVSWNSGALTSGVHTFPAVLQSSGLYSLSSVVTVPS SSLGTQTYICNVNHKPSNTKVDKKV |
| 54 | M3-K2-5 V_{L}-C_{L} | EIVLTQSPATLSVSPGERATLSCKSSQNLFNSRTRKNYLAWFQQKPG QSPRLLIYWASTRESGIPARFSGSGSGTEFTLTISSLQSEDFAVYYCKQ SYIPFTFGGGTKVEIKRTVAAPSVFIFPPSDEQLKSGTASVVCLLNNFY PREAKVQWKVDNALQSGNSQESVTEQDSKDSTYSLSSTLTLSKADY EKHKVYACEVTHQGLSSPVTKSFNRGEC |
| 51 | humM2mc51-1-S93A V_{H}-C_{L} | QVQLQESGPGLVKPSETLSLTCTVSGYSITSDYAWNWIRQPPGKKLE WMGYISYSGRTSYNPSLKSRITISRDTSKNQFSLKLSSVTAADTAVYY CSRHYGDYWGQGTLVTVSSASVAAPSVFIFPPSDEQLKSGTASVVCL LNNFYPREAKVQWKVDNALQSGNSQESVTEQDSKDSTYSLSSTLTLS KADYEKHKVYACEVTHQGLSSPVTKSFNRGEC |
| 52 | humM2mc51-1-S93A V_{L}-C_{H}1 | EIVMTQSPATLSVSPGERATLSCKASQNVGTNVAWYQQKPGQSPRA LIYSASYRYSGIPARFSGSGSGTEFTLTISSLQSEDFAVYFCHQYNANP LTFGGGTKVEIKSSASTKGPSVFPLAPSSKSTSGGTAALGCLVKDYFP EPVTVSWNSGALTSGVHTFPAVLQSSGLYSLSSVVTVPSSSLGTQTYI CNVNHKPSNTKVDKKVEPKSC |
| 55 | M3-K2-6 V_{H}-C_{H}1 | QVQLVQSGAEVKKPGASVKVSCKASGYTFTGKWIEWVRQAPGQGL EWIGEILPGSGSTDYNERFKDRATFTADTSTSTAYMELSSLRSEDTAV YYCVRSEDLWGQGTLVTVSSASTKGPSVFPLAPSSKSTSGGTAALGC LVKDYFPEPVTVSWNSGALTSGVHTFPAVLQSSGLYSLSSVVTVPSSS LGTQTYICNVNHKPSNTKVDKKV |
| 56 | M3-K2-6 V_{L}-C_{L} | DIVLTQSPDSLAVSLGERATINCKSSQNLFNSRTRKNYLAWFQQKPG QSPKLLIYWASTRESGVPDRFSGSGSGTDFTLTISSLQAEDVAVYYCK QSYIPFTFGGGTKVEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC |
| 51 | humM2mc51-1-S93A V_{H}-C_{L} | QVQLQESGPGLVKPSETLSLTCTVSGYSITSDYAWNWIRQPPGKKLE WMGYISYSGRTSYNPSLKSRITISRDTSKNQFSLKLSSVTAADTAVYY CSRHYGDYWGQGTLVTVSSASVAAPSVFIFPPSDEQLKSGTASVVCL LNNFYPREAKVQWKVDNALQSGNSQESVTEQDSKDSTYSLSSTLTLS KADYEKHKVYACEVTHQGLSSPVTKSFNRGEC |
| 52 | humM2mc51-1-S93A V_{L}-C_{H}1 | EIVMTQSPATLSVSPGERATLSCKASQNVGTNVAWYQQKPGQSPRA LIYSASYRYSGIPARFSGSGSGTEFTLTISSLQSEDFAVYFCHQYNANP LTFGGGTKVEIKSSASTKGPSVFPLAPSSKSTSGGTAALGCLVKDYFP EPVTVSWNSGALTSGVHTFPAVLQSSGLYSLSSVVTVPSSSLGTQTYI CNVNHKPSNTKVDKKVEPKSC |
| 57 | M3-K2-8 V_{H}-C_{H}1 | QVQLVQSGAEVKKPGASVKVSCKASGYTFTGKWIEWVRQAPGQGL EWMGEILPGSGSTDYNERFKDRATFTADTSTSTAYMELSSLRSEDTA VYYCVRSEDLWGQGTLVTVSSASTKGPSVFPLAPSSKSTSGGTAALG CLVKDYFPEPVTVSWNSGALTSGVHTFPAVLQSSGLYSLSSVVTVPS SSLGTQTYICNVNHKPSNTKVDKKV |
| 56 | M3-K2-8 V_{L}-C_{L} | DIVLTQSPDSLAVSLGERATINCKSSQNLFNSRTRKNYLAWFQQKPG QSPKLLIYWASTRESGVPDRFSGSGSGTDFTLTISSLQAEDVAVYYCK QSYIPFTFGGGTKVEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC |
| 51 | humM2mc51-1-S93A V_{H}-C_{L} | QVQLQESGPGLVKPSETLSLTCTVSGYSITSDYAWNWIRQPPGKKLE WMGYISYSGRTSYNPSLKSRITISRDTSKNQFSLKLSSVTAADTAVYY CSRHYGDYWGQGTLVTVSSASVAAPSVFIFPPSDEQLKSGTASVVCL LNNFYPREAKVQWKVDNALQSGNSQESVTEQDSKDSTYSLSSTLTLS KADYEKHKVYACEVTHQGLSSPVTKSFNRGEC |
| 52 | humM2mc51-1-S93A V_{L}-C_{H}1 | EIVMTQSPATLSVSPGERATLSCKASQNVGTNVAWYQQKPGQSPRA LIYSASYRYSGIPARFSGSGSGTEFTLTISSLQSEDFAVYFCHQYNANP LTFGGGTKVEIKSSASTKGPSVFPLAPSSKSTSGGTAALGCLVKDYFP EPVTVSWNSGALTSGVHTFPAVLQSSGLYSLSSVVTVPSSSLGTQTYI CNVNHKPSNTKVDKKVEPKSC |
| 53 | M3-K2-11 V_{H}-C_{H}1 | QVQLVQSGAEVKKPGASVKVSCKASGYTFTGKWIEWVRQAPGQGL EWMGEILPGSGSTDYNERFKDRVTFTADTSTSTVYMELSSLRSEDTA VYYCVRSEDLWGQGTLVTVSSASTKGPSVFPLAPSSKSTSGGTAALG CLVKDYFPEPVTVSWNSGALTSGVHTFPAVLQSSGLYSLSSVVTVPS SSLGTQTYICNVNHKPSNTKVDKKV |
| 58 | M3-K2-11 V_{L}-C_{L} | DIVMTQSPDSLAVSLGERATINCKSSQNLFNSRTRKNYLAWFQQKPG QSPKLLIYWASTRESGVPDRFSGSGSGTDFTLTISSLQAEDVAVYYCK QSYIPFTFGGGTKVEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC |

**Table 8: Full-length heavy chain and light chain sequences of exemplary Hetero H, CrossMab format multi-specific antibody specifically binding to MASP3 and MASP2**

| **SEQ ID NO:** | **Description** | **Sequence** |
|---|---|---|
| 59 | humM2mc5 1-1-S93A V_{H}-C_{L}-IgG1 Fc | QVQLQESGPGLVKPSETLSLTCTVSGYSITSDYAWNWIRQPPGKKLEWMG YISYSGRTSYNPSLKSRITISRDTSKNQFSLKLSSVTAADTAVYYCSRHYGD YWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEV THQGLSSPVTKSFNRGECDKTHTCPPCPAPEAAGGPSVFLFPPKPKDTLMIS RTPEVTCVVVDVSHEDPEVKFNWYVDGVEVHNAKTKPREEQYNSTYRVV SVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFF LVSKLTVDKSRWQQGNVFSCSVMHEALHNHYTQKSLSLSPGK |
| 52 | humM2mc5 1-1-S93A V_{L}-C_{H}1 | EIVMTQSPATLSVSPGERATLSCKASQNVGTNVAWYQQKPGQSPRALIYSA SYRYSGIPARFSGSGSGTEFTLTISSLQSEDFAVYFCHQYNANPLTFGGGTK VEIKSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKV EPKSC |
| 60 | M3-K2-5 V_{H}-IgG1 C_{H} | QVQLVQSGAEVKKPGASVKVSCKASGYTFTGKWIEWVRQAPGQGLEWM GEILPGSGSTDYNERFKDRVTFTADTSTSTVYMELSSLRSEDTAVYYCVRSE DLWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPS NTKVDKKVEPKSCDKTHTCPPCPAPEAAGGPSVFLFPPKPKDTLMISRTPEV TCVVVDVSHEDPEVKFNWYVDGVEVHNAKTKPREEQYNSTYRVVSVLTV LHQDWLNGKEYKCKVSNKALPAPIEKTISKAKGQPREPQVYTLPPCRDELT KNQVSLWCLVKGFYPSDIAVEWESNGQPENNYKTTPPVLDSDGSFFLYSKL TVDKSRWQQGNVFSCSVMHEALHNHYTQKSLSLSPGK |
| 54 | M3-K2-5 V_{L}-C_{L} | EIVLTQSPATLSVSPGERATLSCKSSQNLFNSRTRKNYLAWFQQKPGQSPRL LIYWASTRESGIPARFSGSGSGTEFTLTISSLQSEDFAVYYCKQSYIPFTFGGG TKVEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLSS PVTKSFNRGEC |
| 59 | humM2mc5 1-1-S93A V_{H}-C_{L}-IgG1 Fc | QVQLQESGPGLVKPSETLSLTCTVSGYSITSDYAWNWIRQPPGKKLEWMG YISYSGRTSYNPSLKSRITISRDTSKNQFSLKLSSVTAADTAVYYCSRHYGD YWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEV THQGLSSPVTKSFNRGECDKTHTCPPCPAPEAAGGPSVFLFPPKPKDTLMIS RTPEVTCVVVDVSHEDPEVKFNWYVDGVEVHNAKTKPREEQYNSTYRVV SVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFF LVSKLTVDKSRWQQGNVFSCSVMHEALHNHYTQKSLSLSPGK |
| 52 | humM2mc5 1-1-S93A V_{L}-C_{H}1 | EIVMTQSPATLSVSPGERATLSCKASQNVGTNVAWYQQKPGQSPRALIYSA SYRYSGIPARFSGSGSGTEFTLTISSLQSEDFAVYFCHQYNANPLTFGGGTK VEIKSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKV EPKSC |
| 61 | M3-K2-6 V_{H}-IgG1 C_{H} | QVQLVQSGAEVKKPGASVKVSCKASGYTFTGKWIEWVRQAPGQGLEWIG EILPGSGSTDYNERFKDRATFTADTSTSTAYMELSSLRSEDTAVYYCVRSED LWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSN TKVDKKVEPKSCDKTHTCPPCPAPEAAGGPSVFLFPPKPKDTLMISRTPEVT CVVVDVSHEDPEVKFNWYVDGVEVHNAKTKPREEQYNSTYRVVSVLTVL HQDWLNGKEYKCKVSNKALPAPIEKTISKAKGQPREPQVYTLPPCRDELTK NQVSLWCLVKGFYPSDIAVEWESNGQPENNYKTTPPVLDSDGSFFLYSKLT VDKSRWQQGNVFSCSVMHEALHNHYTQKSLSLSPGK |
| 56 | M3-K2-6 V_{L}-C_{L} | DIVLTQSPDSLAVSLGERATINCKSSQNLFNSRTRKNYLAWFQQKPGQSPKL LIYWASTRESGVPDRFSGSGSGTDFTLTISSLQAEDVAVYYCKQSYIPFTFG GGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| 59 | humM2mc5 1-1-S93A V_{H}-C_{L}-IgG1 Fc | QVQLQESGPGLVKPSETLSLTCTVSGYSITSDYAWNWIRQPPGKKLEWMG YISYSGRTSYNPSLKSRITISRDTSKNQFSLKLSSVTAADTAVYYCSRHYGD YWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEV THQGLSSPVTKSFNRGECDKTHTCPPCPAPEAAGGPSVFLFPPKPKDTLMIS RTPEVTCVVVDVSHEDPEVKFNWYVDGVEVHNAKTKPREEQYNSTYRVV SVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFF LVSKLTVDKSRWQQGNVFSCSVMHEALHNHYTQKSLSLSPGK |
| 52 | humM2mc5 1-1-S93A V_{L}-C_{H}1 | EIVMTQSPATLSVSPGERATLSCKASQNVGTNVAWYQQKPGQSPRALIYSA SYRYSGIPARFSGSGSGTEFTLTISSLQSEDFAVYFCHQYNANPLTFGGGTK VEIKSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKV EPKSC |
| 62 | M3-K2-8 V_{H}-IgG1 C_{H} | QVQLVQSGAEVKKPGASVKVSCKASGYTFTGKWIEWVRQAPGQGLEWM GEILPGSGSTDYNERFKDRATFTADTSTSTAYMELSSLRSEDTAVYYCVRSE DLWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPS NTKVDKKVEPKSCDKTHTCPPCPAPEAAGGPSVFLFPPKPKDTLMISRTPEV TCVVVDVSHEDPEVKFNWYVDGVEVHNAKTKPREEQYNSTYRVVSVLTV LHQDWLNGKEYKCKVSNKALPAPIEKTISKAKGQPREPQVYTLPPCRDELT KNQVSLWCLVKGFYPSDIAVEWESNGQPENNYKTTPPVLDSDGSFFLYSKL TVDKSRWQQGNVFSCSVMHEALHNHYTQKSLSLSPGK |
| 56 | M3-K2-8 V_{L}-C_{L} | DIVLTQSPDSLAVSLGERATINCKSSQNLFNSRTRKNYLAWFQQKPGQSPKL LIYWASTRESGVPDRFSGSGSGTDFTLTISSLQAEDVAVYYCKQSYIPFTFG GGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |
| 59 | humM2mc5 1-1-S93A V_{H}-C_{L}-IgG1 Fc | QVQLQESGPGLVKPSETLSLTCTVSGYSITSDYAWNWIRQPPGKKLEWMG YISYSGRTSYNPSLKSRITISRDTSKNQFSLKLSSVTAADTAVYYCSRHYGD YWGQGTLVTVSSASVAAPSVFIFPPSDEQLKSGTASVVCLLNNFYPREAKV QWKVDNALQSGNSQESVTEQDSKDSTYSLSSTLTLSKADYEKHKVYACEV THQGLSSPVTKSFNRGECDKTHTCPPCPAPEAAGGPSVFLFPPKPKDTLMIS RTPEVTCVVVDVSHEDPEVKFNWYVDGVEVHNAKTKPREEQYNSTYRVV SVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFF LVSKLTVDKSRWQQGNVFSCSVMHEALHNHYTQKSLSLSPGK |
| 52 | humM2mc5 1-1-S93A V_{L}-C_{H}1 | EIVMTQSPATLSVSPGERATLSCKASQNVGTNVAWYQQKPGQSPRALIYSA SYRYSGIPARFSGSGSGTEFTLTISSLQSEDFAVYFCHQYNANPLTFGGGTK VEIKSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKV EPKSC |
| 60 | M3-K2-11 V_{H}-IgG1 C_{H} | QVQLVQSGAEVKKPGASVKVSCKASGYTFTGKWIEWVRQAPGQGLEWM GEILPGSGSTDYNERFKDRVTFTADTSTSTVYMELSSLRSEDTAVYYCVRSE DLWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPS NTKVDKKVEPKSCDKTHTCPPCPAPEAAGGPSVFLFPPKPKDTLMISRTPEV TCVVVDVSHEDPEVKFNWYVDGVEVHNAKTKPREEQYNSTYRVVSVLTV LHQDWLNGKEYKCKVSNKALPAPIEKTISKAKGQPREPQVYTLPPCRDELT KNQVSLWCLVKGFYPSDIAVEWESNGQPENNYKTTPPVLDSDGSFFLYSKL TVDKSRWQQGNVFSCSVMHEALHNHYTQKSLSLSPGK |
| 58 | M3-K2-11 V_{L}-C_{L} | DIVMTQSPDSLAVSLGERATINCKSSQNLFNSRTRKNYLAWFQQKPGQSPK LLIYWASTRESGVPDRFSGSGSGTDFTLTISSLQAEDVAVYYCKQSYIPFTFG GGTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC |

**Table 9: Exemplary polypeptide linker (or linker) sequences**

| **SEQ ID NO:** | **Sequence** |
|---|---|
| 63 | GGGGSGGGGS |
| 64 | GGGGSGGGGSGGGGSGGGGS |
| 65 | GGGGSGGGGSGGGGS |
| 66 | ASTKGP |
| 67 | TVAAP |
| 68 | EPKSDKTGGGGSGGGGS |
| 69 | EPKSCGKTGGGGSGGGGS |
| 70 | EPKSCGGGGSGGGGS |
| 71 | GGGGSGGGGSEPKSDKTHTCPPCP |
| 72 | GGGGSGGGGSCPPCP |
| 73 | GGGGSGGGGSDKTHTCPPCP |
| 74 | GKPGSGKPGSGKPGSGKPGS |
| 75 | GGGGSGGGGTGGGGS |
| 76 | GGGGSGGGGSESKYGPPCPPCP |

### Combination of antibodies specifically binding to MASP3 and MASP2

In one aspect, the present application provides a pharmaceutical composition comprising (i) an antibody or antigen-binding fragment specifically binding to MASP3 and (ii) an antibody or antigen-binding fragment specifically binding to MASP2.

In some embodiments, there is provided a pharmaceutical composition comprising (i) an antibody or antigen-binding fragment specifically binding to MASP3 and (ii) an antibody or antigen-binding fragment specifically binding to MASP2, wherein: the antibody or antigen-binding fragment specifically binding to MASP3 comprises: (a) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); (b) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P; or (c) a V_{H} comprising an HC-CDR1, an HC-CDR2, and an HC-CDR3 of a V_{H} comprising the amino acid sequence of any one of SEQ ID NOs: 11-15; and a V_{L} comprising an LC-CDR1, an LC-CDR2, and an LC-CDR3 of a V_{L} comprising the amino acid sequence of any one of SEQ ID NOs: 16-22. In some embodiments, the antibody or antigen-binding fragment specifically binding to MASP2 comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S; or (c) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of any one of SEQ ID NOs: 36-41.

In some embodiments, there is provided a pharmaceutical composition comprising (i) an antibody or antigen-binding fragment specifically binding to MASP3 and (ii) an antibody or antigen-binding fragment specifically binding to MASP2, wherein: the antibody or antigen-binding fragment specifically binding to MASP2 comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S; or (c) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of any one of SEQ ID NOs: 36-41.

In some embodiments, the antibody or antigen-binding fragment specifically binding to MASP3 in the pharmaceutical composition comprises: (i) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 16; or (ii) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19.

In some embodiments, the antibody or antigen-binding fragment specifically binding to MASP2 in the pharmaceutical composition comprises: (i) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:36; (ii) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 37; (iii) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:38; (iv) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 39; (v) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:40; or (vi) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 41.

In some embodiments, the antibody or antigen-binding fragment specifically binding to MASP3 in the pharmaceutical composition comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of any one of SEQ ID NOs: 4-5, or a variant thereof comprising up to about 3 amino acid substitutions, an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions.

In some embodiments, the antibody or antigen-binding fragment specifically binding to MASP2 in the pharmaceutical composition comprises: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of any one of SEQ ID NOs: 28-32, or a variant thereof comprising up to about 3 amino acid substitutions.

In some embodiments, the antibody or antigen-binding fragment specifically binding to MASP3 in the pharmaceutical composition comprises: (i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7; or (ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

In some embodiments, the antibody or antigen-binding fragment specifically binding to MASP2 in the pharmaceutical composition comprises: (i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28; (ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29; (iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30; (iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or (v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

In some embodiments, the antibody or antigen-binding fragment specifically binding to MASP3 in the pharmaceutical composition comprises: a V_{H} comprising the amino acid sequence of any one of SEQ ID NOs: 11-15, or a variant thereof having at least about 80% sequence identity to the amino acid sequence of any one of SEQ ID NOs: 11-15; and a V_{L} comprising the amino acid sequence of any one of SEQ ID NOs: 16-22, or a variant thereof having at least about 80% sequence identity to the amino acid sequence of any one of SEQ ID NOs: 16-22.

In some embodiments, the antibody or antigen-binding fragment specifically binding to MASP2 in the pharmaceutical composition comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of any one of SEQ ID NOs: 36-41, or a variant thereof having at least about 80% sequence identity to the amino acid sequence of any one of SEQ ID NOs: 36-41.

In some embodiments, the antibody or antigen-binding fragment specifically binding to MASP3 in the pharmaceutical composition comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, the antibody or antigen-binding fragment specifically binding to MASP2 in the pharmaceutical composition comprises: (i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36; (ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; (iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38; (iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39; (v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or (vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

In some embodiments, there is provided a pharmaceutical composition comprising: an antibody or antigen-binding fragment specifically binding to MASP3 and an antibody or antigen-binding fragment specifically binding to MASP2, wherein the antibody or antigen-binding fragment specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8; and wherein the antibody or antigen-binding fragment specifically binding to MASP2 comprises: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 29.

In some embodiments, there is provided a pharmaceutical composition comprising: an antibody or antigen-binding fragment specifically binding to MASP3 and an antibody or antigen-binding fragment specifically binding to MASP2, wherein the antibody or antigen-binding fragment specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, there is provided a pharmaceutical composition comprising: an antibody or antigen-binding fragment specifically binding to MASP3 and an antibody or antigen-binding fragment specifically binding to MASP2, wherein the antibody or antigen-binding fragment specifically binding to MASP3 comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, there is provided a pharmaceutical composition comprising: an antibody or antigen-binding fragment specifically binding to MASP3 and an antibody or antigen-binding fragment specifically binding to MASP2, wherein the antibody or antigen-binding fragment specifically binding to MASP3 comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, there is provided a pharmaceutical composition comprising: an antibody or antigen-binding fragment specifically binding to MASP3 and an antibody or antigen-binding fragment specifically binding to MASP2, wherein the antibody or antigen-binding fragment specifically binding to MASP3 comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the pharmaceutical compositions described herein, wherein the molar ratio of the antibody or antigen-binding fragment specifically binding to MASP3 to the antibody or antigen-binding fragment specifically binding to MASP2 is about any one of: 20:1, 10:1, 9:1, 8:1, 7:1, 6:1, 5:1, 4:1, 3:1, 2: 1, 1:1, 1:2, 1:3, 1:4, 1:5, 1:6, 1:7, 1:8, 1:9, 1:10 or 1:20. In some embodiments, the molar ratio of the antibody or antigen-binding fragment specifically binding to MASP3 to the antibody or antigen-binding fragment specifically binding to MASP2 is about any one of: 5:1, 4:1, 3:1, 2:1, 1:1, 1:2, 1:3, 1:4 or 1:5. In some embodiments, the molar ratio of the antibody or antigen-binding fragment specifically binding to MASP3 to the antibody or antigen-binding fragment specifically binding to MASP2 is about 2:1 or about 1:1.

### Binding affinity

Binding affinity can be indicated by Kd, Koff, Kon, or Ka. The term "Koff", as used herein, is intended to refer to the off-rate constant for dissociation of an antigen-binding fragment from the antigen-binding fragment/antigen complex, as determined from a kinetic selection set up. The term "Kon", as used herein, is intended to refer to the on-rate constant for association of an antibody to the antigen to form the antigen-binding fragment/antigen complex. The term dissociation constant "Kd", as used herein, refers to the dissociation constant of a particular antibody-antigen interaction, and describes the concentration of antigen required to occupy one half of all of the antigen-binding fragments present in a solution of antibody molecules at equilibrium, and is equal to Koff/Kon. The measurement of Kd presupposes that all binding agents are in solution. In the case where the antigen-binding fragment is tethered to a cell wall, *e.g.,* in a yeast expression system, the corresponding equilibrium rate constant is expressed as EC50, which gives a good approximation of Kd. The affinity constant, Ka, is the inverse of the dissociation constant, Kd.

The dissociation constant (Kd) is used as an indicator showing affinity of antigen-binding fragment moieties to antigens. For example, easy analysis is possible by the Scatchard method using antibodies marked with a variety of marker agents, as well as by using Biacore (made by Amersham Biosciences), analysis of biomolecular interactions by surface plasmon resonance, according to the user's manual and attached kit. The Kd value that can be derived using these methods is expressed in units of M. An antibody that specifically binds to a target may have a Kd of, for example, ≤ 10⁻⁷ M, ≤ 10⁻⁸ M, ≤ 10⁻⁹ M, ≤ 10⁻¹¹ M, ≤ 10⁻¹¹ M, ≤ 10⁻¹² M, or ≤ 10⁻¹³ M.

Binding specificity of the antibody can be determined experimentally by methods known in the art. Such methods comprise, but are not limited to, Western blots, ELISA-, RIA-, ECL-, IRMA-, EIA-, BIAcore-tests and peptide scans.

In some embodiments, the antibody or antigen-binding fragment specifically binding to MASP3 specifically binds to a target MASP3 with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the antibody or antigen-binding fragment specifically binding to MASP3 and MASP3, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1 ×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the antibody or antigen-binding fragment specifically binding to MASP3 and MASP3 is about 10⁻⁷ M to about 10⁻¹³ M.

In some embodiments, the Kd of the binding between the antibody or antigen-binding fragment specifically binding to MASP3 and a non-target is higher than the Kd of the binding between the antibody or antigen-binding fragment specifically binding to MASP3 and the target, and is herein referred to in some embodiments as the binding affinity of the antibody or antigen-binding fragment specifically binding to MASP3 to the target (*e*.*g*., MASP3) is higher than that to a non-target. In some embodiments, the non-target is an antigen that is not MASP3. In some embodiments, the Kd of the binding between the antibody or antigen-binding fragment specifically binding to MASP3 and non-MASP3 target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody or antigen-binding fragment specifically binding to MASP3 and a target MASP3.

In some embodiments, the antibody or antigen-binding fragment specifically binding to MASP2 specifically binds to a target MASP2 with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the antibody or antigen-binding fragment specifically binding to MASP2 and MASP2, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the antibody or antigen-binding fragment specifically binding to MASP2 and MASP2 is about 10⁻⁷ M to about 10⁻¹³ M.

In some embodiments, the Kd of the binding between the antibody or antigen-binding fragment specifically binding to MASP2 and a non-target is higher than the Kd of the binding between the antibody or antigen-binding fragment specifically binding to MASP2 and the target, and is herein referred to in some embodiments as the binding affinity of the antibody or antigen-binding fragment specifically binding to MASP2 to the target (e.g., MASP2) is higher than that to a non-target. In some embodiments, the non-target is an antigen that is not MASP2. In some embodiments, the Kd of the binding between the antibody or antigen-binding fragment specifically binding to MASP2 and a non-MASP2 target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the antibody or antigen-binding fragment specifically binding to MASP2 and a target MASP2.

In some embodiments, the multi-specific antibody according to any one of the present application comprises the first antigen-binding domain specifically binding to MASP3 and the second antigen-binding domain specifically binding to MASP2. Wherein the first antigen-binding domain specifically binds to a target MASP3 with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the first antigen-binding domain and MASP3, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁹ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1 ×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about 10⁻¹³ M, about 1×10⁻¹⁰ M to about 5×10⁻¹³ M, about 1 ×10⁻¹⁰ M to about 1×10⁻¹² M, about 1 ×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the first antigen-binding domain and MASP3 is about 10⁻⁷ M to about 10⁻¹³ M. Wherein the second antigen-binding domain specifically binds to a target MASP2 with a Kd of about 10⁻⁷ M to about 10⁻¹³ M (such as about 10⁻⁷ M to about 10⁻¹³ M, about 10⁻⁸ M to about 10⁻¹³ M, about 10⁻⁹ M to about 10⁻¹³ M, or about 10⁻¹⁰ M to about 10⁻¹² M). Thus in some embodiments, the Kd of the binding between the second antigen-binding domain and MASP2, is about 10⁻⁷ M to about 10⁻¹³ M, about 1×10⁻⁷ M to about 5×10⁻¹³ M, about 10⁻⁷ M to about 10⁻¹² M, about 10⁻⁷ M to about 10⁻¹¹ M, about 10⁻⁷ M to about 10⁻¹⁰ M, about 10⁻⁷ M to about 10⁻⁹ M, about 10⁻⁸ M to about 10⁻¹³ M, about 1×10⁻⁸ M to about 5×10⁻¹³ M, about 10⁻⁸ M to about 10⁻¹² M, about 10⁻⁸ M to about 10⁻¹¹ M, about 10⁻⁸ M to about 10⁻¹⁰ M, about 10⁻⁸ M to about 10⁻⁹ M, about 5×10⁻⁹ M to about 1×10⁻¹³ M, about 5×10⁻⁹ M to about 1×10⁻¹² M, about 5×10⁻⁹ M to about 1×10⁻¹¹ M, about 5×10⁻⁹ M to about 1×10⁻¹⁰ M, about 10⁻⁹ M to about 10⁻¹³ M, about 10⁻⁹ M to about 10⁻¹² M, about 10⁻⁹ M to about 10⁻¹¹ M, about 10⁻⁹ M to about 10⁻¹⁰ M, about 5×10⁻¹⁰ M to about 1×10⁻¹³ M, about 5×10⁻¹⁰ M to about 1×10⁻¹² M, about 5×10⁻¹⁰ M to about 1×10⁻¹¹ M, about 10⁻¹⁰ M to about10⁻¹³ M, about 1 ×10⁻¹⁰ M to about 5×10⁻¹³ M, about 1×10⁻¹⁰ M to about 1×10⁻¹² M, about 1×10⁻¹⁰ M to about 5×10⁻¹² M, about 1×10⁻¹⁰ M to about 1×10⁻¹¹ M, about 10⁻¹¹ M to about 10⁻¹³ M, about 1×10⁻¹¹ M to about 5×10⁻¹³ M, about 10⁻¹¹ M to about 10⁻¹² M, or about 10⁻¹² M to about 10⁻¹³ M. In some embodiments, the Kd of the binding between the second antigen-binding domain and MASP2 is about 10⁻⁷ M to about 10⁻¹³ M.

In some embodiments, the multi-specific antibody according to any one of the present application comprises the first antigen-binding domain specifically binding to MASP3 and the second antigen-binding domain specifically binding to MASP2. Wherein the Kd of the binding between the first antigen-binding domain and a non-target is higher than the Kd of the binding between the first antigen-binding domain and the target, and is herein referred to in some embodiments as the binding affinity of the first antigen-binding domain to the target (e.g., MASP3) is higher than that to a non-target. In some embodiments, the non-target is an antigen that is not MASP3. In some embodiments, the Kd of the binding between the first antigen-binding domain and non-MASP3 target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the first antigen-binding domain and a target MASP3. Wherein the Kd of the binding between the second antigen-binding domain and a non-target is higher than the Kd of the binding between the second antigen-binding domain and the target, and is herein referred to in some embodiments as the binding affinity of the second antigen-binding domain to the target (e.g., MASP2) is higher than that to a non-target. In some embodiments, the non-target is an antigen that is not MASP2. In some embodiments, the Kd of the binding between the second antigen-binding domain and non-MASP2 target can be at least about 10 times, such as about 10-100 times, about 100-1000 times, about 10³-10⁴ times, about 10⁴-10⁵ times, about 10⁵-10⁶ times, about 10⁶-10⁷ times, about 10⁷-10⁸ times, about 10⁸-10⁹ times, about 10⁹-10¹⁰ times, about 10¹⁰-10¹¹ times, or about 10¹¹-10¹² times of the Kd of the binding between the second antigen-binding domain and a target MASP2.

### Nucleic Acids and Vectors

Nucleic acid molecules encoding the antibody or antigen-binding fragment specifically binding to MASP3, the antibody or antigen-binding fragment specifically binding to MASP2 and the multi-specific antibody are also contemplated.

In some embodiments, there is provided a nucleic acid (or a set of nucleic acids) encoding an antibody or antigen-binding fragment specifically binding to MASP3 or the antibody or antigen-binding fragment specifically binding to MASP2 or the multi-specific antibody specifically binding to MASP3 and MASP2, including any one of the antibody or antigen-binding fragment specifically binding to MASP3 or the antibody or antigen-binding fragment specifically binding to MASP2 or the multi-specific antibody specifically binding to MASP3 and MASP2 described herein. In some embodiments, the nucleic acid (or a set of nucleic acids) encoding the antibody or antigen-binding fragment or multi-specific antibody described herein may further comprises a nucleic acid sequence encoding a peptide tag (such as protein purification tag, e.g., His-tag, HA tag).

Also contemplated here are isolated host cells comprising an antibody or antigen-binding fragment specifically binding to MASP3, antibody or antigen-binding fragment specifically binding to MASP2, or multi-specific antibody specifically binding to MASP3 and MASP2; or a nucleic acid molecular coding the antibodies described herein; or a vector comprising a nucleic acid molecular described herein. The present application also includes variants to these nucleic acid sequences. For example, the variants include nucleotide sequences that hybridize to the nucleic acid sequences encoding the antibodies or antigen-binding fragments or multi-specific antibodies of the present application under at least moderately stringent hybridization conditions.

The present application also includes variants to these nucleic acid sequences. For example, the variants include nucleotide sequences that hybridize to the nucleic acid sequences encoding the antibodies or antigen-binding fragments or multi-specific antibodies of the present application under at least moderately stringent hybridization conditions.

The present application also provides vectors in which a nucleic acid of the present application is inserted.

In brief summary, the expression of an antibody or antigen-binding fragment or multi-specific antibody by a natural or synthetic nucleic acid encoding the antibody or antigen-binding fragment or multi-specific antibody can be achieved by inserting the nucleic acid into an appropriate expression vector, such that the nucleic acid is operably linked to 5' and 3' regulatory elements, including for example a promoter *(e.g.,* a lymphocyte-specific promoter) and a 3' untranslated region (UTR). The vectors can be suitable for replication and integration in eukaryotic host cells. Typical cloning and expression vectors contain transcription and translation terminators, initiation sequences, and promoters useful for regulation of the expression of the desired nucleic acid sequence.

The nucleic acids of the present application may also be used for nucleic acid immunization and gene therapy, using standard gene delivery protocols. Methods for gene delivery are known in the art. See, *e.g.,* U.S. Pat. Nos. 5,399,346; 5,580,859; 5,589,466, incorporated by reference herein in their entireties. In some embodiments, the application provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Green and Sambrook (2013, Molecular Cloning: A Laboratory Manual, Cold Spring Harbor Laboratory, New York), and in other virology and molecular biology manuals. Viruses which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers *(see, e.g.,* WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In some embodiments, lentivirus vectors are used. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Additional promoter elements, *e.g*., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

In some embodiments, the expression of the antibody or antigen-binding fragment or multi-specific antibody is inducible. In some embodiments, a nucleic acid sequence encoding the antibody or antigen-binding fragment or multi-specific antibody is operably linked to an inducible promoter, including any inducible promoter described herein.

### Inducible promoters

The use of an inducible promoter provides a molecular switch capable of turning on expression of the polynucleotide sequence which it is operatively linked when such expression is desired, or turning off the expression when expression is not desired. Exemplary inducible promoter systems for use in eukaryotic cells include, but are not limited to, hormone-regulated elements *(e.g., see* Mader, S. and White, J. H. (1993) Proc. Natl. Acad. Sci. USA 90:5603-5607), synthetic ligand-regulated elements (see, *e.g*., Spencer, D. M. et al 1993) Science 262: 1019-1024) and ionizing radiation-regulated elements (*e.g., see* Manome, Y. et al. (1993) Biochemistry 32: 10607-10613; Datta, R. et al. (1992) Proc. Natl. Acad. Sci. USA 89: 1014-10153). Further exemplary inducible promoter systems for use in in vitro or in vivo mammalian systems are reviewed in Gingrich et al. (1998) Annual Rev. Neurosci 21:377-405. In some embodiments, the inducible promoter system for use to express the antibody or antigen-binding fragment or multi-specific antibody is the Tet system. In some embodiments, the inducible promoter system for use to express the antibody or antigen-binding fragment or multi-specific antibody is the lac repressor system from *E. coli.*

An exemplary inducible promoter system for use in the present application is the Tet system. Such systems are based on the Tet system described by Gossen *et al.* (1993). In an exemplary embodiment, a polynucleotide of interest is under the control of a promoter that comprises one or more Tet operator (TetO) sites. In the inactive state, Tet repressor (TetR) will bind to the TetO sites and repress transcription from the promoter. In the active state, e.g., in the presence of an inducing agent such as tetracycline (Tc), anhydrotetracycline, doxycycline (Dox), or an active analog thereof, the inducing agent causes release of TetR from TetO, thereby allowing transcription to take place. Doxycycline is a member of the tetracycline family of antibiotics having the chemical name of 1-dimethylamino-2,4a,5,7,12-pentahydroxy-11-methyl-4,6-dioxo-1,4a,11,11a,12,12a-hexahydrotetracene-3-carboxamide.

In one embodiment, a TetR is codon-optimized for expression in mammalian cells, *e.g*., murine or human cells. Most amino acids are encoded by more than one codon due to the degeneracy of the genetic code, allowing for substantial variations in the nucleotide sequence of a given nucleic acid without any alteration in the amino acid sequence encoded by the nucleic acid. However, many organisms display differences in codon usage, also known as "codon bias" (i.e., bias for use of a particular codon(s) for a given amino acid). Codon bias often correlates with the presence of a predominant species of tRNA for a particular codon, which in turn increases efficiency of mRNA translation. Accordingly, a coding sequence derived from a particular organism (*e.g*., a prokaryote) may be tailored for improved expression in a different organism (*e.g*., a eukaryote) through codon optimization.

Other specific variations of the Tet system include the following "Tet-Off" and "Tet-On" systems. In the Tet-Off system, transcription is inactive in the presence of Tc or Dox. In that system, a tetracycline-controlled transactivator protein (tTA), which is composed of TetR fused to the strong transactivating domain of VP16 from Herpes simplex virus, regulates expression of a target nucleic acid that is under transcriptional control of a tetracycline-responsive promoter element (TRE). The TRE is made up of TetO sequence concatamers fused to a promoter (commonly the minimal promoter sequence derived from the human cytomegalovirus (hCMV) immediate-early promoter). In the absence of Tc or Dox, tTA binds to the TRE and activates transcription of the target gene. In the presence of Tc or Dox, tTA cannot bind to the TRE, and expression from the target gene remains inactive.

Conversely, in the Tet-On system, transcription is active in the presence of Tc or Dox. The Tet-On system is based on a reverse tetracycline-controlled transactivator, rtTA. Like tTA, rtTA is a fusion protein comprised of the TetR repressor and the VP16 transactivation domain. However, a four amino acid change in the TetR DNA binding moiety alters rtTA's binding characteristics such that it can only recognize the tetO sequences in the TRE of the target transgene in the presence of Dox. Thus, in the Tet-On system, transcription of the TRE-regulated target gene is stimulated by rtTA only in the presence of Dox.

Another inducible promoter system is the lac repressor system from *E. coli* (*See* Brown et al., Cell 49:603-612 (1987)). The lac repressor system functions by regulating transcription of a polynucleotide of interest operably linked to a promoter comprising the lac operator (lacO). The lac repressor (lacR) binds to LacO, thus preventing transcription of the polynucleotide of interest. Expression of the polynucleotide of interest is induced by a suitable inducing agent, *e.g*., isopropyl-β-D-thiogalactopyranoside (IPTG).

In order to assess the expression of a polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, *e.g*., enzymatic activity. Expression of the reporter gene is assayed at a suitable time after the DNA has been introduced into the recipient cells. Suitable reporter genes may include genes encoding luciferase, β-galactosidase, chloramphenicol acetyl transferase, secreted alkaline phosphatase, or the green fluorescent protein gene *(e.g.,* Ui-Tel et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some embodiments, there is provided nucleic acid encoding an antibody or antigen-binding fragment or multi-specific antibody according to any of the antigen-binding fragment, antigen-binding protein or multi-specific molecule described herein. In some embodiments, the nucleic acid comprises one or more nucleic acid sequences encoding the heavy and light chains of the antibody or antigen-binding fragment or multi-specific antibody. In some embodiments, each of the one or more nucleic acid sequences is contained in separate vectors. In some embodiments, at least some of the nucleic acid sequences are contained in the same vector. In some embodiments, all of the nucleic acid sequences are contained in the same vector. Vectors may be selected, for example, from the group consisting of mammalian expression vectors and viral vectors (such as those derived from retroviruses, adenoviruses, adeno-associated viruses, herpes viruses, and lentiviruses).

Methods of introducing and expressing genes into a cell are known in the art. In the context of an expression vector, the vector can be readily introduced into a host cell, *e.g*., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Green and Sambrook (2013, Molecular Cloning: A Laboratory Manual, Cold Spring Harbor Laboratory, New York). In some embodiments, the introduction of a polynucleotide into a host cell is carried out by calcium phosphate transfection.

Biological methods for introducing a polynucleotide of interest into a host cell include the use of DNA and RNA vectors. Viral vectors, and especially retroviral vectors, have become the most widely used method of inserting genes into mammalian, *e.g*., human cells. Other viral vectors can be derived from lentivirus, poxviruses, herpes simplex virus 1, adenoviruses and adeno-associated viruses, and the like. *See,* for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome *(e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Regardless of the method used to introduce exogenous nucleic acids into a host cell or otherwise expose a cell to the inhibitor of the present application, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the application.

### Preparation of antibodies or antigen-binding fragments or multi-specific antibodies

In some embodiments, the antibody or antigen-binding fragment (*e.g*. an antibody or antigen-binding fragment specifically binding to MASP3 or MASP2) is a monoclonal antibody. In some embodiments, the antibody or antigen-binding fragment or multi-specific antibody is derived from a monoclonal antibody. In some embodiments, the antibody or antigen-binding fragment or multi-specific antibody comprises V_{H} and V_{L} domains, or variants thereof, from the monoclonal antibody. In some embodiments, the antibody or antigen-binding fragment or multi-specific antibody further comprises C_{H}1 and C_{L} domains, or variants thereof, from the monoclonal antibody. Monoclonal antibodies can be prepared, *e.g*., using known methods in the art, including hybridoma methods, yeast display, phage display methods, or using recombinant DNA methods. Additionally, exemplary yeast display and phage display methods are described herein and in the Examples below. The multi-specific antibody can be prepared by methods known in the art, including chemical coupling method, hybridoma method, and genetic engineering method, *etc..*

In a hybridoma method, a hamster, mouse, or other appropriate host animal is typically immunized with an immunizing agent to elicit lymphocytes that produce or are capable of producing antibodies that will specifically bind to the immunizing agent. Alternatively, the lymphocytes can be immunized *in vitro.* The immunizing agent can include a polypeptide or a fusion protein of the protein of interest. Generally, peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine, and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

In some embodiments, the immortalized cell lines fuse efficiently, support stable high-level expression of antibody by the selected antibody-producing cells, and are sensitive to a medium such as HAT medium. In some embodiments, the immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies.

The culture medium in which the hybridoma cells are cultured can then be assayed for the presence of monoclonal antibodies directed against the polypeptide. The binding specificity of monoclonal antibodies produced by the hybridoma cells can be determined by immunoprecipitation or by an *in vitro* binding assay, such as radioimmunoassay (RIA) or enzyme-linked immunosorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones can be sub cloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells can be grown *in vivo* as ascites in a mammal.

The monoclonal antibodies secreted by the sub clones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

In some embodiments, according to any of the antibodies or antigen-binding fragments or multi-specific antibodies described herein, the antibody or antigen-binding fragment or multi-specific antibody comprises sequences from a clone selected from an antibody library (such as a phage library presenting scFv or Fab fragments). The clone may be identified by screening combinatorial libraries for antibody fragments with the desired activity or activities. For example, a variety of methods are known in the art for generating phage display libraries and screening such libraries for antibodies possessing the desired binding characteristics. Such methods are reviewed, *e.g.,* in Hoogenboom et al., Methods in Molecular Biology 178:1-37 (O'Brien et al., ed., Human Press, Totowa, N.J., 2001) and further described, *e.g.,* in McCafferty et al., Nature 348:552-554; Clackson et al., Nature 352: 624-628 (1991); Marks et al., J. Mol. Biol. 222: 581-597 (1992); Marks and Bradbury, Methods in Molecular Biology 248:161-175 (Lo, ed., Human Press, Totowa, N.J., 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of V_{H} and V_{L} genes are separately cloned by polymerase chain reaction (PCR) and recombined randomly in phage libraries, which can then be screened for antigen-binding phage as described in Winter et al., Ann. Rev. Immunol., 12: 433-455 (1994). Phages typically display antibody fragments, either as scFv fragments or as Fab fragments. Libraries from immunized sources provide high-affinity antibodies to the immunogen without the requirement of constructing hybridomas. Alternatively, the naive repertoire can be cloned (*e.g*., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: U.S. Pat. No. 5,750,373, and US Patent Publication Nos. 2005/0079574, 2005/0119455, 2005/0266000, 2007/0117126, 2007/0160598, 2007/0237764, 2007/0292936, and 2009/0002360.

The antibodies or antigen-binding fragments or multi-specific antibodies can be prepared using phage display to screen libraries for antigen-binding moieties specific to the target antigen (such as MASP3 or MASP2). The library can be a human scFv phage display library having a diversity of at least 1 ×10⁹ (such as at least about any of 1 × 10⁹, 2.5 × 10⁹, 5 × 10⁹, 7.5 × 10⁹, 1 × 10¹⁰, 2.5 × 10¹⁰, 5 × 10¹⁰, 7.5 × 10¹⁰, or 1 × 10¹¹) unique human antibody fragments. In some embodiments, the library is a naive human library constructed from DNA extracted from human PMBCs and spleens from healthy donors, encompassing all human heavy and light chain subfamilies. In some embodiments, the library is a naive human library constructed from DNA extracted from PBMCs isolated from patients with various diseases, such as patients with autoimmune diseases, cancer patients, and patients with infectious diseases. In some embodiments, the library is a semi-synthetic human library, wherein heavy chain CDR3 is completely randomized, with all amino acids (with the exception of cysteine) equally likely to be present at any given position (*see, e.g.,* Hoet, R.M. et al., Nat. Biotechnol. 23(3):344-348, 2005). In some embodiments, the heavy chain CDR3 of the semi-synthetic human library has a length from about 5 to about 24 (such as about any of 5, 6, 7, 8, 9, 10, 11, 12, 13, 14, 15, 16, 17, 18, 19, 20, 21, 22, 23, or 24) amino acids. In some embodiments, the library is a fully-synthetic phage display library. In some embodiments, the library is a non-human phage display library.

Phage clones that bind to the target antigen (such as MASP3 or MASP2) with high affinity can be selected by iterative binding of phage to the target antigen, which is bound to a solid support (such as, for example, beads for solution panning or mammalian cells for cell panning), followed by removal of nonbound phage and by elution of specifically bound phage. The bound phage clones are then eluted and used to infect an appropriate host cell, such as *E. coli* XL1-Blue, for expression and purification. The panning can be performed for multiple (such as about any of 2, 3, 4, 5, 6 or more) rounds with solution panning, cell panning, or a combination of both, to enrich for phage clones binding specifically to the target antigen. Enriched phage clones can be tested for specific binding to the target antigen by any methods known in the art, including for example ELISA and FACS.

Monoclonal antibodies and bispecific molecules can also be made by recombinant DNA methods, such as those described in U.S. Patent No. 4,816,567. DNA encoding the monoclonal antibodies of the application can be readily isolated and sequenced using conventional procedures (*e.g*., by using oligonucleotide probes that are capable of binding specifically to genes encoding the heavy and light chains of murine antibodies). Hybridoma cells as described above or antigen-specific phage clones of the application can serve as a source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy- and light-chain constant regions and/or framework regions in place of the homologous non-human sequences (U.S. Patent No. 4,816,567; Morrison et al.*, supra*) or by covalently joining to the immunoglobulin coding sequence all or part of the coding sequence for a non-immunoglobulin polypeptide. Such a non-immunoglobulin polypeptide can be substituted for the constant regions of an antibody of the application, or can be substituted for the variable domains of one antigen-combining site of an antibody of the application to create a chimeric bivalent antibody. In some embodiments, additional variable domains targeting a different epitope or antigen can be included to generate a chimeric multi-specific antibody.

The antigen-binding proteins can be monovalent antibodies. Methods for preparing monovalent antibodies are known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using any method known in the art.

Chemical coupling method is the technology that was first applied to prepare multi-specific antibodies. In 1985, Brennan used the chemical binding of two monoclonal antibody G1 fragments for the first time to prepare multi-specific antibodies (Brennan M, et al. Preparation of bispecific antibodies by chemical recombination of monoclonal immunoglobulin G1 fragments [J] . Science, 1985, 229(4708):81-83). The chemical coupling method mainly comprises two modes, one is to directly couple two monoclonal antibodies or their derivatives to form multi-specific antibodies; the other is to dissociate two monoclonal antibodies into free light chains and heavy chains through various physical and chemical methods firstly, and then recombine these light chains and heavy chains. The advantages of chemical coupling method are rapidness, simple operation and high recovery rate, but it is also easy to disrupt the antigen-binding domain of antibodies, affect antibody activity, and easy to form polymers.

Preparation of multi-specific antibody by hybridoma cell lines refers to the fusion of two different hybridoma cell lines by cell fusion techniques, followed by identification and isolation of cells that can produce antibodies with specific therapeutics (Kohler, G, et al. Continuous cultures of fused cells secreting antibody of predefined specificity [J] . J Immunol., 2005, 174(5):2453-2455). Because two kinds of hybridoma cells can produce two different light-heavy chains, and the light-heavy chains can be randomly combined, the multi-specific antibody prepared by the method has larger randomness and low preparation efficiency.

Genetic engineering technologies are also currently used to prepare a variety of multi-specific antibodies (Roland E K. Antibody-cytokine fusion proteins [J] . Arch Biochem Biophys., 2012, 526(2):194-205). Editing recombinant antibodies by genetic engineering can solve the problem of random combination by limiting the binding selectivity of two pairs of light-heavy chain in variety ways. KiH (Knob into hole) and CrossMab are two common technologies currently applied to improve the problem of light-heavy chain pairing. KiH technology introduces asymmetric mutation structures into C_{H}3 domain ("knob" mutation refers to substituting a smaller residue with a larger amino acid residue in C_{H}3 domain, whereas "hole" mutation refers to substituting a larger residue with a smaller amino acid residue). The Fc region of engineered multi-specific antibodies is more prone to heterodimerization than homodimerization due to steric effects (Ridgway J B, et al. "Knobs-into-holes" engineering of antibody CH3 domains for heavy chain heterodimerization [J] . Protein Eng. 1996, 9(7):617-621). While introducing a Y349C mutation in the glycosylated C_{H}3 domain can lead to the formation of disulfide bonds between glycosylated heavy chains, enhancing the stability of KiH (Kuglstatter A, et al. Structural differences between glycosylated, disulfide-linked heterodimeric knob-into-hole Fc fragment and its homodimeric knob-knob and hole-hole side products [J] . Protein Eng Des Sel. ,2017, 30(9):649-656).

In addition to steric effects, the charge effect of amino acid residues is also used to enhance heterodimerization between two heavy chains of a multi-specific antibody. "Positive electricity" is generated in one chain and negative electricity is generated in the paired chain through structural simulation and molecular design modification, and the formation of heavy chain heterodimer is promoted by the mode that like charges repel each other and unlike charges attractive each other. The formation of heterodimer can be increased to some extent by the mode of respective mutation K409D and D399K, K409D/K392D and D399K/E356K, or E356K/E357K/D399K and K370E/K409D/K439E in two chains (IGAWA T, *et al.* Methods for producing polypeptides by regulating polypeptide; association: US, 20100015133A1 [P] .2006). Combining the KiH steric effect with the charge effect is also one of the strategies to further enhance heterodimerization.

The CrossMab technology is a new antibody pairing technology developed by Roche based on KiH technology by exchanging the domain of light chain with the heavy chain of one Fab of multi-specific antibodies, with no exchange on the other one. The exchanged light chain will contain a portion of the homologous heavy chain fragment, making them unable to pair with the non-exchanged heavy chain, thereby ensuring the correct combination between the light chains and heavy chains (Schaefer W, et al. Immunoglobulin domain crossover as a generic approach for the production of bispecific IgG antibodies [J ] . Proc Natl Acad Sci USA, 2011, 108 (27):11187-11192). Includes the form of "CrossMab Fab", "CrossMab V_{H}-V_{L}" or "CrossMab C_{H}1-C_{L}" *etc.*

Antibody variable domains with the desired binding specificities (antibody-antigen combining sites) can be fused to immunoglobulin constant-domain sequences. The fusion preferably is with an immunoglobulin heavy-chain constant region, comprising at least part of the hinge, CH2, and CH3 domains. In some embodiments, the heavy-chain constant domain 1 (CH1) containing the site necessary for light-chain binding is present in at least one of the fusions. DNAs encoding the immunoglobulin heavy-chain fusions and, if desired, the immunoglobulin light chain, are inserted into separate expression vectors, and are co-transfected into a suitable host organism. In some embodiments, antibody variable domains targeting different epitopes or different antigens can be fused to immunoglobulin constant-domain sequences to generate a chimeric bispecific antibody.

### Human and Humanized Antibodies

The antibodies or antigen-binding fragments or multi-specific antibodies can comprise humanized antibody moieties or human antibody moieties. Humanized forms of non-human (*e.g*., murine) antibody moieties are chimeric immunoglobulins, immunoglobulin chains, or fragments thereof (such as Fv, Fab, Fab', F(ab')₂, scFv, or other antigen-binding subsequences of antibodies) that typically contain minimal sequence derived from non-human immunoglobulin. Humanized antibody moieties include human immunoglobulins, immunoglobulin chains, or fragments thereof (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibody moieties can also comprise residues that are found neither in the recipient antibody nor in the imported CDR or framework sequences. In general, the humanized antibody can comprise substantially at least one, and typically two, variable domains, in which all or substantially all of the CDR regions correspond to those of a non-human immunoglobulin, and all or substantially all of the FR regions are those of a human immunoglobulin consensus sequence.

Generally, a humanized antibody or humanized antibody moiety has one or more amino acid residues introduced into it from a source that is non-human. These non-human amino acid residues are often referred to as "import" residues, which are typically taken from an "import" variable domain. According to some embodiments, humanization can be essentially performed following the method of Winter and co-workers (Jones et al., Nature, 321: 522-525 (1986); Riechmann et al., Nature, 332: 323-327 (1988); Verhoeyen et al., Science, 239: 1534-1536 (1988)), by substituting rodent CDRs or CDR sequences for the corresponding sequences of a human antibody. Accordingly, such "humanized" antibody moieties are antibody moieties (U.S. Patent No. 4,816,567), wherein substantially less than an intact human variable domain has been substituted by the corresponding sequence from a non-human species. In practice, humanized antibody moieties are typically human antibody moieties in which some CDR residues and possibly some FR residues are substituted by residues from analogous sites in rodent antibodies.

As an alternative to humanization, human antibody moieties can be generated. For example, it is now possible to produce transgenic animals (*e.g*., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., PNAS USA, 90:2551 (1993); Jakobovits et al., Nature, 362:255-258 (1993); Bruggemann et al., Year in Immunol., 7:33 (1993); U.S. Patent Nos. 5,545,806, 5,569,825, 5,591,669; 5,545,807; and WO 97/17852. Alternatively, human antibodies can be made by introducing human immunoglobulin loci into transgenic animals, *e.g*., mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

Human antibodies or human antibody moieties may also be generated by *in vitro* activated B cells (see U.S. Patents 5,567,610 and 5,229,275) or by using various techniques known in the art, including phage display libraries. Hoogenboom and Winter, J. Mol. Biol., 227:381 (1991); Marks et al., J. Mol. Biol., 222:581 (1991). The techniques of Cole *et al.* and Boerner *et al.* are also available for the preparation of human monoclonal antibodies. Cole et al., Monoclonal Antibodies and Cancer Therapy, Alan R. Liss, p. 77 (1985) and Boerner et al., J. Immunol., 147(1): 86-95 (1991).

### Variants of antibodies or antigen-binding fragments

In some embodiments, amino acid sequences of the antibodies or antigen-binding fragments (*e.g*., antibody specifically binding to MASP3 or MASP2, or multi-specific antibody specifically binding to MASP3 and MASP2) variants provided herein are contemplated. For example, it may be desirable to improve the binding affinity and/or other biological properties of the antibody or antigen-binding fragment. Amino acid sequences of an antigen-binding entity variants may be prepared by introducing appropriate modifications into the nucleotide sequence encoding the antigen-binding entity, or by peptide synthesis. Such modifications include, for example, deletions from, and/or insertions into and/or substitutions of residues within the amino acid sequences of the antigen-binding entity. Any combination of deletion, insertion, and substitution can be made to arrive at the final construct, provided that the final construct possesses the desired characteristics, *e.g*., antigen-binding.

In some embodiments, variants of the antibodies or antigen-binding fragments having one or more amino acid substitutions are provided. Sites of interest for substitutional mutagenesis include the HVRs and FRs. Amino acid substitutions may be introduced into an antibody of interest and the products screened for a desired activity, *e.g*., improved bioactivity or antibody affinity. In some embodiments, the amino acid substitutions described herein are limited to "exemplary substitutions" shown in Table 10 of this application. In some embodiments, the amino acid substitutions are limited to "preferred substitutions" shown in Table 10 of this application.

Conservative substitutions are shown in Table 10 below

| **TABLE 10: CONSERVATIVE SUBSTITITIONS** | | |
|---|---|---|
| **Original Residue** | **Exemplary Substitutions** | **Preferred Substitutions** |
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped into different classes according to common side-chain properties:
a. hydrophobic: Norleucine, Met, Ala, Val, Leu, Ile;
b. neutral hydrophilic: Cys, Ser, Thr, Asn, Gln;
c. acidic: Asp, Glu;
d. basic: His, Lys, Arg;
e. residues that influence chain orientation: Gly, Pro;
f. aromatic: Trp, Tyr, Phe.

Non-conservative substitutions will entail exchanging a member of one of these classes for another class.

An exemplary substitutional variant is an affinity matured antibody, which may be conveniently generated, *e.g*., using phage display-based affinity maturation techniques. Briefly, one or more CDR residues are mutated and the variant antibody moieties displayed on phage and screened for a particular biological activity (*e.g*., bioactivity based on RBC lysis inhibition assay or binding affinity). Alterations *(e.g.,* substitutions) may be made in HVRs, *e.g.,* to improve bioactivity based on RBC lysis inhibition assay or antibody affinity. Such alterations may be made in HVR "hotspots," *i.e.,* residues encoded by codons that undergo mutation at high frequency during the somatic maturation process (*see, e.g.,* Chowdhury, Methods Mol. Biol. 207:179-196 (2008)), and/or specificity determining residues (SDRs), with the resulting variant V_{H} and V_{L} being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).)

In some embodiments of affinity maturation, diversity is introduced into the variable genes chosen for maturation by any of a variety of methods (*e.g*., error-prone PCR, chain shuffling, or oligonucleotidedirected mutagenesis). A secondary library is then created. The library is then screened to identify any antibody variants with the desired affinity. Another method to introduce diversity involves HVR-directed approaches, in which several HVR residues (*e.g*., 4-6 residues at a time) are randomized. HVR residues involved in antigen binding may be specifically identified, *e.g*., using alanine scanning mutagenesis or modeling. CDR-H3 and CDR-L3 in particular are often targeted.

In some embodiments, substitutions, insertions, or deletions may occur within one or more HVRs so long as such alterations do not substantially reduce the ability of the antibody to bind antigen. For example, conservative alterations (*e.g*., conservative substitutions as provided herein) that do not substantially reduce binding affinity may be made in HVRs. Such alterations may be outside of HVR "hotspots" or SDRs. In some embodiments of the variant V_{H} and V_{L} sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues *(e.g.,* charged residues such as Arg, Asp, His, Lys, and Glu) are identified and replaced by a neutral or negatively charged amino acid *(e.g.,* Ala or Glu) to determine whether the interaction of the antibody with antigen is affected. Further substitutions may be introduced at the amino acid locations to demonstrate functional sensitivity to the initial substitutions. Alternatively, or additionally, a crystal structure of an antigen-antibody complex can be determined to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antigen-binding moiety with an N-terminal methionyl residue. Other insertional variants of the antigen-binding moiety include the fusion to the N- or C-terminus of the antigen-binding moiety to an enzyme (*e.g*. for ADEPT) or a polypeptide which increases the serum half-life of the antigen-binding moiety.

### Fc Variants

In some embodiments, one or more amino acid modifications may be introduced into the Fc region of an antibody or antigen-binding fragment (*e.g*., a full-length antibody specifically binding to MASP3, full-length antibody specifically binding to MASP2, full-length multi-specific antibody specifically binding to MASP3 and MASP2, or fusion protein comprising the antibody or antigen-binding fragment or multi-specific antibody) provided herein, thereby generating an Fc variant. In some embodiments, the Fc variant has enhanced ADCC effector function, often related to binding to Fc receptors (FcRs). In some embodiments, the Fc variant has decreased ADCC effector function. There are many examples of changes or mutations to Fc sequences that can alter effector function. For example, WO 00/42072 and Shields et al. J Biol. Chem. 9(2): 6591-6604 (2001) describe antibody variants with improved or diminished binding to FcRs. The contents of those publications are specifically incorporated herein by reference.

Antibody-Dependent Cell-Mediated Cytotoxicity (ADCC) is a mechanism of action of therapeutic antibodies against tumor cells. ADCC is a cell-mediated immune defense whereby an effector cell of the immune system actively lyses a target cell *(e.g.,* an infected cell), whose membrane-surface antigens have been bound by specific antigen-binding moieties (*e.g*., an antibody specifically binding to MASP3, antibody specifically binding to MASP2 or multi-specific antibody specifically binding to MASP3 and MASP2). The typical ADCC involves activation of NK cells by antibodies. An NK cell expresses CD16 which is an Fc receptor. This receptor recognizes, and binds to, the Fc portion of an antibody bound to the surface of a target cell. The most common Fc receptor on the surface of an NK cell is called CD16 or FcγRIII. Binding of the Fc receptor to the Fc region of an antibody results in NK cell activation, release of cytolytic granules and consequent target cell apoptosis.

In some embodiments, the application contemplates the variant of antibody or antigen binding fragment specifically binding to MASP3, antibody or antigen binding fragment specifically binding to MASP2 or multi-specific antibody specifically binding to MASP3 and MASP2 (such as a variant of full-length antibody specifically binding to MASP3, full-length antibody specifically binding to MASP2 or full-length multi-specific antibody specifically binding to MASP3 and MASP2) comprising an Fc region that possesses some but not all effector functions, which makes it a desirable candidate for applications in which the half-life of the antibody or antigen-binding fragment specifically binding to MASP3, antibody or antigen-binding fragment specifically binding to MASP2 or multi-specific antibody specifically binding to MASP3 and MASP2 *in vivo* is important yet certain effector functions (such as CDC and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Pat. No. 5,500,362 (see, *e.g.* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); U.S. Pat. No. 5,821,337 (see Bruggemann, M. et al., J. Exp. Med. 166:1351-1361 (1987)). Alternatively, non-radioactive assay methods may be employed (see, for example, ACTI^{™} non-radioactive cytotoxicity assay for flow cytometry (CellTechnology, Inc. Mountain View, Calif.; and CytoTox 96^{™} non-radioactive cytotoxicity assay (Promega, Madison, Wis.). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M. S. et al., Blood 101:1045-1052 (2003); and Cragg, M. S. and M. J. Glennie, Blood 103:2738-2743 (2004)). FcRn binding and *in vivo* clearance/half-life determinations can also be performed using methods known in the art (see, *e.g.,* Petkova, S. B. et al., Int'l. Immunol. 18(12):1759-1769 (2006)).

Antibodies with reduced effector function include those with substitution of one or more of Fc region residues 238, 265, 269, 270, 297, 327 and 329 (U.S. Pat. No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (U.S. Pat. No. 7,332,581).

Certain antibody variants with improved or diminished binding to FcRs are described. (See, *e.g.,* U.S. Pat. No. 6,737,056; WO 2004/056312, and Shields et al., J. Biol. Chem. 9(2): 6591-6604 (2001).)

In some embodiments, alterations are made in the Fc region that result in altered (*i.e.,* either improved or diminished) opsonization, *e.g.* such as described in Moore et al., MAbs. 2(2): 181-189 (2010).

In some embodiments, there is provided an antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) variant comprising a variant Fc region comprising one or more amino acid substitutions which increase half-life and/or improve binding to the neonatal Fc receptor (FcRn). Antibodies with increased half-lives and improved binding to FcRn are described in US2005/0014934A1 (Hinton et al.)*.* Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (U.S. Pat. No. 7,371,826).

*See* also Duncan & Winter, Nature 322:738-40 (1988); U.S. Pat. No. 5,648,260; U.S. Pat. No. 5,624,821; and WO 94/29351 concerning other examples of Fc variants.

Antibodies or antigen-binding fragments specifically binding to MASP3, antibodies or antigen-binding fragments specifically binding to MASP2 or multi-specific antibodies specifically binding to MASP3 and MASP2 (such as full-length antibodies specifically binding to MASP3, full-length antibodies specifically binding to MASP2 or full-length multi-specific antibodies specifically binding to MASP3 and MASP2) comprising any of the Fc variants described herein, or combinations thereof, are contemplated.

### Glycosylation Variants

In some embodiments, an antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) provided herein is altered to increase or decrease the glycosylation extent of the antibody or antigen-binding fragment specifically binding to MASP3, antibody or antigen-binding fragment specifically binding to MASP2 or multi-specific antibody specifically binding to MASP3 and MASP2. Addition or deletion of glycosylation sites to an antibody specifically binding to MASP3, antibody specifically binding to MASP2 or bispecific antibody specifically binding to MASP3 and MASP2 may be conveniently accomplished by altering the amino acid sequence of the antibody or antigen-binding fragment specifically binding to MASP3, antibody or antigen-binding fragment specifically binding to MASP2 or multi-specific antibody specifically binding to MASP3 and MASP2 or polypeptide portion thereof such that one or more glycosylation sites is created or removed.

Where the antibody or antigen-binding fragment specifically binding to MASP3, antibody or antigen-binding fragment specifically binding to MASP2 or multi-specific antibody specifically binding to MASP3 and MASP2 comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al., TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody or antigen-binding fragment specifically binding to MASP3, antibody or antigen-binding fragment specifically binding to MASP2 or multi-specific antibody specifically binding to MASP3 and MASP2 of the application may be made in order to create antibody or antigen-binding fragment specifically binding to MASP3, antibody or antigen-binding fragment specifically binding to MASP2 or multi-specific antibody specifically binding to MASP3 and MASP2 variants with certain improved properties.

The N-glycans attached to the CH2 domain of Fc is heterogeneous. Antibodies or Fc fusion proteins generated in CHO cells are fucosylated by fucosyltransferase activity. *See* Shoji-Hosaka et al., J. Biochem. 2006, 140:777- 83. Normally, a small percentage of naturally occurring afucosylated IgGs may be detected in human serum. N-glycosylation of the Fc is important for binding to Fc 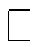 R; and afucosylation of the N-glycan increases Fc's binding capacity to Fc □RIIIa. Increased Fc□RIIIa binding can enhance ADCC, which can be advantageous in certain antibody therapeutic applications in which cytotoxicity is desirable.

In some embodiments, an enhanced effector function can be detrimental when Fc-mediated cytotoxicity is undesirable. In some embodiments, the Fc fragment or CH2 domain is not glycosylated. In some embodiments, the N-glycosylation site in the CH2 domain is mutated to prevent from glycosylation.

In some embodiments, the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) variants are provided comprising an Fc region wherein a carbohydrate structure attached to the Fc region has reduced fucose or lacks fucose, which may improve ADCC function. Specifically, the antibody or antigen-binding fragment specifically binding to MASP3, antibody or antigen-binding fragment specifically binding to MASP2 or multi-specific antibody specifically binding to MASP3 and MASP2 variants are contemplated herein that have reduced fucose relative to the amount of fucose on the same antibody or antigen-binding fragment specifically binding to MASP3, antibody or antigen-binding fragment specifically binding to MASP2 or multi-specific antibody specifically binding to MASP3 and MASP2 produced in a wild-type CHO cell. That is, they are characterized by having a lower amount of fucose than they would otherwise have if produced by native CHO cells *(e.g.,* a CHO cell that produce a native glycosylation pattern, such as, a CHO cell containing a native FUT8 gene). In some embodiments, the antibody or antigen-binding fragment specifically binding to MASP3, antibody or antigen-binding fragment specifically binding to MASP2 or multi-specific antibody specifically binding to MASP3 and MASP2 is one wherein less than about 50%, 40%, 30%, 20%, 10%, or 5% of the N-linked glycans thereon comprise fucose. For example, the amount of fucose in such an antibody or antigen-binding fragment specifically binding to MASP3, antibody or antigen-binding fragment specifically binding to MASP2 or multi-specific antibody specifically binding to MASP3 and MASP2 may be from 1% to 80%, from 1% to 65%, from 5% to 65% or from 20% to 40%. In some embodiments, the antibody or antigen-binding fragment specifically binding to MASP3, antibody or antigen-binding fragment specifically binding to MASP2 or multi-specific antibody specifically binding to MASP3 and MASP2 is one wherein none of the N-linked glycans thereon comprise fucose, *i.e.,* wherein the antibody or antigen-binding fragment specifically binding to MASP3, antibody or antigen-binding fragment specifically binding to MASP2 or multi-specific antibody specifically binding to MASP3 and MASP2 is completely without fucose, or has no fucose or is afucosylated. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ±3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such asα-1,6-fucosyltransferase gene, FUT8, knockout CHO cells (see, *e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) variants are further provided with bisected oligosaccharides, *e.g*., in which a biantennary oligosaccharide attached to the Fc region of the antibody or antigen-binding fragment specifically binding to MASP3, antibody or antigen-binding fragment specifically binding to MASP2 or multi-specific antibody specifically binding to MASP3 and MASP2 is bisected by GlcNAc. Such antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) variants may have reduced fucosylation and/or improved ADCC function. Examples of such antibody variants are described, *e.g.,* in WO 2003/011878 (Jean-Mairet et al.); U.S. Pat. No. 6,602,684 (Umana et al.); US 2005/0123546 (Umana et al.)*,* and Ferrara et al., Biotechnology and Bioengineering, 93(5): 851-861 (2006). Antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) variants with at least one galactose residue in the oligosaccharide attached to the Fc region are also provided. Such antibody or antigen-binding fragment specifically binding to MASP3, antibody or antigen-binding fragment specifically binding to MASP2 or multi-specific antibody specifically binding to MASP3 and MASP2 variants may have improved CDC function. Such antibody variants are described, *e.g.,* in WO 1997/30087 (Patel et al.); WO 1998/58964 (Raju, S.); and WO 1999/22764 (Raju, S.).

In some embodiments, the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) variants comprising an Fc region are capable of binding to an FcγRIII. In some embodiments, the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) variants comprising an Fc region have ADCC activity in the presence of human effector cells *(e.g.,* T cell) or have increased ADCC activity in the presence of human effector cells compared to the otherwise same antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) comprising a human wild-type Fc region.

### Cysteine Engineered Variants

In some embodiments, it may be desirable to create cysteine engineered antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) in which one or more amino acid residues are substituted with cysteine residues. In some embodiments, the substituted residues occur at accessible sites of the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2). By substituting those residues with cysteine, reactive thiol groups are thereby positioned at accessible sites of the antibody or antigen-binding fragment specifically binding to MASP3, antibody or antigen-binding fragment specifically binding to MASP2 or multi-specific antibody specifically binding to MASP3 and MASP2 and may be used to conjugate the antibody or antigen-binding fragment specifically binding to MASP3, antibody or antigen-binding fragment specifically binding to MASP2 or multi-specific antibody specifically binding to MASP3 and MASP2 to other moieties, such as drug moieties or linkerdrug moieties, to create an antibody or antigen-binding fragment specifically binding to MASP3, antibody or antigen-binding fragment specifically binding to MASP2 or multi-specific antibody specifically binding to MASP3 and MASP2 immunoconjugate, as described further herein. Cysteine engineered antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length bispecific antibody specifically binding to MASP3 and MASP2) may be generated as described, e.g., in U.S. Pat. No. 7,521,541.

### Derivatives

In some embodiments, a multi-specific antibody specifically binding to MASP3 and MASP2 (such as a full-length multi-specific antibody specifically binding to MASP3 and MASP2) provided herein may be further modified to contain additional non-proteinaceous moieties that are known in the art and readily available. The moieties suitable for derivatization of the multi-specific antibodies specifically binding to MASP3 and MASP2 include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone) polyethylene glycol, propropylene glycol homopolymers, prolypropylene oxide/ethylene oxide co-polymers, polyoxyethylated polyols (*e.g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the multi-specific antibodies specifically binding to MASP3 and MASP2 may vary, and if more than one polymer are attached, they can be the same or different molecules. In general, the number and/or type of polymers used for derivatization can be determined based on considerations including, but not limited to, the particular properties or functions of the multi-specific antibodies specifically binding to MASP3 and MASP2 to be improved, whether the multi-specific antibodies specifically binding to MASP3 and MASP2 derivative will be used in a therapy under defined conditions, *etc.*

### Pharmaceutical Compositions

The present application provides the pharmaceutical compositions described herein, which comprises: (i) an antibody or antigen-binding fragment specifically binding to MASP3 and (ii) an antibody or antigen-binding fragment specifically binding to MASP2. Besides, also provided herein are compositions (such as pharmaceutical compositions, also referred to herein as formulations) comprising any of the antibodies or antigen-binding fragments specifically binding to MASP3, antibodies or antigen-binding fragments specifically binding to MASP2 or multi-specific antibodies specifically binding to MASP3 and MASP2 (such as a full-length antibody specifically binding to MASP3, full-length antibody specifically binding to MASP2 or full-length multi-specific antibody specifically binding to MASP3 and MASP2), nucleic acids encoding the antibodies or antigen-binding fragments, vectors comprising the nucleic acids encoding the antibodies or antigen-binding fragments, or host cells comprising the nucleic acids or vectors described herein. In some embodiments, there is provided a pharmaceutical composition comprising any one of the antibodies or antigen-binding fragments specifically binding to MASP3, antibodies or antigen-binding fragments specifically binding to MASP2 or multi-specific antibodies specifically binding to MASP3 and MASP2 described herein and a pharmaceutically acceptable carrier.

Suitable formulations of the antibodies or antigen-binding fragments specifically binding to MASP3, antibodies or antigen-binding fragments specifically binding to MASP2 or multi-specific antibodies specifically binding to MASP3 and MASP2 are obtained by mixing an antibody or antigen-binding fragment specifically binding to MASP3, antibody or antigen-binding fragment specifically binding to MASP2 or multi-specific antibody specifically binding to MASP3 and MASP2 having the desired degree of purity with optional pharmaceutically acceptable carriers, excipients or stabilizers *(*Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes *(e.g*. Zn-protein complexes); and/or non-ionic surfactants such as TWEEN^{™}, PLURONICS^{™} or polyethylene glycol (PEG). Exemplary formulations are described in WO98/56418, expressly incorporated herein by reference. Lyophilized formulations adapted for subcutaneous administration are described in WO97/04801. Such lyophilized formulations may be reconstituted with a suitable diluent to a high protein concentration and the reconstituted formulation may be administered subcutaneously to the individual to be treated herein. Lipofectins or liposomes can be used to deliver the antibodies or antigen-binding fragments specifically binding to MASP3, antibodies or antigen-binding fragments specifically binding to MASP2 or multi-specific antibodies specifically binding to MASP3 and MASP2 of this application into cells.

The formulation herein may also contain one or more active compounds in addition to the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) as necessary for the particular indication being treated, preferably those with complementary activities that do not adversely affect each other. For example, it may be desirable to further provide other agents with therapeutic activity, such as antibiotics agent in addition to the antibody or antigen-binding fragment specifically binding to MASP3, antibody or antigen-binding fragment specifically binding to MASP2 or multi-specific antibody specifically binding to MASP3 and MASP2. Such molecules are suitably present in combination in amounts that are effective for the purpose intended. The effective amount of such other active agents depends on the amount of antibody or antigen-binding fragment specifically binding to MASP3, antibody or antigen-binding fragment specifically binding to MASP2 or multi-specific antibody specifically binding to MASP3 and MASP2 present in the formulation, the type of disease or disorder or treatment, and other factors discussed above. These are generally used in the same dosages and with administration routes as described herein or about from 1 to 99% of the heretofore employed dosages.

The antibodies or antigen-binding fragments specifically binding to MASP3, antibodies or antigen-binding fragments specifically binding to MASP2 or multi-specific antibodies specifically binding to MASP3 and MASP2 (such as a full-length antibodies specifically binding to MASP3, full-length antibodies specifically binding to MASP2 or full-length multi-specific antibodies specifically binding to MASP3 and MASP2) may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Sustained-release preparations may be prepared.

Sustained-release preparations of the antibodies or antigen-binding fragments specifically binding to MASP3, antibodies or antigen-binding fragments specifically binding to MASP2 or multi-specific antibodies specifically binding to MASP3 and MASP2 (such as full-length antibodies specifically binding to MASP3, full-length antibodies specifically binding to MASP2 or full-length multi-specific antibodies specifically binding to MASP3 and MASP2) can be prepared. Suitable examples of sustained-release preparations include semipermeable matrices of solid hydrophobic polymers containing the antibody (or fragment thereof), which matrices are in the form of shaped articles, *e.g*., films, or microcapsules. Examples of sustained-release matrices include polyesters, hydrogels (for example, poly(2-hydroxyethylmethacrylate ), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D (-)-3-hydroxybutyric acid. While polymers such as ethylene-vinyl acetate and lactic acid-glycolic acid enable release of molecules for over 100 days, certain hydro gels release proteins for shorter time periods. When encapsulated antibody remain in the body for a long time, they can denature or aggregate as a result of exposure to moisture at 37 °C, resulting in a loss of biological activity and possible changes in immunogenicity. Rational strategies can be devised for stabilization of antibodies or antigen-binding fragments specifically binding to MASP3, antibodies or antigen-binding fragments specifically binding to MASP2 or multi-specific antibodies specifically binding to MASP3 and MASP2 depending on the mechanism involved. For example, if the aggregation mechanism is discovered to be intermolecular S-S bond formation through thio-disulfide interchange, stabilization can be achieved by modifying sulfhydryl residues, lyophilizing from acidic solutions, controlling moisture content, using appropriate additives, and developing specific polymer matrix compositions.

In some embodiments, the antibody or antigen-binding fragment specifically binding to MASP3, antibody or antigen-binding fragment specifically binding to MASP2 or multi-specific antibody specifically binding to MASP3 and MASP2 (such as a full-length antibody specifically binding to MASP3, full-length antibody specifically binding to MASP2 or full-length multi-specific antibody specifically binding to MASP3 and MASP2) is formulated in a buffer comprising a citrate, NaCl, acetate, succinate, glycine, polysorbate 80 (Tween 80), or any combination of the foregoing. In some embodiments, the antibody or antigen-binding fragment specifically binding to MASP3, antibody or antigen-binding fragment specifically binding to MASP2 or multi-specific antibody specifically binding to MASP3 and MASP2 is formulated in a buffer having a pH between about 4-9.

The formulations to be used for *in vivo* administration must be sterile. This is readily accomplished by, e.g., filtration through sterile filtration membranes.

### Methods of preventing or treating disease

In certain aspects, there is provided a method of preventing or treating a disease in an individual in need comprising administering to the individual an effective amount of a composition comprising any of the antibodies or antigen-binding fragments specifically binding to MASP3, and/or composition comprising any of the multi-specific antibodies specifically binding to MASP3 and MASP2 described herein, and/or composition comprising the antibodies or antigen-binding fragments specifically binding to MASP3 and the antibodies or antigen-binding fragments specifically binding to MASP2 described herein.

In some embodiments, the present application also provides the use of any of the antibodies or antigen-binding fragments specifically binding to MASP3 or compositions thereof, and/or any of the multi-specific antibodies specifically binding to MASP3 and MASP2 or compositions thereof, and/or the antibodies or antigen-binding fragments specifically binding to MASP3 and the antibodies or antigen-binding fragments specifically binding to MASP2 or compositions thereof in the manufacture of a medicament for preventing or treating a disease or condition in an individual in need.

In some embodiments, the disease or condition is related to complement dysregulation, the disease or condition comprises autoimmune disease, transplantation-related disease, inflammatory disease, hemopathy, coagulation disease, angiogenesis-dependent diseases and/or viral infectious disease . In some embodiments, the disease or condition selected from the group consisting of ischemia reperfusion injury, atherosclerosis, mesangial proliferative glomerulonephritis, membranous glomerulonephritis, membranoproliferative glomerulonephritis, acute post infection glomerulonephritis, cryoglobulinemic glomerulonephritis, lupus nephritis, systemic lupus erythematosus (SLE), Henoch Schonlein purpura nephritis, IgA nephropathy, ischemic shock, hemolytic anemia, autoimmune thrombotic thrombocytopenic purpura (TTP), hemolytic uremic syndrome (HUS), atypical hemolytic uremic syndrome (aHUS), paroxysmal nocturnal hemoglobinuria (PNH), transplant-associated TMA, Upshaw-Schulman syndrome, arthritis, traumatic brain injury, aspiration pneumonia, neuromyelitis optica, multiple sclerosis, amyotrophic lateral sclerosis (ALS), chronic obstructive pulmonary disease (COPD), C3 glomerulopathy, transplant rejection, graft-versus-host disease (GVHD), sepsis, systemic inflammatory response syndrome (SIRS) Acute respiratory distress syndrome (ARDS), ANCA vasculitis, antiphospholipid syndrome, myasthenia gravis, Degos disease, disseminated intravascular coagulation (DIC), Angiogenesis dependent cancer, age-related macular degeneration, retinopathy, proliferative diabetes, retinopathy secondary vitreous hemorrhage, neovascular glaucoma, corneal neovascularization, retinopathy of prematurity and respiratory distress syndrome or pneumonia caused by coronavirus infection.

In some embodiments, according to the methods or uses described herein, the antibody or antigen-binding fragment specifically binding to MASP3 comprises: a heavy chain variable domain (V_{H}) comprising a heavy chain complementarity determining region (HC-CDR) 1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a light chain variable domain (V_{L}) comprising a light chain complementarity determining region (LC-CDR) 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

In some embodiments, according to the methods or uses described herein, the antibody or antigen-binding fragment specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of any one of SEQ ID NOs: 4-5, or a variant thereof comprising up to about 3 amino acid substitutions, an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions.

In some embodiments, according to the methods or uses described herein, the antibody or antigen-binding fragment specifically binding to MASP3 comprises: a V_{H} comprising an HC-CDR1, an HC-CDR2, and an HC-CDR3 of a V_{H} comprising the amino acid sequence of any one of SEQ ID NOs: 11-15; and a V_{L} comprising an LC-CDR1, an LC-CDR2, and an LC-CDR3 of a V_{L} comprising the amino acid sequence of any one of SEQ ID NOs: 16-22.

In some embodiments, according to the methods or uses described herein, the antibody or antigen-binding fragment specifically binding to MASP3 comprises: (i) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 16; or (ii) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19.

In some embodiments, according to the methods or uses described herein, the antibody or antigen-binding fragment specifically binding to MASP3 comprises: (i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3, or a variant thereof comprising up to about 5 amino acid substitutions in the HC-CDRs; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7, or a variant thereof comprising up to about 5 amino acid substitutions in the LC-CDRs; or (ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3, or a variant thereof comprising up to about 5 amino acid substitutions in the HC-CDRs; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8, or a variant thereof comprising up to about 5 amino acid substitutions in the LC-CDRs.

In some embodiments, according to the methods or uses described herein, the antibody or antigen-binding fragment specifically binding to MASP3 comprises: a V_{H} comprising the amino acid sequence of any one of SEQ ID NOs: 11-15, or a variant thereof having at least about 80% *(e.g.,* at least 80%, 85%, 90%, 95%, 96%, 97%, 98%, or 99%) sequence identity to the amino acid sequence of any one of SEQ ID NOs: 11-15; and a V_{L} comprising the amino acid sequence of any one of SEQ ID NOs: 16-22, or a variant thereof having at least about 80% *(e.g.,* at least 80%, 85%, 90%, 95%, 96%, 97%, 98%, or 99%) sequence identity to the amino acid sequence of any one of SEQ ID NOs: 16-22.

In some embodiments, according to the methods or uses described herein, the antibody or antigen-binding fragment specifically binding to MASP3 comprises: (i) a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 16, or a variant thereof having at least about 80% sequence identity to the amino acid sequence of SEQ ID NO: 16; (ii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 18, or a variant thereof having at least about 80% sequence identity to the amino acid sequence of SEQ ID NO: 18; (iv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 18, or a variant thereof having at least about 80% sequence identity to the amino acid sequence of SEQ ID NO: 18; (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (vii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 18, or a variant thereof having at least about 80% sequence identity to the amino acid sequence of SEQ ID NO: 18; (viii) a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 19, or a variant thereof having at least about 80% sequence identity to the amino acid sequence of SEQ ID NO: 19; (ix) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 20, or a variant thereof having at least about 80% sequence identity to the amino acid sequence of SEQ ID NO: 20; (x) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 20, or a variant thereof having at least about 80% sequence identity to the amino acid sequence of SEQ ID NO: 20; (xi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (xii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (xiii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 22, or a variant thereof having at least about 80% sequence identity to the amino acid sequence of SEQ ID NO: 22; (xiv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; (xv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22; (xvi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 21, or a variant thereof having at least about 80% sequence identity to the amino acid sequence of SEQ ID NO: 21; or (xvii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, there is provided a method of preventing or treating a disease in an individual in need comprising administering to the individual an effective amount of a multi-specific antibody (e.g., bispecific antibody) specifically binding to MASP3 and MASP2, or a composition comprising the multi-specific antibody, wherein the multi-specific antibody comprises a first antigen-binding domain specifically binding to MASP3, and a second antigen-binding domain specifically binding to MASP2.

In some embodiments, there is provided the use of the multi-specific antibody (e.g., bispecific antibody) specifically binding to MASP3 and MASP2, or the composition comprising the multi-specific antibody in the manufacture of a medicament for preventing or treating a disease or condition in an individual in need, wherein the multi-specific antibody comprises a first antigen-binding domain specifically binding to MASP3, and a second antigen-binding domain specifically binding to MASP2.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: (a) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); or (b) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P. In some embodiments, the second antigen-binding domain comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); or (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, according to the methods or uses described herein, the multi-specific antibody comprises a first antigen-binding domain specifically binding to MASP3, and a second antigen-binding domain specifically binding to MASP2, wherein the second antigen-binding domain comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); or (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

In some embodiments, according to the methods or uses described herein, the first antigen-binding domain specifically binding to MASP3 comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of any one of SEQ ID NOs: 4-5, or a variant thereof comprising up to about 3 amino acid substitutions, an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions.

In some embodiments, according to the methods or uses described herein, the first antigen-binding domain specifically binding to MASP3 comprises: (i) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 4; an LC-CDR2 comprising the amino acid sequence of SEQ ID NO: 6; and an LC-CDR3 comprising the amino acid sequence of SEQ ID NO: 7; or (ii) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8.

In some embodiments, according to the methods or uses described herein, the second antigen-binding domain specifically binding to MASP2 comprises: a V_{H} comprises an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprises an LC-CDR1 comprising the amino acid sequence of any one of SEQ ID NO: 26, an LC-CDR2 comprising the amino acid sequence of SEQ ID NO: 27, and an LC-CDR3 comprising the amino acid sequence of any one of SEQ ID NOs: 28-32, or a variant thereof comprising up to about 3 amino acid substitutions.

In some embodiments, according to the methods or uses described herein, the second antigen-binding domain specifically binding to MASP2 comprises: (i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28; (ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29; (iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30; (iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or (v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

In some embodiments, according to the methods or uses described herein, the first antigen-binding domain specifically binding to MASP3 comprises: a V_{H} comprising the amino acid sequence of any one of SEQ ID NOs: 11-15, or a variant thereof having at least about 80% *(e.g.,* at least 80%, 85%, 90%, 95%, 96%, 97%, 98%, or 99%) sequence identity to the amino acid sequence of any one of SEQ ID NOs: 11-15; and a V_{L} comprising the amino acid sequence of any one of SEQ ID NOs: 16-22, or a variant thereof having at least about 80% *(e.g.,* at least 80%, 85%, 90%, 95%, 96%, 97%, 98%, or 99%) sequence identity to the amino acid sequence of any one of SEQ ID NOs: 16-22.

In some embodiments, according to the methods or uses described herein, the first antigen-binding domain specifically binding to MASP3 comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, according to the methods or uses described herein, the second antigen-binding domain specifically binding to MASP2 comprises: a V_{H} comprising the amino acid sequence of SEQ ID NO: 34, or a variant thereof having at least about 80% *(e.g.,* at least 80%, 85%, 90%, 95%, 96%, 97%, 98%, or 99%) sequence identity to the amino acid sequence of SEQ ID NO: 34; and a V_{L} comprising the amino acid sequence of any one of SEQ ID NOs: 36-41, or a variant thereof having at least about 80% *(e.g.,* at least 80%, 85%, 90%, 95%, 96%, 97%, 98%, or 99%) sequence identity to the amino acid sequence of any one of SEQ ID NOs: 36-41.

In some embodiments, according to the methods or uses described herein, the second antigen-binding domain specifically binding to MASP2 comprises: (i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36; (ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; (iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38; (iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39; (v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or (vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8; wherein the second antigen-binding domain comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19; wherein the second antigen-binding domain comprises: a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 37.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, according to the methods or uses described herein, the multi-specific antibody comprises a first antigen-binding domain specifically binding to MASP3, and a second antigen-binding domain specifically binding to MASP2, wherein the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; wherein the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, there is provided a method of treating or preventing a disease or condition in an individual in need comprising administering an effective amount of a composition comprising the multi-specific antibody specifically binding to MASP3 and MASP2 is more efficacious than administering a same valency amount of an antibody specifically binding to MASP3 or a same valency amount of an antibody specifically binding to MASP2. In some embodiments, the method comprising administering an effective amount of a multi-specific antibody specifically binding to MASP3 and MASP2 enhances the inhibition of MASP2 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to MASP3 or a same valency amount of an antibody specifically binding to MASP2. In some embodiments, the method comprising administering an effective amount of a multi-specific antibody specifically binding to MASP3 and MASP2 enhances the inhibition of MASP3 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to MASP3 or a same valency amount of an antibody specifically binding to MASP2. In some embodiments, the method comprising administering an effective amount of a multi-specific antibody specifically binding to MASP3 and MASP2 enhances the inhibition of MASP2 and MASP3 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody specifically binding to MASP3 or a same valency amount of an antibody specifically binding to MASP2.

In some embodiments, there is provided a method of preventing or treating a disease or condition in an individual in need comprising administering to the individual an effective amount of a pharmaceutical composition comprising (i) an antibody or antigen-binding fragment specifically binding to MASP3 and (ii) an antibody or antigen-binding fragment specifically binding to MASP2.

In some embodiments, there is provided the use of the composition comprising (i) an antibody or antigen-binding fragment specifically binding to MASP3 and (ii) an antibody or antigen-binding fragment specifically binding to MASP2 in the manufacture of a medicament for treating a disease or condition in an individual in need.

In some embodiments, there is provided a method of preventing or treating a disease or condition in an individual in need comprising administering to the individual: (i) an antibody or antigen-binding fragment specifically binding to MASP3 and (ii) an antibody or antigen-binding fragment specifically binding to MASP2.

In some embodiments, there is provided the use of (i) an antibody or antigen-binding fragment specifically binding to MASP3 and (ii) an antibody or antigen-binding fragment specifically binding to MASP2 in the manufacture of a medicament for treating a disease or condition in an individual in need.

In some embodiments, the antibody or antigen-binding fragment specifically binding to MASP3 and the antibody or antigen-binding fragment specifically binding to MASP2 are administered concurrently. In some embodiments, the antibody or antigen-binding fragment specifically binding to MASP3 and the antibody or antigen-binding fragment specifically binding to MASP2 are administered consecutively.

In some embodiments, the antibody or antigen-binding fragment specifically binding to MASP3 in the method or use comprises: (a) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); (b) a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P; or (c) a V_{H} comprising an HC-CDR1, an HC-CDR2, and an HC-CDR3 of a V_{H} comprising the amino acid sequence of any one of SEQ ID NOs: 11-15; and a V_{L} comprising an LC-CDR1, an LC-CDR2, and an LC-CDR3 of a V_{L} comprising the amino acid sequence of any one of SEQ ID NOs: 16-22.

In some embodiments, the antibody or antigen-binding fragment specifically binding to MASP2 in the method or use comprises: (a) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); (b) a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S; or (c) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of any one of SEQ ID NOs: 36-41.

In some embodiments, the antibody or antigen-binding fragment specifically binding to MASP3 in the method or use comprises: (i) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 16; or (ii) a V_{H} comprising an HC-CDR1, an HC-CDR2, and an HC-CDR3 of a V_{H} comprising the amino acid sequence of SEQ ID NO: 11; and a V_{L} comprising an LC-CDR1, an LC-CDR2, and an LC-CDR3 of a V_{L} comprising the amino acid sequence of SEQ ID NO: 19.

In some embodiments, the antibody or antigen-binding fragment specifically binding to MASP2 in the method or use comprises: (i) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:36; (ii) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 37; (iii) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:38; (iv) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 39; (v) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO:40; or (vi) a V_{H} comprising an HC-CDR1, an HC-CDR2, and an HC-CDR3 of a V_{H} comprising the amino acid sequence of SEQ ID NO: 34; and a V_{L} comprising an LC-CDR1, an LC-CDR2, and an LC-CDR3 of a V_{L} comprising the amino acid sequence of SEQ ID NO: 41.

In some embodiments, the antibody or antigen-binding fragment specifically binding to MASP3 in the method or use comprises: a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of any one of SEQ ID NOs: 4-5, or a variant thereof comprising up to about 3 amino acid substitutions, an LC-CDR2 comprising the amino acid sequence of SEQ ID NO: 6, and an LC-CDR3 comprising the amino acid sequence of any one of SEQ ID NOs: 7-8, or a variant thereof comprising up to about 3 amino acid substitutions.

In some embodiments, the antibody or antigen-binding fragment specifically binding to MASP3 in the method or use comprises: (i) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 4; an LC-CDR2 comprising the amino acid sequence of SEQ ID NO: 6; and an LC-CDR3 comprising the amino acid sequence of SEQ ID NO: 7; or (ii) a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8.

In some embodiments, the antibody or antigen-binding fragment specifically binding to MASP2 in the method or use comprises: a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of any one of SEQ ID NOs: 28-32, or a variant thereof comprising up to about 3 amino acid substitutions.

In some embodiments, the antibody or antigen-binding fragment specifically binding to MASP2 in the method or use comprises: (i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28; (ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29; (iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30; (iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or (v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

In some embodiments, the antibody or antigen-binding fragment specifically binding to MASP3 in the method or use comprises: a V_{H} comprising the amino acid sequence of any one of SEQ ID NOs: 11-15, or a variant thereof having at least about 80% *(e.g.,* at least 80%, 85%, 90%, 95%, 96%, 97%, 98%, or 99%) sequence identity to the amino acid sequence of any one of SEQ ID NOs: 11-15; and a V_{L} comprising the amino acid sequence of any one of SEQ ID NOs: 16-22, or a variant thereof having at least about 80% *(e.g.,* at least 80%, 85%, 90%, 95%, 96%, 97%, 98%, or 99%) sequence identity to the amino acid sequence of any one of SEQ ID NOs: 16-22.

In some embodiments, the antibody or antigen-binding fragment specifically binding to MASP3 in the method or use comprises: (i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17; (ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17; (iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; (iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; (v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or (vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

In some embodiments, the antibody or antigen-binding fragment specifically binding to MASP2 in the method or use comprises: a V_{H} comprising the amino acid sequence of SEQ ID NO: 34, or a variant thereof having at least about 80% *(e.g.,* at least 80%, 85%, 90%, 95%, 96%, 97%, 98%, or 99%) sequence identity to the amino acid sequence of SEQ ID NO: 34; and a V_{L} comprising the amino acid sequence of any one of SEQ ID NOs: 36-41, or a variant thereof having at least about 80% *(e.g.,* at least 80%, 85%, 90%, 95%, 96%, 97%, 98%, or 99%) sequence identity to the amino acid sequence of any one of SEQ ID NOs: 36-41.

In some embodiments, the antibody or antigen-binding fragment specifically binding to MASP2 in the method or use comprises: (i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36; (ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; (iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38; (iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39; (v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or (vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

In some embodiments, the antibody or antigen-binding fragment specifically binding to MASP3 in the method or use comprises: a V_{H} comprising an HC-CDR1 comprising the amino acid sequence of SEQ ID NO: 1; an HC-CDR2 comprising the amino acid sequence of SEQ ID NO: 2; and an HC-CDR3 comprising the amino acid sequence of SEQ ID NO: 3; and a V_{L} comprising an LC-CDR1 comprising the amino acid sequence of SEQ ID NO: 5; an LC-CDR2 comprising the amino acid sequence of SEQ ID NO: 6; and an LC-CDR3 comprising the amino acid sequence of SEQ ID NO: 8; and wherein the antibody or antigen-binding fragment specifically binding to MASP2 comprises: a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29.

In some embodiments, the antibody or antigen-binding fragment specifically binding to MASP3 in the method or use comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the antibody or antigen-binding fragment specifically binding to MASP3 in the method or use comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the antibody or antigen-binding fragment specifically binding to MASP3 in the method or use comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the antibody or antigen-binding fragment specifically binding to MASP3 in the method or use comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein the antibody or antigen-binding fragment specifically binding to MASP2 comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

In some embodiments, the method comprises administering pharmaceutical compositions, the compositions comprises: the antibody or antigen-binding fragment specifically binding to MASP3 described herein and the antibody or antigen-binding fragment specifically binding to MASP2 described herein, wherein the molar ratio of the antibody or antigen-binding fragment specifically binding to MASP3 to the antibody or antigen-binding fragment specifically binding to MASP2 is about any one of: 20:1, 10:1, 9:1, 8:1, 7:1, 6:1, 5:1, 4:1, 3:1, 2: 1, 1:1, 1:2, 1:3, 1:4, 1:5, 1:6, 1:7, 1:8, 1:9, 1:10 or 1:20. In some embodiments, the molar ratio of the antibody or antigen-binding fragment specifically binding to MASP3 to the antibody or antigen-binding fragment specifically binding to MASP2 is about any one of: 5:1, 4:1, 3:1, 2:1, 1:1, 1:2, 1:3, 1:4 or 1:5. In some embodiments, the molar ratio of the antibody or antigen-binding fragment specifically binding to MASP3 to the antibody or antigen-binding fragment specifically binding to MASP2 is about 2:1 or about 1:1.

In some embodiments, the method comprises administering (i) an antibody or antigen-binding fragment specifically binding to MASP3 described herein and (ii) an antibody or antigen-binding fragment specifically binding to MASP2 described herein, wherein the molar ratio of the antibody or antigen-binding fragment specifically binding to MASP3 to the antibody or antigen-binding fragment specifically binding to MASP2 is about any one of: 20:1, 10:1, 9:1, 8:1, 7:1, 6:1, 5:1, 4:1, 3:1, 2: 1, 1:1, 1:2, 1:3, 1:4, 1:5, 1:6, 1:7, 1:8, 1:9, 1:10 or 1:20. In some embodiments, the molar ratio of the antibody or antigen-binding fragment specifically binding to MASP3 to the antibody or antigen-binding fragment specifically binding to MASP2 is about any one of: 5:1, 4:1, 3:1, 2:1, 1:1, 1:2, 1:3, 1:4 or 1:5. In some embodiments, the molar ratio of the antibody or antigen-binding fragment specifically binding to MASP3 to the antibody or antigen-binding fragment specifically binding to MASP2 is about 2:1 or about 1:1.

In some embodiments, there is provided a method of preventing or treating a disease or condition in an individual in need, comprising administering an effective amount of a composition comprising antibody or antigen-binding fragment specifically binding to MASP3 described herein and antibody or antigen-binding fragment specifically binding to MASP2 described herein, which is more efficacious than administering a same valency amount of an antibody or antigen-binding fragment specifically binding to MASP3 or a same valency amount of an antibody or antigen-binding fragment specifically binding to MASP2. In some embodiments, the method comprising administering an effective amount of a composition comprising antibody or antigen-binding fragment specifically binding to MASP3 described herein and antibody or antigen-binding fragment specifically binding to MASP2 described herein enhances the inhibition of MASP3 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2. In some embodiments, the method comprising administering an effective amount of a composition comprising antibody or antigen-binding fragment specifically binding to MASP3 described herein and antibody or antigen-binding fragment specifically binding to MASP2 described herein enhances the inhibition of MASP2 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2. In some embodiments, the method comprising administering an effective amount of a composition comprising antibody or antigen-binding fragment specifically binding to MASP3 described herein and antibody or antigen-binding fragment specifically binding to MASP2 described herein enhances the inhibition of MASP3 and MASP2 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2.

In some embodiments, there is provided a method of preventing or treating a disease or condition in an individual in need, comprising administering an effective amount of (i) an antibody or antigen-binding fragment specifically binding to MASP3 described herein and (ii) an antibody or antigen-binding fragment specifically binding to MASP2 described herein, which is more efficacious than administering a same valency amount of an antibody or antigen-binding fragment specifically binding to MASP3 or a same valency amount of an antibody or antigen-binding fragment specifically binding to MASP2. In some embodiments, the method comprising administering an effective amount of (i) an antibody or antigen-binding fragment specifically binding to MASP3 described herein (ii) an antibody or antigen-binding fragment specifically binding to MASP2 described herein enhances the inhibition of MASP3 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2. In some embodiments, the method comprising administering an effective amount of (i) an antibody or antigen-binding fragment specifically binding to MASP3 described herein (ii) an antibody or antigen-binding fragment specifically binding to MASP2 described herein enhances the inhibition of MASP2 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2. In some embodiments, the method comprising administering an effective amount of (i) an antibody or antigen-binding fragment specifically binding to MASP3 described herein (ii) an antibody or antigen-binding fragment specifically binding to MASP2 described herein enhances the inhibition of MASP3 and MASP2 induced cell apoptosis activity by about any one of 10%, 20%, 30%, 40%, 50%, 60%, 70%, 80%, 90%, 100%, 2-fold, 5-fold, 10-fold, 20-fold, 50-fold, or 100-fold more compared to administering a same valency amount of an antibody or antigen-binding fragment specifically binding to MASP3 or a same valency amount of an antibody or antigen-binding fragment specifically binding to MASP2.

### Articles of Manufacture and Kits

In some embodiments of the application, there is provided an article of manufacture containing materials useful for preventing or treating a disease or condition in an individual in need, or for delivering a antibody or antigen-binding fragment (such as an antibody specifically binding to MASP3 and antibody specifically binding to MASP2) or multi-specific antibody (such as a bispecific antibody specifically binding to MASP3 and MASP2) or a pharmaceutical composition comprising antibodies or antigen-binding fragments specifically binding to MASP3 or antibodies or antigen-binding fragments specifically binding to MASP2, to a cell attached by a pathogen expressing MASP3 and MASP2. The article of manufacture can comprise a container and a label or package insert on or associated with the container. Suitable containers include, for example, bottles, vials, syringes, *etc.* The containers may be formed from a variety of materials such as glass or plastic. Generally, the container holds a composition which is effective for treating a disease or disorder described herein, and may have a sterile access port (for example the container may be an intravenous solution bag or a vial having a stopper pierceable by a hypodermic injection needle). In some embodiments, at least one active agent in the composition is a antibody or antigen-binding fragment or multi-specific antibody of the application. The label or package insert indicates that the composition is used for treating the particular condition. The label or package insert will further comprise instructions for administering the multi-specific antibody or the pharmaceutical composition to the patient. Articles of manufacture and kits comprising combinatorial therapies described herein are also contemplated.

Package insert refers to instructions customarily included in commercial packages of therapeutic products that contain information about the indications, usage, dosage, administration, contraindications and/or warnings concerning the use of such therapeutic products. In some embodiments, the package insert indicates that the composition is used for treating bacterial infections. In some embodiments, the package insert indicates that the composition is used for treating a disease or condition in an individual in need.

Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, *e.g*., useful for preventing or treating a disease or condition in an individual in need, or for delivering an antibody or antigen-binding fragment specifically binding to MASP3, antibody or antigen-binding fragment specifically binding to MASP2, or multi-specific antibody specifically binding to MASP3 and MASP2 (such as a full-length antibody specifically binding to MASP3, full-length antibody specifically binding to MASP2, or full-length bispecific antibody specifically binding to MASP3 and MASP2) to a cell attached by a pathogen MASP3 antigen or MASP2, optionally in combination with the articles of manufacture. Kits of the application include one or more containers comprising antibody or antigen-binding fragment specifically binding to MASP3, antibody or antigen-binding fragment specifically binding to MASP2, or multi-specific antibody specifically binding to MASP3 and MASP2 composition (or unit dosage form and/or article of manufacture), and in some embodiments, further comprise another agent (such as the agents described herein) and/or instructions for use in accordance with any of the methods described herein. The kit may further comprise a description of selection of individuals suitable for treatment. Instructions supplied in the kits of the application are typically written instructions on a label or package insert *(e.g.,* a paper sheet included in the kit), but machine-readable instructions (*e.g*., instructions carried on a magnetic or optical storage disk) are also acceptable.

For example, in some embodiments, the kit comprises a composition comprising an antibody or antigen-binding fragment specifically binding to MASP3, antibody or antigen-binding fragment specifically binding to MASP2, or multi-specific antibody specifically binding to MASP3 and MASP2 (such as a full-length antibody specifically binding to MASP3, full-length antibody specifically binding to MASP2, or full-length multi-specific antibody specifically binding to MASP3 and MASP2). In some embodiments, the kit comprises a) a composition comprising any one of the antibodies or antigen-binding fragments specifically binding to MASP3, antibodies or antigen-binding fragments specifically binding to MASP2, or multi-specific antibodies specifically binding to MASP3 and MASP2 described herein, and b) an effective amount of at least one other agent, wherein the other agent enhances the effect (*e.g*., treatment effect, detecting effect) of the antibody or antigen-binding fragment specifically binding to MASP3, antibody or antigen-binding fragment specifically binding to MASP2, or multi-specific antibody specifically binding to MASP3 and MASP2. In some embodiments, the kit comprises a) a composition comprising any one of the antibodies or antigen-binding fragments specifically binding to MASP3, antibodies or antigen-binding fragments specifically binding to MASP2, or multi-specific antibodies specifically binding to MASP3 and MASP2 described herein, and b) instructions for administering the antibody or antigen-binding fragment specifically binding to MASP3, antibody or antigen-binding fragment specifically binding to MASP2, or multi-specific antibody specifically binding to MASP3 and MASP2 composition to an individual for treating a disease or condition in an individual in need. In some embodiments, the kit comprises a) a composition comprising any one of the antibodies or antigen-binding fragments specifically binding to MASP3, antibodies or antigen-binding fragments specifically binding to MASP2, or multi-specific antibodies specifically binding to MASP3 and MASP2 described herein, b) an effective amount of at least one other agent, wherein the other agent enhances the effect (*e.g*., treatment effect, detecting effect) of the antibody or antigen-binding fragment specifically binding to MASP3, antibody or antigen-binding fragment specifically binding to MASP2, or multi-specific antibody specifically binding to MASP3 and MASP2, and c) instructions for administering the antibody or antigen-binding fragment specifically binding to MASP3, antibody or antigen-binding fragment specifically binding to MASP2, or multi-specific antibody specifically binding to MASP3 and MASP2 composition and the other agent(s) to an individual for useful for treating a disease or condition in an individual in need. The antibody or antigen-binding fragment specifically binding to MASP3, antibody or antigen-binding fragment specifically binding to MASP2, or multi-specific antibody specifically binding to MASP3 and MASP2 and the other agent(s) can be present in separate containers or in a single container. For example, the kit may comprise one distinct composition or two or more compositions wherein one composition comprises an antibody or antigen-binding fragment specifically binding to MASP3, antibody or antigen-binding fragment specifically binding to MASP2, or multi-specific antibody specifically binding to MASP3 and MASP2 and another composition comprises another agent.

In some embodiments, the kit comprises a nucleic acid (or set of nucleic acids) encoding an antibody or antigen-binding fragment specifically binding to MASP3, antibody or antigen-binding fragment specifically binding to MASP2, or multi-specific antibody specifically binding to MASP3 and MASP2 (such as a full-length antibody specifically binding to MASP3, full-length antibody specifically binding to MASP2, or full-length multi-specific antibody specifically binding to MASP3 and MASP2). In some embodiments, the kit comprises a) a nucleic acid (or set of nucleic acids) encoding an antibody or antigen-binding fragment specifically binding to MASP3, antibody or antigen-binding fragment specifically binding to MASP2, or multi-specific antibody specifically binding to MASP3 and MASP2, and b) a host cell for expressing the nucleic acid (or set of nucleic acids). In some embodiments, the kit comprises a) a nucleic acid (or set of nucleic acids) encoding an antibody or antigen-binding fragment specifically binding to MASP3, antibody or antigen-binding fragment specifically binding to MASP2, or multi-specific antibody specifically binding to MASP3 and MASP2 and b) instructions for i) expressing the antibody or antigen-binding fragment specifically binding to MASP3, antibody or antigen-binding fragment specifically binding to MASP2, or multi-specific antibody specifically binding to MASP3 and MASP2 in a host cell, ii) preparing a composition comprising the antibody or antigen-binding fragment specifically binding to MASP3, antibody or antigen-binding fragment specifically binding to MASP2, or multi-specific antibody specifically binding to MASP3 and MASP2, and iii) administering the composition comprising the antibody or antigen-binding fragment specifically binding to MASP3, antibody or antigen-binding fragment specifically binding to MASP2, or multi-specific antibody specifically binding to MASP3 and MASP2 to an individual for treating or preventing a disease or condition in an individual in need. In some embodiments, the kit comprises a) a nucleic acid (or set of nucleic acids) encoding an antibody or antigen-binding fragment specifically binding to MASP3, antibody or antigen-binding fragment specifically binding to MASP2, or multi-specific antibody specifically binding to MASP3 and MASP2, b) a host cell for expressing the nucleic acid (or set of nucleic acids), and c) instructions for i) expressing the antibody or antigen-binding fragment specifically binding to MASP3, antibody or antigen-binding fragment specifically binding to MASP2, or multi-specific antibody specifically binding to MASP3 and MASP2 in the host cell, ii) preparing a composition comprising the antibody or antigen-binding fragment specifically binding to MASP3, antibody or antigen-binding fragment specifically binding to MASP2, or multi-specific antibody specifically binding to MASP3 and MASP2 and iii) administering the composition comprising the antibody or antigen-binding fragment specifically binding to MASP3, antibody or antigen-binding fragment specifically binding to MASP2, or multi-specific antibody specifically binding to MASP3 and MASP2 to an individual for treating or preventing a disease or condition in an individual.

The kits of the application are in suitable packaging. Suitable packaging includes, but is not limited to, vials, bottles, jars, flexible packaging (*e.g*., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the antibody or antigen-binding fragment specifically binding to MASP3, antibody or antigen-binding fragment specifically binding to MASP2, or multi-specific antibody specifically binding to MASP3 and MASP2 compositions generally include information as to dosage, dosing schedule, and route of administration for the intended treatment. The containers may be unit doses, bulk packages (*e.g*., multi-dose packages) or sub-unit doses. For example, kits may be provided that contain sufficient dosages of an antibody or antigen-binding fragment specifically binding to MASP3, antibody or antigen-binding fragment specifically binding to MASP2, or multi-specific antibody specifically binding to MASP3 and MASP2 (such as a full-length antibody specifically binding to MASP3, full-length antibody specifically binding to MASP2, or full-length multi-specific antibody specifically binding to MASP3 and MASP2) as disclosed herein to provide effective treatment of an individual for an extended period, such as any of a week, 8 days, 9 days, 10 days, 11 days, 12 days, 13 days, 2 weeks, 3 weeks, 4 weeks, 6 weeks, 8 weeks, 3 months, 4 months, 5 months, 7 months, 8 months, 9 months, or more. Kits may also include multiple unit doses of the antibody or antigen-binding fragment specifically binding to MASP3, antibody or antigen-binding fragment specifically binding to MASP2, or multi-specific antibody specifically binding to MASP3 and MASP2 and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this application. The application will now be described in greater detail by reference to the following non-limiting examples. The following examples further illustrate the application but, of course, should not be construed as in any way limiting its scope.

### EXAMPLES

In the following disclosed embodiments, the following abbreviations are applicable: MASP2 (Mannan Binding Lectin Associated Serine Protease-2); MASP3 (Mannan Binding Lectin Associated Serine Protease-3); MBL (Mannan Binding Lectin).

### Example 1: Generation of recombinant protein

### 1.1 Generation of recombinant full-length MASP3 protein and derived polypeptide

The cDNA encoding human MASP3 (GenBank ID: 5648), monkey MASP3 (GenBank ID: 708206) or mouse MASP3 (GenBank ID: 17174) was subcloned into eukaryotic expression vector respectively. The cDNA encoding CCP2-SP domain or SP domain of human MASP3 was subcloned into eukaryotic expression vector respectively. His-tag and/or Avi-tag and/or other conventionally used tags were added to the terminal of cDNA described above, and plasmids containing the encoding sequences of human, monkey or mouse MASP3 proteins and derived polypeptides thereof were constructed. The plasmids were transfected into 293F cells to express and produce full-length MASP3 fusion proteins and MASP3 derived polypeptides, such as human MASP3-His, human MASP3-Avi-His, monkey MASP3-His, mouse MASP3-His, human MASP3 CCP2-SP-His, human MASP3 SP-His, wherein "His" stands for His tag, "Avi" stands for avidin tag.

Recombinant proteins with His-tag were purified using a nickel column (Ni) according to manufacturer's protocol. In addition, according to the manufacturer's protocol of biotinylated ligase B0101A (GeneCopoeia), biotinylated labeled MASP3-Avi-His recombinant protein was successfully prepared and named as human MASP3-Bavih.

### 1.2 Generation of recombinant human pre-Factor D

The cDNA encoding human pre-Factor D (GenBank ID: 1675) was subcloned into prokaryotic expression vector. His-tag and/or other conventionally used tags were added to the terminal of cDNA described above, and plasmids containing the encoding sequences of human pre-Factor D were constructed. The plasmids were transfected into *E. coli* to express and produce pre-Factor D (pFD) fusion proteins, such as pFD-His, wherein "His" stands for His-tag. Recombinant proteins with His-tag were purified using a nickel column (Ni) according to manufacturer's protocol.

### Example 2: Selection of anti-MASP3 single chain antibodies (scFv)

### 2.1 Construction of scFv Antibody phage display library

6-8 week MASP1/3 gene knockout mice (Purchased from Shanghai Model Organisms Center, Inc., NM-KO-200353) were immunized with human MASP3 CCP2-SP-His or human MASP3 SP-His antigen and equal (v/v) adjuvant. Serum was collected from each immunized mouse and the titer of antigen-specific IgG in the serum was detected by ELISA. After several rounds of immunization, spleen was used to establish a phage library. Briefly, RNAs were extracted from spleen of the immunized mice and then cDNAs was obtained through reverse transcription. V_{H} and V_{K} fragments were amplified using V_{H}- and V_{K}-specific primers. Upon gel extraction and purification, scFvs were generated by linking V_{H} and V_{K}, and were cloned into the phage display plasmid. Subsequently, the plasmid was electroporated into E. coli *TG1* competent cells, and the scFv antibody phage display library was generated by infecting *TG1* with phage.

### 2.2 Selection of anti-MASP3 single chain antibodies (scFv)

After several rounds of panning, phages which specifically bound to human MASP3 were isolated from the phage display library by two selection strategy.

Solid phase screening: the enzyme-linked immunosorbent assay (ELISA) plate was coated with the human MASP3-His or MASP3 SP-His antigen prepared in Example 1, and the constructed phage display library was added to it, with the input amount of 4×10¹¹ pfu/well. The plate was incubated at 37°C for 2 hours. Positive phages bound to the antigen were screened using anti-M13 antibody (Sino Biological, 11973-MM05T-H), and eluted with alkaline elution buffer. After multiple rounds of screening, positive phages that bind to antigens were enriched. By further ELISA detection, a series of positive scFv antibodies were obtained by analyzing individual positive clones.

Liquid phase screening: The human MASP3-Bavih antigen (10µg) prepared in Example 1 was mixed with the constructed phage display library (2×10¹² pfu) and incubate at 37 °C for 1 hour. The incubated mixture was added to the blocked magnetic beads coated with streptavidin, screen for positive bacteriophages that bind to the antigen, and elute the positive phages with alkaline eluent. After multiple rounds of screening, enrich the positive phages that bind to the antigen. By further ELISA detection, a series of positive scFv antibodies were obtained by analyzing individual positive clones.

### Example 3: Generation and characterization of full-length anti-MASP3 chimeric antibodies

### 3.1 Generation of full-length anti-MASP3 chimeric antibodies

The positive scFv antibodies enriched after multiple rounds of screening were sequenced, V_{H} and V_{L} were amplified from the phase display vectors and introduced into eukaryotic expression vectors pTTa1-L (containing kappa constant domain) and pTTa1-H4 (containing IgG4 heavy chain constant domain), respectively. Plasmids expressing the light chain and heavy chain were co-transfected into 293F cells and cultured at 37°C, 8% CO₂ and 120rpm for 5 days. The culture media was purified using Protein A affinity chromatography. Briefly, Protein A column was first equilibrated with 6 × column volume of 50mM PBS buffer (containing 0.15M NaCl, pH7.2) at a flow rate of 150cm/h. The supernatant of the culture media (pH was adjusted to 7.2) was passed through the column at 150cm/h. Upon further equilibration, 50mM sodium citrate (pH3.5) was used to wash the column and the elution was collected, the pH value was adjusted to neutral. The IgG solution was replaced with the PBS buffer by using the desalination column, and the IgG concentration was measured after ultrafiltration concentration.

### 3.2 The binding affinity of anti-MASP3 chimeric antibody to MASP3 antigen

The selected anti-MASP3 antibody were subjected to binding assays with human, monkey, mouse MAPS3 antigen. This assay was used to identify the binding activity of anti-MASP3 antibody with human, monkey, mouse MASP3 antigen. Briefly, the human MASP3-His, monkey MASP3-His, or mouse MASP3-His antigen was coated on 96-well plates with 0. 1µg/well followed by incubation at 4°C overnight. The 96-well plates were blocked with 5% milk for 1h at 37°C and washed with PBST for 6 times. Firstly, each antibody sample was diluted to 10 µg/ml, and then serially diluted at a ratio of 1:8. 100µL of serially diluted antibodies were added to 96-well plates with 100µl each well and incubated for 1h at 37°C and washed with PBST for 6 times. 100µL of the goat anti-human IgG-HRP (1:10000 dilution, Sigma, A8667) was added to each well and incubated for 1h at 37°C. After washing with PBST for 6 times, TMB was added with 100 µL/well and incubated for 10-20 minutes at 37°C. 2M H₂SO₄ was used to stop the reaction. OD450 was measured, and the binding curves were generated by PRISM. EC₅₀ values was calculated, and the affinity of anti-MASP3 antibody to human, monkey, mouse MASP3 antigen was analyzed.

As shown in Table 11, both anti-MASP3 antibodies M3-K1 and M3-K2 can bind to human, monkey, or mouse MASP3, and the binding activity is significantly better than the control antibody OMS906.

**Table 11: The cross binding ability of anti-MASP3 antibody to MASP3 antigen**

| Antibody Name | EC₅₀(nM) | | |
|---|---|---|---|
| | human | monkey | mouse |
| M3-K1 | 0.08 | 0.79 | 0.23 |
| M3-K2 | 0.04 | 0.24 | 0.08 |
| OMS906 | 0.98 | 2.97 | 5.33 |

### 3.3 Determination of the inhibitory effect of anti-MASP3 chimeric antibodies on MASP3 enzymatic activity:

MASP3 is a serine protease with the ability to hydrolyze specific amino acids, which can cleave the amide bond of the fluorescent tripeptide substrate Boc-Val-Pro-Arg-AMC under Ca²⁺ conditions, and releasing AMC (7-amino-4-methylcoumarin, a highly fluorescent group) through hydrolysis. Under the conditions of excitation light 380nm/emission light 460nm, the fluorescence values at different time periods were detected to reflect the inhibitory ability of anti-MASP3 chimeric antibodies on MASP3 enzymatic activity.

The anti-MASP3 antibody was diluted with reaction buffer (containing 25mM Tris-base, 10mM CaCl₂, and 0.15M NaCl) to 2000µg/ml, followed by gradient dilution at a ratio of 1:5 for future use. Different concentrations of anti-MASP3 antibodies were incubated with human MASP3-His (final concentration of 0.25µM) at room temperature for 15 minutes. A fluorescent tripeptide substrate Boc-Val-Pro-Arg-AMC (R&D system, ES011) was added with a final concentration of 0.1 mM to the above system, mix well, and placed in an enzyme-linked immunosorbent assay reader at 37 °C. The fluorescence intensity (RFU) was measured at excitation light 380nm/emission light 460nm every 5 minutes for a total of 60 minutes. The inhibition curves were generated using PRISM and the IC₅₀ values were calculated.

As shown in Table 12, anti-MASP3 antibodies M3-K1 and M3-K2 can inhibit the enzymatic activity of MASP3, and their inhibitory activity is significantly better than the control antibody OMS906.

**Table 12: Inhibitory effect of anti-MASP3 chimeric antibodies on MASP3 enzyme activity**

| Antibody Name | IC₅₀ (nM) |
|---|---|
| M3-K1 | 33.98 |
| M3-K2 | 38.61 |
| OMS906 | 199.75 |

### 3.4 Determination of the inhibitory effect of anti-MASP3 chimeric antibodies on complement alternative pathway:

In the complement alternative pathway, the serine protease activity of MASP3 can activate the pre complement Factor D (pFD) in blood into active complement Factor D (FD). FD further cleaves complement Factor B (FB) into Bb, which forms an alternative pathway C3 convertase (C3bBb) with C3b formed by C3 hydrolysis. C3 convertase is one of the important central regulatory molecules in the complement system, which cleaves complement molecule C3 to form two highly pro-inflammatory molecules: the anaphylatoxin C3a and the regulatory molecule C3b. C3b further binds with the alternative pathway C3 convertase (C3bBb) to form C5 convertase (C3bBb3b), C5 convertase is another important central regulatory molecule in the complement system, the hydrolytic enzyme activity of which cleaves C5, forming anaphylatoxin C5a and C5b. Finally, C5b and C6-9 form the biological effector molecule of complement - the membrane attack complex MAC (C5b-9).

Un-sensitized rabbit or guinea pig red blood cells can activate the complement alternative pathway in human serum, which produces a series of protein cascade reactions after complement activation, ultimately leading to rabbit cell lysis. After adding anti-MASP3 antibody, the hemolytic reaction mediated by alternative pathway can be inhibited. The absorbance value at 415nm (OD415) was detected using the enzyme-linked immunosorbent assay (ELISA) reader, and the hemolytic activity of anti-MASP3 antibody was calculated based on this value. In addition, adding ethylene glycol diamine tetraacetic acid (EGTA) to the reaction system can chelate with Ca²⁺in plasma, but has weak binding ability with Mg²⁺. Therefore, adding EGTA can block the classical pathway and eliminate its influence.

Firstly, the anti-MASP3 antibody was diluted to 900µg/ml using HBS-Mg²⁺-EGTA buffer (containing 5mM MgCl₂·6H₂O and 10mM EGTA), followed by gradient dilution of the antibody in a ratio of 1:8. At room temperature, different concentrations of anti-MASP3 antibodies were incubated with Factor D depleted human plasma for 15 minutes, and the pre Factor D prepared in Example 1 (final concentration of 2.5µg/ml) was added. The fresh rabbit or guinea pig red blood cells was washed three times with HBSS buffer (containing 0.1% gelatin), and diluted with HBS-Mg²⁺-EGTA buffer. The diluted red blood cells was added to the above system. Incubated at 37 °C for 45 minutes, mixed well again, and continued incubating for 20 minutes. After incubation, 80µl of pre cooled termination buffer (containing 10mM EDTA, 1×HBSS buffer, pH 7.4) was added to each reaction well, centrifuged at 4 °C, 1200g for 5 minutes. 100µl of supernatant was added to the 96-well plate and OD415 was measured. The inhibition curves were generated using PRISM and the IC50 values were calculated.

As shown in Table 13, anti-MASP3 antibodies M3-K1 and M3-K2 can inhibit the hemolytic reaction mediated by complement alternative pathway activation, and their inhibitory activity is significantly better than the control antibody OMS906.

**Table 13: Inhibitory activity of anti-MASP3 chimeric antibodies on hemolysis**

| Antibody Name | Inhibit Hemolysis IC₅₀ (nM) |
|---|---|
| M3-K1 | 2.05 |
| M3-K2 | 1.31 |
| OMS906 | 13.02 |

### Example 4: Humanization of Anti-MASP3 Antibodies

Based on the typical structure of light and heavy chain variable domain CDR of the mouse antibody M3-K1 or M3-K2 respectively, light and heavy chain variable domain sequences were aligned with the sequences in antibody Germline database to obtain human germline templates of high homology. The CDR regions of the mouse antibody were grafted into selected human germline templates to produce humanized variable regions, which were then recombined with corresponding human IgG constant regions (preferably IgG4 heavy chain and κ light chain). The embedded residues, the residues that interact directly with the CDR regions, and the residues that have an important effect on the conformation of V_{L} and V_{H} were then reverse-mutated based on the three-dimensional structure of the mouse antibody, meanwhile, and different light chains and heavy chains can be designed and combined to obtain a series of humanized molecules.

### Example 5: Characterization of the humanized Antibodies

Biological activity (*e.g.,* inhibition hemolysis assay mediated by complement alternative pathway, inhibition MASP3 enzymatic activity assay) of the M3-K1 or M3-K2 humanized antibodies were tested according to the corresponding protocol as described in Example 3, and their binding ability to MASP antigens were detected.

### 5.1 Determination of the inhibitory effect of anti-MASP3 humanized antibodies on MASP3 enzymatic activity:

Using the corresponding protocol described in Example 3.3, partial of the M3-K1 or M3-K2 humanized antibodies (reformatted as human IgG4) were used as examples to detect their inhibitory activity in fluorescent tripeptide cleavage experiment.

As shown in Table 14, the exemplary humanized antibodies M3-K1-1~M3-K1-6 can inhibit the enzymatic activity of MASP3, and the inhibitory activity is equivalent to that of M3-K1. The exemplary humanized antibodies M3-K2-6~ M3-K2-11 can also inhibit the enzymatic activity of MASP3, and the inhibitory activity is equivalent to that of M3-K2.

**Table 14: Inhibitory effect of anti-MASP3 chimeric antibodies on MASP3 enzyme activity**

| **Antibody Name** | **IC₅₀ (nM)** | **Antibody Name** | **IC₅₀(nM)** |
|---|---|---|---|
| M3-K1 | 32.07 | M3-K2 | 21.35 |
| M3-K1-1 | 31.49 | M3-K2-6 | 29.52 |
| M3-K1-2 | 31.24 | M3-K2-7 | 25.24 |
| M3-K1-3 | 24.17 | M3-K2-8 | 21.93 |
| M3-K1-4 | 29.93 | M3-K2-9 | 33.73 |
| M3-K1-5 | 27.88 | M3-K2-10 | 36.97 |
| M3-K1-6 | 28.2 | M3-K2-11 | 26.42 |

### 5.2 Determination of the inhibitory effect of anti-MASP3 humanized antibodies on complement alternative pathway:

Using the corresponding protocol described in Example 3.4, partial humanized antibodies (reformatted as human IgG4) of the M3-K1 or M3-K2 were used as examples to detect their inhibitory activity in the hemolysis assay mediated by complement alternative pathway.

As shown in FIG. 1A-1C, the exemplary humanized antibodies M3-K1-1~M3-K1-6, M3-K2-2, M3-K2-3, M3-K2-5, M3-K2-6~M3-K2-11 can inhibit the hemolytic activity mediated by complement alternative pathway activation, and the inhibitory activity is equivalent to that of the parent antibody molecules before humanization modification. For example, the IC₅₀ (nM) of M3-K2-2, M3-K2-3, and M3-K2-5 inhibitory activity is about 1.2-1.5 nM, which is equivalent to that of M3-K2.

### 5.3 Determination of the binding affinity of anti-MASP3 humanized antibodies

Partial of the humanized antibodies were selected as examples for affinity determination. Biacore 8K (GE) was used to characterized the binding affinity of the anti-MASP3 humanized antibody to human MASP3, monkey MASP3, and mouse MASP3. The anti-MASP3 antibody was immobilized on the sensor chip CM5, the affinity of different concentrations of human MASP3-His, monkey MASP3-His, or mouse MASP3-His antigen to the anti-MASP3 antibody was detected, the binding and dissociation rates of the antibody was measured by using SPR technology, and the binding affinity was determined.

As shown in Table 15, the exemplary humanized antibodies M3-K2-6, M3-K2-8, and M3-K2-11 can bind to human, monkey, and mouse MASP3, and the binding affinity can be 10⁻⁹M~10⁻¹⁰M.

**Table 15: The binding affinity of anti-MASP3 humanized antibodies**

| **Antigen** | **Antibody Name** | **Kₒₙ (1/Ms)** | **K_{off} (1/s)** | **KD (M)** |
|---|---|---|---|---|
| **Human MASP3** | M3-K2-6 | 1.726E+5 | 2.499E-4 | 1.448E-9 |
| | M3-K2-8 | 1.819E+5 | 1.892E-4 | 1.040E-9 |
| | M3-K2-11 | 1.630E+5 | 2.178E-4 | 1.336E-9 |
| **Monkey MASP3** | M3-K2-6 | 1.652E+5 | 1.465E-4 | 8.867E-10 |
| | M3-K2-8 | 1.779E+5 | 1.369E-4 | 7.696E-10 |
| | M3-K2-11 | 1.673E+5 | 1.438E-4 | 8.599E-10 |
| **Mouse MASP3** | M3-K2-6 | 1.679E+5 | 1.488E-4 | 8.866E-10 |
| | M3-K2-8 | 1.778E+5 | 1.369E-4 | 7.698E-10 |
| | M3-K2-11 | 1.684E+5 | 1.448E-4 | 8.599E-10 |

### Example 6: Preparation of MASP2 antigen and Selection of anti-MASP2 antibodies

### 6.1 Preparation of recombinant full-length human MASP2 protein and human MASP2 derived polypeptides without self-cleavage activity

To obtain high-yield and stable MASP2 antigen, the cDNA encoding human MASP2 protein (synthesized by Shanghai Generay Biotech Co., Ltd) was first constructed into an expression vector through subcloning. By using PCR site directed mutation, the arginine residue at position 444 in the protease domain (SP) was replaced with glutamine residue to construct a full-length MASP2 mutant without self-cleavage activity, which was named MASP2 (R444Q). On the basis of the mutant, cDNA of the CCP1-CCP2-SP domain of human MASP2 was subcloned to generate human MASP2 derived polypeptides CCP1-CCP2-SP (R444Q). His-tag or other conventionally used tags were added to the C-terminus of cDNA encoding human MASP2 (R444Q) protein and human MASP2 derived polypeptides CCP1-CCP2-SP (R444Q), and the tag plasmids encoding human MASP2 (R444Q) and human MASP2 derived polypeptides CCP1-CCP2-SP (R444Q) were constructed. The above tag plasmid cells were expressed and purified according to the His tag instructions to obtain the corresponding fusion protein. In addition, the monkey or mouse MASP2 fusion proteins and derived polypeptides thereof were also obtained by the method above, respectively.

### 6.2 Acquisition of anti-MASP2 antibodies:

By screening the constructed scFv phage library and identifying the activity of antibody molecules, the anti-human MASP2 chimeric antibody M2mc51 was obtained. Subsequently, the antibody molecule was humanized to obtain a series of humanized antibody molecules. Finally, through a combination of appropriately designed biochemical and biological experiments (such as ELISA binding assay and complement molecule deposition assay), the anti-MASP2 antibody humM2mc51-1 was screened. Based on the antibody, some potential deamidation sites in the V_{L} CDR3 were modified to improve the stability of the antibody, thereby obtaining a series of mutated antibodies. The sequences of the anti-MASP2 antibodies mentioned above are shown in Tables 4 and 5.

### 6.3 Determination of the affinity of mutated anti-MASP2 antibodies

The binding assay was performed between the mutated anti-MASP2 antibodies (reformatted as human IgG4) and MASP2. In short, human MASP2 (R444Q) - His antigen was dissolved in PBS solution and coated in a 96-well plate at a concentration of 0.1µg/well, and incubatd overnight at 4°C. Blocked with 5% milk at 37°C for 1 hour, then washed 6 times with TBST solution. Firstly, each antibody sample was diluted to 2µg/ml, followed by gradient dilution at a ratio of 1:8. The gradient diluted samples were added into 96-well plates, with 100µl per well, and incubated at 37°C for 1 hour. Then washed 6 times with TBST solution. 100µl of secondary antibody (goat anti human IgG HRP (1:10000)) was added to each well and incubated at 37°C for 1 hour. Washed 6 times with TBST solution. 100µl of TMB was added to each well, incubated at 37°C for 10-20 minutes, and the reaction was terminated with 2M H₂SO₄. The absorbance value was read at 450nm using the enzyme-linked immunosorbent assay (ELISA) reader. The binding curves were generated using PRISM, and the EC50 values were calculated, and the affinity of mutated anti-MASP2 antibodies to antigens was analyzed based on this value.

As shown in Table 16, compared with the parent antibody humM2mc51-1, the binding activity between the mutated humM2mc51-1-S93A, humM2mc51-1-S93E, humM2mc51-1-N92Q, humM2mc51-1-N92S, humM2mc51-1-ES93DA and MASP2 is no significant change, and still with high binding activity.

**Table 16: The binding ability of anti-MASP2 antibodies to human MASP2**

| Antibody Name | EC₅₀ Value(nM) |
|---|---|
| humM2mc51-1 | 0.086 |
| humM2mc51-1-N92Q | 0.072 |
| humM2mc51-1-N92S | 0.079 |
| humM2mc51-1-S93E | 0.085 |
| humM2mc51-1-S93A | 0.089 |
| humM2mc51-1-ES93DA | 0.082 |

### 6.4 Inhibitory activity of the mutated anti-MASP2 antibodies on complement lectin pathway

Mannan is a known lectin pathway activator, which can bind to MBL and activate the complement lectin pathway. After activation of the complement lectin pathway, the proteolytic activity of MASP2 cleaves its substrate complement molecule C4 into C4a and C4b, and C2 into C2a and C2b, where C4b and C2a further form C3 convertase (C4b2a). C3 convertase is one of the important central regulatory molecules in the complement system, which cleaves complement molecule C3 into two highly pro-inflammatory molecules: the anaphylatoxin C3a and the regulatory molecule C3b. C3b further binds with C4b2a to form C5 convertase (C4b2a3b). C5 convertase is another important central regulatory molecule in the complement system, whose hydrolytic activity cleaves C5 into the anaphylatoxin C5a and C5b, and finally C5b forms the membrane attack complex MAC (C5b-9) with C6-9. C3b, C4b, and MAC (C5b-9) all contain highly reactive thioester groups, which can form covalent bonds with hydroxyl or amino groups fixed to the bottom of plastic pores through ester or amide bonds, allowing C3b, C4b, and MAC (C5b-9) to deposit at the bottom of plastic enzyme-linked immunosorbent assay plates. Therefore, ELISA method can be used to detect the deposition of these molecules.

Based on the principles described above, ELISA was used to detect the deposition level of C4b, reflecting the activity of anti-MASP2 antibodies in inhibiting MASP2 cleavage of its direct substrate C4. Firstly, the mannan (Sigma, M7504) was diluted to 100µg/ml using alkaline coating buffer (15mM Na₂CO₃, 35mM NaHCO₃, pH 9.8). 100µl of mannan was then coated in each well and incubated overnight at 4 °C. Washed 3 times with PBST buffer next day. 100µl of blocking solution (1% human serum albumin HSA) was added to each well and incubated at 37 °C for 1 hour. Washed 3 times with PBST buffer. The normal human plasma was diluted to 2% using Ca²⁺ and Mg²⁺ buffer solution (10mM Tris HCl (pH 7.4), 150mM NaCl, 0.5mM MgCl₂, 2mM CaCl₂, 0.1% gelatin, 0.05% Tween-20, pH 7.4). The anti-MASP2 antibody (reformatted as human IgG4) was diluted with an initial concentration of 66.67µg/ml in Ca²⁺ and Mg²⁺ buffer at a ratio of 1:10. The diluted plasma and gradient diluted anti-MASP2 antibody were mixed in equal volumes (so that the final concentration of human plasma in the reaction system is 1% and the maximum final concentration of anti-MASP2 antibody is 33.335µg/ml), and incubated at 37 °C for 2 hours. The reaction well was washed with PBST buffer to terminate the reaction. The chicken anti-human C4b antibody (Abcam, ab48582) was added as capture antibody to the sample wells described above and sheep anti-chicken antibody (Southern Biotech, 6100-05) was added as detection antibody to detect C4b deposition in human plasma. The enzyme-linked immunosorbent assay (ELISA) reader was used to measure the absorbance at 450nm, and the binding curves were generated by Graphpad Prism, and the IC50 value was calculated.

As shown in Table 17, the mutated anti-MASP2 antibody humM2mc51-1-S93A, humM2mc51-1-S93E, humM2mc51-1-N92Q, humM2mc51-1-N92S, humM2mc51-1-ES93DA can effectively inhibit the lectin pathway, and its inhibitory activity has no significant change compared to the parent antibody humM2mc51-1.

**Table17: Inhibitory activity of anti-MASP2 antibody on complement C4b deposition**

| Antibody Name | IC₅₀ (nM) |
|---|---|
| humM2mc51-1 | 0.055 |
| humM2mc51-1-N92Q | 0.11 |
| humM2mc51-1-N92S | 0.09 |
| humM2mc51-1-S93E | 0.05 |
| humM2mc51-1-S93A | 0.037 |
| humM2mc51-1-ES93DA | 0.045 |

In addition, the changes in charge heterogeneity and aggregates of the mutated anti-MASP2 antibody after pressurization were evaluated using capillary isoelectric focusing and molecular exclusion chromatography methods. The results showed that the aggregation content of the exemplary anti-MASP2 antibodies humM2mc51-1-S93A, humM2mc51-1-N92Q, and humM2mc51-1-ES93DA had all significantly decreased; and the charge heterogeneity was also improved to a certain extent after pressurization (results not shown).

### Example 7: Preparation of multi-specific antibodies specifically binding to MASP3 and MASP2

In the following sequence design: V_{H} And V_{L} represent the heavy chain variable domain and the light chain variable domain of an antibody, respectively; C_{H} represents the antibody heavy chain constant domain, including C_{H}1, C_{H}2 and C_{H}3 domain; scFv is an antibody formed by linking V_{H} and V_{L} of the antibody through a peptide linker (Linker); IgG1 Fc represents the Fc region of an IgG1 subclass antibody, comprising C_{H}2 and C_{H}3 domain; C_{L} represents the light chain constant domain.

### 7.1 Construction of Hetero H, CrossMab format bispecific antibodies:

Hetero H, CrossMab format bispecific antibody, *i.e.,the* knob-in-hole structure (KIH) is designed in the Fc region, and introduces two Cys residue mutations that form stable disulfide bridges (S354C on the "knob" side and Y349C on the "hole" side). KIH method, *i.e.,* substitute threonine (T) at position 366 in antibody C_{H}3 domain with tryptophan (W) to form "knobs" structure, and substitute threonine (T) at position 366 with serine (S), leucine (L) at position 368 with alanine (A), tyrosine (Y) at position 407 with valine (V) in the C_{H}3 domain to form "holes" structure, which promotes dimerization of heterologous heavy chains by the decrease of steric hindrance effect after mutation and the formation of covalent disulfide bonds in the hinge region, wherein the numbering is in accordance with EU index of Kabat. Simultaneously, the CrossMab technology is also applied to ensure proper pairing between the light chains and heavy chains of antibody. CrossMAb technology is based on the exchange of domains in one Fab arm of bispecific IgG antibodies, either as an exchange of intact Fab domains (CrossMAb Fab), or as an exchange of only variable domains in the Fab (CrossMAb V_{H}-V_{L}) or constant region only (CrossMAb C_{H}1-C_{L}). Hetero H, CrossMab format bispecific antibodies are described in literature Klein C, et al. The use of CrossMAb technology for the generation of bi- and multispecific antibodies. MAbs. 2016 Aug-Sep;8(6):1010-20. In addition, it can also introduce LALA mutations into the Fc structure to further enhance the stability. The present application uses the CrossMab C_{H}1-C_{L} format for specific description, and the schematic diagram of the format was shown in FIG. 2C. Table 18 showed the composition design of Hetero H, CrossMab format bispecific antibodies used in the examples, and the amino acid sequences of the heavy chains and light chains thereof were shown in Table 8. We commissioned Kingsray Biotechnology to synthesize the genes encoding the light chain (L chain) and heavy chain (H chain) of bispecific antibodies, respectively, and optimized the coding genes to make them suitable for expression in 293F cells.

### 7.2 Expression and purification of bispecific antibodies

After restriction enzyme digestion, the light and heavy chain genes encoding bispecific antibodies were subcloned into the pTT α1 vector, respectively. Recombinant plasmids expressing light and heavy chains of antibodies were extracted and co-transfected into 293F cells. Cultured at 37 °C and 5% CO₂ at 120 rpm for 7 days, and the culture medium was purified using a protein A affinity chromatography column. In short, protein A column was first equilibrated using a 50 mM PBS buffer (containing 0.15 M NaCl, pH 7.2) with 6 column volumes and a flow rate of 150 cm/h. Adjust the pH of the supernatant of the culture medium to 7.2 and load the sample at a flow rate of 150 cm/h. After the sample loading is completed, the column was equilibrated again and finally eluted with 50mM citrate sodium citrate buffer (pH 3.5) to collect the eluent. The eluent was concentrate using a 30 kDa ultrafiltration tube and the IgG protein was replaced with PBS solution. After using the endotoxin removal kit (SA031K, Changzhou Tiandirenhe Biotechnology Co., Ltd.) to remove endotoxins, the concentration of IgG protein and the content of endotoxins were measured separately.

**Table 18: Composition design of heavy chains and light chains of Hetero H, CrossMab format bispecific antibodies specifically binding to MASP3 and MASP2**

| **Bispecific antibody form** | **Antibody name** | **Heavy chain** | **Light chain** |
|---|---|---|---|
| | humM2mc51-1-S93A+M3-K2-5-CrossMab-IgG1 | humM2mc51-1-S93A V_{H}-C_{L}-IgG1 Fc-hole | humM2mc51-1-S93A V_{L}-C_{H}1 |
| | | M3-K2-5 V_{H}-IgG1 C_{H}-knob | M3-K2-5 V_{L}-C_{L} |
| Hetero H, CrossMab | humM2mc51-1-S93A+M3-K2-6-CrossMab-IgG1 | humM2mc51-1-S93A V_{H}-C_{L}-IgG1 Fc-hole | humM2mc51-1-S93A V_{L}-C_{H}1 |
| | | M3-K2-6 V_{H}-IgG1 C_{H}-knob | M3-K2-6 V_{L}-C_{L} |
| | humM2mc51-1-S93A+M3-K2-8-CrossMab-IgG1 | humM2mc51-1-S93A V_{H}-C_{L}-IgG1 Fc-hole | humM2mc51-1-S93A V_{L}-C_{H}1 |
| | | M3-K2-8 V_{H}-IgG1 C_{H}-knob | M3-K2-8 V_{L}-C_{L} |
| | humM2mc51-1-S93A+M3-K2-11-CrossMab-IgG1 | humM2mc51-1-S93A V_{H}-C_{L}-IgG1 Fc-hole | humM2mc51-1-S93A V_{L}-C_{H}1 |
| | | M3-K2-11 V_{H}-IgG1 C_{H}-knob | M3-K2-11 V_{L}-C_{L} |

### Example 8: Characterization of the activity of the bispecific antibodies specifically binding to MASP3 and MASP2

### 8.1 Binding affinity of the bispecific antibodies to MASP3 or MASP2

Biacore 8K (GE) was used to characterize the affinity of bispecific antibodies to human, monkey, and mouse MASP2 and MASP3. Among them, bispecific antibodies such as humM2mc51-S93A+M3-K2-6-CrossMab IgG1, humM2mc51-S93A+M3-K2-8CrossMab IgG1, and humM2mc51-S93A+M3-K2-11-CrossMab IgG1 were taken as examples. The antigens used in this example: human MASP2 CCP1-CCP2-SP (R444Q) - His, monkey MASP2 CCP1-CCP2-SP (R443Q) - His, mouse MASP2 CCP1-CCP2-SP (R443Q) - His were obtained by the method described in Example 6.1. Human MASP3 -His, monkey MASP3 -His, and mouse MASP3 -His were all prepared and obtained by the method described in Example 1.1.

Bispecific antibodies were immobilized on the CM5 sensor chip, and the affinity of antibodies with human, monkey, and mouse MASP2 and MASP3 at different concentrations was detected. The binding and dissociation rates of antibodies were measured using SPR technology, and the binding affinity was determined.

As shown in Table 19, the exemplary antibodies humM2mc51-1-S93A+M3-K2-6-CrossMab-IgG1, humM2mc51-1-S93A+M3-K2-8-CrossMab-IgG1, humM2mc51-1-S93A+M3-K2-11-CrossMab-IgG1 can bind well to human, monkey MASP2 and human, mouse, monkey MASP3 antigens, and its affinity constant is 10⁻⁹M-10⁻¹¹M.

**Table 19: Kon, Koff and Kd value of the bispecific antibodies**

| Antigen | Antibody Name | Kon (1/Ms) | Koff (1/s) | KD (M) |
|---|---|---|---|---|
| Human MASP2 | humM2mc51-1-S93A+M3-K2-6-CrossMab-IgG1 | 1.477E+6 | 3.022E-4 | 2.046E-10 |
| | humM2mc51-1-S93A+M3-K2-8-CrossMab-IgG1 | 1.321E+6 | 3.738E-4 | 2.830E-10 |
| | humM2mc51-1-S93A+M3-K2-11-CrossMab-IgG1 | 1.344E+6 | 3.274E-4 | 2.437E-10 |
| Human MASP3 | humM2mc51-1-S93A+M3-K2-6-CrossMab-IgG1 | 1.853E+5 | 1.136E-4 | 6.132E-10 |
| | humM2mc51-1-S93A+M3-K2-8-CrossMab-IgG1 | 1.583E+5 | 1.670E-4 | 1.055E-9 |
| | humM2mc51-1-S93A+M3-K2-11-CrossMab-IgG1 | 1.659E+5 | 2.985E-4 | 1.799E-9 |
| Monkey MASP2 | humM2mc51-1-S93A+M3-K2-6-CrossMab-IgG1 | 1.303E+5 | 8.828E-5 | 6.777E-10 |
| | humM2mc51-1-S93A+M3-K2-8-CrossMab-IgG1 | 1.162E+5 | 1.360E-4 | 1.171E-9 |
| | humM2mc51-1-S93A+M3-K2-11-CrossMab-IgG1 | 1.079E+5 | 1.275E-4 | 1.182E-9 |
| Monkey MASP3 | humM2mc51-1-S93A+M3-K2-6-CrossMab-IgG1 | 3.317E+6 | 3.436E-4 | 1.036E-10 |
| | humM2mc51-1-S93A+M3-K2-8-CrossMab-IgG1 | 3.352E+6 | 3.502E-4 | 1.045E-10 |
| | humM2mc51-1-S93A+M3-K2-11-CrossMab-IgG1 | 3.266E+6 | 3.090E-4 | 9.463E-11 |
| Mouse MASP2 | humM2mc51-1-S93A+M3-K2-6-CrossMab-IgG1 | / | / | 2.573E-7 |
| | humM2mc51-1-S93A+M3-K2-8-CrossMab-IgG1 | / | / | 5.336E-7 |
| | humM2mc51-1-S93A+M3-K2-11-CrossMab-IgG1 | / | / | 2.418E-7 |
| | humM2mc51-1-S93A+M3-K2-6-CrossMab-IgG1 | 1.953E+5 | 4.659E-5 | 2.385E-10 |
| Mouse MASP3 | humM2mc51-1-S93A+M3-K2-8-CrossMab-IgG1 | 1.662E+5 | 1.234E-4 | 7.425E-10 |
| | humM2mc51-1-S93A+M3-K2-11-CrossMab-IgG1 | 1.518E+5 | 1.363E-4 | 8.983E-10 |

### 8.2 Inhibition activity of bispecific antibodies in lectin pathway:

Similar to the experimental principle in Example 6.4, ELISA method was used to detect the deposition of complement molecule C4b in human plasma to reflect the biological activity of bispecific antibodies in the lectin pathway.

Mannan (Sigma, M7504) was diluted to 100µg/ml using alkaline coating buffer (15mM Na₂CO₃, 35mM NaHCO₃, pH 9.8), 100µl of the mixture was coated in each well, and incubated overnight at 4 °C. The next day, wash three times with PBST buffer. 100µl of blocking solution (1% human serum albumin HSA) was added to each well and incubated at 37 °C for 1 hour. Wash 3 times with PBST buffer. Normal human plasma was diluted to 2% using Ca²⁺ and Mg²⁺ buffer solutions (10mM Tris HCl, 150mM NaCl, 0.5mM MgCl₂, 2mM CaCl₂, 0.1% gelatin, 0.05% Tween-20, pH 7.4). The bispecific antibody was diluted with an initial concentration of 100µg/ml in a 1:6 gradient using Ca²⁺ and Mg²⁺ buffer solutions. The diluted plasma and gradient diluted bispecific antibody were mixed in equal volumes (with a final concentration of 1% human plasma and a maximum final concentration of 50µg/ml bispecific antibody in the reaction system), and incubated at 37 °C for 2 hours. Wash the reaction well with PBST buffer to terminate the reaction. Chicken anti human C4b antibody (Abcam, ab48582) was added as the capture antibody and sheep anti chicken antibody (Southern Biotech, 6100-05) was added as the detection antibody to detect C4b deposition in human plasma. The absorbance at 450nm was measured using the enzyme-linked immunosorbent assay (ELISA) reader, and the binding curve was generated using Graphpad Prism to calculate the IC50 value.

As shown in Table 20, exemplary bispecific antibodies humM2mc51-1-S93A+M3-K2-5-CrossMab-IgG1, humM2mc51-1-S93A+M3-K2-6-CrossMab-IgG1, humM2mc51-1-S93A+M3-K2-8-CrossMab-IgG1 and humM2mc51-S93A+M3-K2-11-CrossMab IgG1 can inhibit C4b deposition in the lectin pathway, and their inhibitory activity is significantly better than that of the anti-MASP2 monoclonal antibody OMS721.

**Table 20: Inhibitory activity of bispecific antibodies on complement C4b deposition**

| Antibody Name | IC₅₀ (nM) |
|---|---|
| humM2mc51-1-S93A | 0.05 |
| humM2mc51-1-S93A+M3-K2-5-CrossMab-IgG1 | 0.16 |
| humM2mc51-1-S93A+M3-K2-6-CrossMab-IgG1 | 0.10 |
| humM2mc51-1-S93A+M3-K2-8-CrossMab-IgG1 | 0.19 |
| humM2mc51-1-S93A+M3-K2-11-CrossMab-IgG1 | 0.12 |
| OMS721 | 1.82 |

### 8.3 Inhibition activity of bispecific antibodies in alternative pathways:

Similar to the experimental principle in Example 3.4, hemolysis assay was used to detect the biological activity of bispecific antibodies in alternative pathways.

Firstly, the bispecific antibody was diluted to 300µg/ml using HBS-Mg²⁺-EGTA buffer (containing 5mM MgCl₂·6H₂O and 10mM EGTA), followed by gradient dilution of the antibody in a ratio of 1:8. At room temperature, different concentrations of bispecific antibodies were incubated with factor D depleted human plasma for 15 minutes, and then the pre factor D prepared in Example 1 (final concentration of 2.5µg/ml) was added. Fresh rabbit or guinea pig red blood cells were washed three times with HBSS buffer (containing 0.1% gelatin), and the washed red blood cells were diluted with HBS-Mg²⁺-EGTA buffer. Diluted red blood cells were added to the above system, and incubated at 37 °C for 45 minutes. Mix well again, and continue incubating for 20 minutes. After incubation, 80µl of pre cooled termination buffer (containing 10 mM EDTA, 1×HBSS buffer, pH 7.4) was added to each reaction well, centrifuge at 4°C, 1200g for 5 minutes. Take 100µl of supernatant into a 96-well flat plate and measure OD415. The inhibition curves was generated using PRISM and IC50 values was calculated.

As shown in Table 21, exemplary bispecific antibodies humM2mc51-1-S93A+M3-K2-5-CrossMab-IgG1, humM2mc51-1-S93A+M3-K2-6-CrossMab-IgG1, humM2mc51-1-S93A+M3-K2-8-CrossMab-IgG1 and humM2mc51-S93A+M3-K2-11-CrossMab IgG1 can inhibit hemolytic reactions mediated by alternative pathways, and their inhibitory activity is superior to that of anti-MASP3 monoclonal antibody OMS906.

**Table 21: Inhibitory activity of the bispecific antibodies on hemolysis**

| Antibody Name | Inhibit hemolysis IC₅₀ (nM) |
|---|---|
| M3-K2-5 | 3.41 |
| M3-K2-6 | 3.18 |
| M3-K2-8 | 2.11 |
| M3-K2-11 | 2.20 |
| humM2mc51-1-S93A+M3-K2-5-CrossMab-IgG1 | 6.21 |
| humM2mc51-1-S93A+M3-K2-6-CrossMab-IgG1 | 6.65 |
| humM2mc51-1-S93A+M3-K2-8-CrossMab-IgG1 | 4.72 |
| humM2mc51-1-S93A+M3-K2-11-CrossMab-IgG1 | 4.51 |
| OMS906 | 17.14 |

The above results indicate that the exemplary bispecific antibody humM2mc51-1-S93A+M3-K2-5-CrossMab-IgG1, humM2mc51-1-S93A+M3-K2-8-CrossMab-IgG1, humM2mc51-1-S93A+M3-K2-8-CrossMab-IgG1, and humM2mc51-S93A+M3-K2-11-CrossMab IgG1 can simultaneously inhibit the biological activity of MASP2 and MASP3, i.e., they have the ability to simultaneously inhibit both lectin and alternative pathways.

### Example 9: Characterization of in vitro activity of bispecific antibodies specifically binding to MASP3 and MASP2

Female cynomolgus monkeys were randomly divided into four groups: a blank control group (normal saline, NS), an anti-MASP2 monoclonal antibody group, an anti-MASP3 monoclonal antibody group, and an anti-MASP2-MASP3 bispecific antibody group. The monoclonal antibody group was given a dose of 5 mg/kg, and the anti-MASP2-MASP3 bispecific antibody group was given a dose of 10 mg/kg. Each group of animals received a single intravenous injection with a dosage of 2ml/kg and an injection rate of approximately 3-4ml/min. Plasma samples were collected for testing before administration (D0), on the first day of administration (D1), on the fourth day (D4), and on the seventh day (D7).

### 9.1 Inhibition activity of bispecific antibodies in lectin pathway:

Similar to the experimental principle in Example 6.4, ELISA method was used to detect the deposition of MAC in the plasma of cynomolgus monkeys to reflect the biological activity of bispecific antibodies in the lectin pathway. Firstly, mannan (Sigma, M7504) was diluted to 100µg/ml using alkaline coating buffer (15mM Na₂CO₃, 35mM NaHCO₃, pH 9.8), and 100µl was coated each well and incubated overnight at 4 °C. The next day, wash three times with PBST buffer. 100µl of blocking solution (1% human serum albumin HSA) was added to each well and incubated at 37 °C for 1 hour. Wash 3 times with PBST buffer. Monkey plasma collected at different time points was diluted with Ca²⁺ and Mg²⁺ buffer solutions (10mM Tris HCl, 150mM NaCl, 0.5mM MgCl₂, 2mM CaCl₂, 0.1% gelatin, 0.05% Tween-20, pH 7.4) to 20%, and incubated at 37 °C for 2 hours. Wash the reaction well with PBST buffer to terminate the reaction. Rabbit anti human MAC antibody (Merck, 204903) was added as the capture antibody and sheep anti rabbit antibody (Life, 656120) was added as the detection antibody to the above-mentioned sample wells to detect MAC deposition in monkey plasma. Read the absorbance value at 450nm using the enzyme-linked immunosorbent assay (ELISA) reader. Inhibition rate=(OD450nm of samples at different time points after administration - OD450nm of D0 sample)/OD450nm of D0 sample.

### 9.2 Inhibition activity of bispecific antibodies in alternative pathways:

Similar to the experimental principle in Example 3.4, hemolysis assay was used to detect the biological activity of bispecific antibodies in alternative pathways.

Firstly, the monkey plasma collected at different time points was diluted to 40% using HBS-Mg²⁺-EGTA buffer (containing 5mM MgCl₂·6H₂O and 10mM EGTA). Fresh rabbit or guinea pig red blood cells was washed three times with HBSS buffer (containing 0.1% gelatin), and the washed red blood cells was diluted with HBS-Mg²⁺-EGTA buffer. 75µl diluted red blood cells and 75µl diluted monkey plasma was added to the reaction well, mix well, and incubate at 37 °C for 1 hour. After incubation, 80µl of pre cooled termination buffer (containing 10 mM EDTA, 1×HBSS buffer, pH 7.4) was added to each reaction well, and centrifuged at 4 °C, 1200g for 5 minutes. 100µl of supernatant was added to a 96-well plate to measure OD415. Inhibition rate=(OD415nm of samples at different time points after administration - OD415nm of D0 sample)/OD415nm of D0 sample.

The results showed that in cynomolgus monkeys, bispecific antibodies that specifically bind to MASP2 and MASP3 can inhibit both the lectin pathway and alternative pathway, thus simultaneously blocking the lectin pathway and alternative pathway in the complement pathway.

## Claims

1. An isolated antibody or antigen-binding fragment specifically binding to MASP3, comprising:
a heavy chain variable domain (V_{H}) comprising a heavy chain complementarity determining region (HC-CDR) 1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a light chain variable domain (V_{L}) comprising a light chain complementarity determining region (LC-CDR) 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

2. The isolated antibody or antigen-binding fragment specifically binding to MASP3 of claim 1, comprising:
(i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7; or
(ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

3. The isolated antibody or antigen-binding fragment specifically binding to MASP3 of any one of claims 1-2, comprising:
(i) a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 16, or a variant thereof having at least about 80% sequence identity to the amino acid sequence of SEQ ID NO: 16;
(ii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 17, or a variant thereof having at least about 80% sequence identity to the amino acid sequence of SEQ ID NO: 17;
(iii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 18, or a variant thereof having at least about 80% sequence identity to the amino acid sequence of SEQ ID NO: 18;
(iv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17;
(v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 18, or a variant thereof having at least about 80% sequence identity to the amino acid sequence of SEQ ID NO: 18;
(vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17;
(vii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 18, or a variant thereof having at least about 80% sequence identity to the amino acid sequence of SEQ ID NO: 18;
(viii)a V_{H} comprising the amino acid sequence of SEQ ID NO: 11, or a variant thereof having at least about 80% sequence identity to the amino acid sequence of SEQ ID NO: 11; and a V_{L} comprising the amino acid sequence of SEQ ID NO: 19, or a variant thereof having at least about 80% sequence identity to the amino acid sequence of SEQ ID NO: 19;
(ix) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 20, or a variant thereof having at least about 80% sequence identity to the amino acid sequence of SEQ ID NO: 20;
(x) a V_{H} comprising the amino acid sequence of SEQ ID NO: 15, or a variant thereof having at least about 80% sequence identity to the amino acid sequence of SEQ ID NO: 15; and a V_{L} comprising the amino acid sequence of SEQ ID NO: 20, or a variant thereof having at least about 80% sequence identity to the amino acid sequence of SEQ ID NO: 20;
(xi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20;
(xii) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21;
(xiii)a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 22, or a variant thereof having at least about 80% sequence identity to the amino acid sequence of SEQ ID NO: 22;
(xiv)a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21;
(xv) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 22, or a variant thereof having at least about 80% sequence identity to the amino acid sequence of SEQ ID NO: 22;
(xvi)a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 21, or a variant thereof having at least about 80% sequence identity to the amino acid sequence of SEQ ID NO: 21; or
(xvii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

4. The isolated antibody or antigen-binding fragment specifically binding to MASP3 of any one of claims 1-3, wherein the antibody or antigen-binding fragment comprises an Fc fragment.

5. The isolated antibody or antigen-binding fragment specifically binding to MASP3 of claim 4, wherein the antibody or antigen-binding fragment is a full-length IgG antibody.

6. The isolated antibody or antigen-binding fragment specifically binding to MASP3 of claim 5, wherein the antibody or antigen-binding fragment is a full-length IgG1, IgG2, IgG3 or IgG4 antibody.

7. The isolated antibody or antigen-binding fragment specifically binding to MASP3 of any one of claims 1-6, wherein the antibody or antigen-binding fragment is chimeric, humanized, or human antibody.

8. The isolated antibody or antigen-binding fragment specifically binding to MASP3 according to any one of claims 1-3, wherein the antigen-binding fragment is selected from the group consisting of Fab, Fab', F(ab)'2, Fab'-SH, single-chain Fv (scFv), Fv fragment, dAb, Fd, nanobody, diabody, and linear antibody.

9. A multi-specific antibody comprising a first antigen-binding domain specifically binding to MASP3, and a second antigen-binding domain specifically binding to MASP2, wherein the first antigen-binding domain comprises:
a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

10. The multi-specific antibody of claim 9, wherein the first antigen-binding domain comprises:
(i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7; or
(ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

11. The multi-specific antibody of any one of claims 9-10, wherein the second antigen-binding domain comprises:
a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT (SEQ ID NO: 33), wherein X₁ is N, Q or S, and X₂ is A, E or S.

12. The multi-specific antibody of any one of claims 9-11, wherein the second antigen-binding domain comprises:
(i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28;
(ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29;
(iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30;
(iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or
(v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

13. The multi-specific antibody of any one of claims 9-12, wherein the first antigen-binding domain comprises:
a V_{H} comprising an HC-CDR1 comprising the amino acid sequence SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8;
and wherein the second antigen-binding domain comprises:
a V_{H} comprising an HC-CDR1 comprising the amino acid sequence of SEQ ID NO: 23, an HC-CDR2 comprising the amino acid sequence of SEQ ID NO: 24, and an HC-CDR3 comprising the amino acid sequence of SEQ ID NO: 25; and a V_{L} comprising an LC-CDR1 comprising the amino acid sequence of SEQ ID NO: 26, an LC-CDR2 comprising the amino acid sequence of SEQ ID NO: 27, and an LC-CDR3 comprising the amino acid sequence of SEQ ID NO: 29.

14. The multi-specific antibody of any one of claims 9-13, wherein the first antigen-binding domain comprises:
(i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17;
(ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17;
(iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20;
(iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21;
(v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or
(vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

15. The multi-specific antibody of any one of claims 9-14, wherein the second antigen-binding domain comprises:
(i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36;
(ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
(iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38;
(iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39;
(v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or
(vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

16. The multi-specific antibody of any one of claims 9-15, wherein:
a) the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20; and wherein
the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
b) the first antigen-binding domain comprises: a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21; and wherein
the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
c) the first antigen-binding domain comprises: a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; and wherein
the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37; or
d) the first antigen-binding domain comprises: a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22; and wherein
the second antigen-binding domain comprises: a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37.

17. A multi-specific antibody comprising a first antigen-binding domain specifically binding to MASP3, and a second antigen-binding domain specifically binding to MASP2, wherein the second antigen-binding domain comprises:
a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT
(SEQ ID NO: 33), wherein X₁ is N, Q or S, and X₂ is A, E or S.

18. The multi-specific antibody of claim 17, wherein the second antigen-binding domain comprises:
(i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28;
(ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29;
(iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30;
(iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or
(v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

19. The multi-specific antibody of any one of claims 17-18, wherein the second antigen-binding domain comprises:
(i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36;
(ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
(iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38;
(iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39;
(v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or
(vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

20. The multi-specific antibody of any one of claims 9-19, wherein the multi-specific antibody has the formats selected from the group consisting of DVD-Ig, Bs4Ab, Hetero H, CrossMab, CrossMab2+1, IgG-(scFv)₂ and scFv-Fab IgG.

21. The multi-specific antibody of any one of claims 9-20, comprising four polypeptide chains:
wherein one of the polypeptide chains comprises V_{H}1-C_{H}1 structure from N- terminus to C-terminus, wherein V_{H}1 is a heavy chain variable domain specifically binding to MASP3; C_{H}1 is a heavy chain constant domain 1; wherein the polypeptide chain further comprises an Fc comprising C_{H}2 and C_{H}3 domains; and
one of the polypeptide chains comprises V_{L}1-C_{L} structure from N- terminus to C-terminus,
wherein V_{L}1 is a light chain variable domain specifically binding to MASP3, C_{L} is a light chain constant domain; and
one of the polypeptide chains comprises V_{H}2-C_{L} structure from N- terminus to C-terminus,
wherein V_{H}2 is a heavy chain variable domain specifically binding to MASP2; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus,
wherein V_{L}2 is a light chain variable domain specifically binding to MASP2, C_{H}1 is a heavy chain constant domain 1.

22. The multi-specific antibody of any one of claims 9-21, comprising four polypeptide chains:
wherein one of the polypeptide chains comprises V_{H}1-C_{H}1 structure from N- terminus to C-terminus, wherein V_{H}1 is a heavy chain variable domain specifically binding to MASP2; C_{H}1 is a heavy chain constant domain 1; wherein the polypeptide chain further comprises an Fc comprising C_{H}2 and C_{H}3 domains; and
one of the polypeptide chains comprises V_{L}1-C_{L} structure from N- terminus to C-terminus,
wherein V_{L}1 is a light chain variable domain specifically binding to MASP2, C_{L} is a light chain constant domain; and
one of the polypeptide chains comprises V_{H}2-C_{L} structure from N- terminus to C-terminus,
wherein V_{H}2 is a heavy chain variable domain specifically binding to MASP3; C_{L} is a light chain constant domain; wherein the polypeptide chain further comprises an Fc comprising C_{H}2 and C_{H}3 domains; and
one of the polypeptide chains comprises V_{L}2-C_{H}1 structure from N- terminus to C-terminus,
wherein V_{L}2 is a light chain variable domain specifically binding to MASP3, C_{H}1 is a heavy chain constant domain 1.

23. The multi-specific antibody of any one of claims 9-22, comprising:
a) the amino acid sequence of SEQ ID NO: 53, or a variant thereof having at least about 80% sequence identity to the amino acid sequence of SEQ ID NO: 53; and/or the amino acid sequence of SEQ ID NO: 54, or a variant thereof having at least about 80% sequence identity to the amino acid sequence of SEQ ID NO: 54; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52;
b) the amino acid sequence of SEQ ID NO: 55, or a variant thereof having at least about 80% sequence identity to the amino acid sequence of SEQ ID NO: 55; and/or the amino acid sequence of SEQ ID NO: 56, or a variant thereof having at least about 80% sequence identity to the amino acid sequence of SEQ ID NO: 56; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52;
c) the amino acid sequence of SEQ ID NO: 57, or a variant thereof having at least about 80% sequence identity to the amino acid sequence of SEQ ID NO: 57; and/or the amino acid sequence of SEQ ID NO: 56, or a variant thereof having at least about 80% sequence identity to the amino acid sequence of SEQ ID NO: 56; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52; or
d) the amino acid sequence of SEQ ID NO: 53, or a variant thereof having at least about 80% sequence identity to the amino acid sequence of SEQ ID NO: 53; and/or the amino acid sequence of SEQ ID NO: 58, or a variant thereof having at least about 80% sequence identity to the amino acid sequence of SEQ ID NO: 58; and/or the amino acid sequence of SEQ ID NO: 51, or a variant thereof having at least about 80% sequence identity to the amino acid sequence of SEQ ID NO: 51; and/or the amino acid sequence of SEQ ID NO: 52, or a variant thereof having at least about 80% sequence identity to the amino acid sequence of SEQ ID NO: 52.

24. The multi-specific antibody of any one of claims 9-23, comprising:
a) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 54, or a variant thereof having at least about 80% sequence identity to the amino acid sequence of SEQ ID NO: 54; and/or the amino acid sequence of SEQ ID NO: 60, or a variant thereof having at least about 80% sequence identity to the amino acid sequence of SEQ ID NO: 60;
b) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 56, or a variant thereof having at least about 80% sequence identity to the amino acid sequence of SEQ ID NO: 56; and/or the amino acid sequence of SEQ ID NO: 61, or a variant thereof having at least about 80% sequence identity to the amino acid sequence of SEQ ID NO: 61;
c) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 56, or a variant thereof having at least about 80% sequence identity to the amino acid sequence of SEQ ID NO: 56; and/or the amino acid sequence of SEQ ID NO: 62, or a variant thereof having at least about 80% sequence identity to the amino acid sequence of SEQ ID NO: 62; or
d) the amino acid sequence of SEQ ID NO: 52, or a variant thereof having at least about 80% sequence identity to the amino acid sequence of SEQ ID NO: 52; and/or the amino acid sequence of SEQ ID NO: 59, or a variant thereof having at least about 80% sequence identity to the amino acid sequence of SEQ ID NO: 59; and/or the amino acid sequence of SEQ ID NO: 58, or a variant thereof having at least about 80% sequence identity to the amino acid sequence of SEQ ID NO: 58; and/or the amino acid sequence of SEQ ID NO: 60, or a variant thereof having at least about 80% sequence identity to the amino acid sequence of SEQ ID NO: 60.

25. An isolated nucleic acid molecule that encodes the antibody or antigen-binding fragment specifically binding to MASP3 according to any one of claims 1-8 or the multi-specific antibody according to any one of claims 9-24.

26. A vector comprising the nucleic acid molecule of claim 25.

27. An isolated host cell comprising the antibody or antigen-binding fragment specifically binding to MASP3 according to any one of claims 1-8, or the multi-specific antibody according to any one of claims 9-24, the nucleic acid molecule of claim 25, or the vector of claim 26.

28. A method of producing the antibody or antigen-binding fragment specifically binding to MASP3 according to any one of claims 1-8 or the multi-specific antibody according to any one of claims 9-24, comprising:
a) culturing the host cell of claim 27 under conditions effective to express antibody; and
b) obtaining the expressed antibody from the host cell.

29. A pharmaceutical composition comprising the antibody or antigen-binding fragment specifically binding to MASP3 according to any one of claims 1-8, the multi-specific antibody according to any one of claims 9-24, the nucleic acid molecule of claim 25, the vector of claim 26, the isolated host cell of claim 27, or the antibody produced according to claim 28 and a pharmaceutically acceptable carrier or adjunct.

30. A method of treating and/or preventing a disease or condition in an individual in need thereof, comprising administering to the individual an effective amount of the antibody or antigen-binding fragment specifically binding to MASP3 according to any one of claims 1-8, the multi-specific antibody according to any one of claims 9-24, the nucleic acid molecule of claim 25, the vector of claim 26, the isolated host cell of claim 27, the antibody produced according to claim 28, or the pharmaceutical composition of claim 29.

31. A pharmaceutical composition comprising an antibody or antigen-binding fragment specifically binding to MASP3 and an antibody or antigen-binding fragment specifically binding to MASP2, wherein the antibody or antigen-binding fragment specifically binding to MASP3 comprises:
a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

32. A method of treating and/or preventing a disease or condition in an individual in need thereof, comprising administering to the individual an effective amount of an antibody or antigen-binding fragment specifically binding to MASP3 and an antibody or antigen-binding fragment specifically binding to MASP2, or a pharmaceutical composition comprising an antibody or antigen-binding fragment specifically binding to MASP3 and an antibody or antigen-binding fragment specifically binding to MASP2, wherein the antibody or antigen-binding fragment specifically binding to MASP3 comprises:
a V_{H} comprising an HC-CDR1 comprising GKWIE (SEQ ID NO: 1); an HC-CDR2 comprising EILPGSGSTDYNERFKD (SEQ ID NO: 2); and an HC-CDR3 comprising SEDL (SEQ ID NO: 3); and a V_{L} comprising an LC-CDR 1 comprising KSSQX₁LX₂NSX₃TRKX₄YLA (SEQ ID NO: 9), wherein X₁ is N or S, X₂ is F or L, X₃ is R or V, and X₄ is N or T; an LC-CDR2 comprising WASTRES (SEQ ID NO: 6), and an LC-CDR3 comprising KQSYX₁X₂X₃T (SEQ ID NO: 10), wherein X₁ is I or T, X₂ is L or P, and X₃ is F or P.

33. The pharmaceutical composition of claim 31 or the method of claim 32, wherein the antibody or antigen-binding fragment specifically binding to MASP3 comprises:
(i) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 4, an LC-CDR2 comprising the amino acid sequence of SEQ ID NO: 6, and an LC-CDR3 comprising the amino acid sequence of SEQ ID NO: 7; or
(ii) a V_{H} comprising an HC-CDR1 comprising the amino acid sequence of SEQ ID NO: 1, an HC-CDR2 comprising the amino acid sequence of SEQ ID NO: 2, and an HC-CDR3 comprising the amino acid sequence of SEQ ID NO: 3; and a V_{L} comprising an LC-CDR1 comprising the amino acid sequence of SEQ ID NO: 5, an LC-CDR2 comprising the amino acid sequence of SEQ ID NO: 6, and an LC-CDR3 comprising the amino acid sequence of SEQ ID NO: 8.

34. The pharmaceutical composition or the method of claim 33, wherein the antibody or antigen-binding fragment specifically binding to MASP3 comprises:
(i) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 17, or a variant thereof having at least about 80% sequence identity to the amino acid sequence of SEQ ID NO: 17;
(ii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 17, or a variant thereof having at least about 80% sequence identity to the amino acid sequence of SEQ ID NO: 17;
(iii) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 20, or a variant thereof having at least about 80% sequence identity to the amino acid sequence of SEQ ID NO: 20;
(iv) a V_{H} comprising the amino acid sequence of SEQ ID NO: 12, or a variant thereof having at least about 80% sequence identity to the amino acid sequence of SEQ ID NO: 12; and a V_{L} comprising the amino acid sequence of SEQ ID NO: 21, or a variant thereof having at least about 80% sequence identity to the amino acid sequence of SEQ ID NO: 21;
(v) a V_{H} comprising the amino acid sequence of SEQ ID NO: 13, or a variant thereof having at least about 80% sequence identity to the amino acid sequence of SEQ ID NO: 13; and a V_{L} comprising the amino acid sequence of SEQ ID NO: 21, or a variant thereof having at least about 80% sequence identity to the amino acid sequence of SEQ ID NO: 21; or
(vi) a V_{H} comprising the amino acid sequence of SEQ ID NO: 14, or a variant thereof having at least about 80% sequence identity to the amino acid sequence of SEQ ID NO: 14; and a V_{L} comprising the amino acid sequence of SEQ ID NO: 22, or a variant thereof having at least about 80% sequence identity to the amino acid sequence of SEQ ID NO: 22.

35. The pharmaceutical composition of any one of claims 31, 33-34, or the method of any one of claims 32-34, wherein the antibody or antigen-binding fragment specifically binding to MASP2 comprises:
a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT
(SEQ ID NO: 33), wherein X₁ is N, Q or S, and X₂ is A, E or S.

36. The pharmaceutical composition or the method of claim 35, wherein the antibody or antigen-binding fragment specifically binding to MASP2 comprises:
(i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28;
(ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29;
(iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30;
(iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or
(v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

37. The pharmaceutical composition or the method of claim 36, wherein the antibody or antigen-binding fragment specifically binding to MASP2 comprises:
(i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36;
(ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
(iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38;
(iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39;
(v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or
(vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

38. A pharmaceutical composition comprising an antibody or antigen-binding fragment specifically binding to MASP3 and an antibody or antigen-binding fragment specifically binding to MASP2, wherein the antibody or antigen-binding fragment specifically binding to MASP2 comprises:
a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT
(SEQ ID NO: 33), wherein X₁ is N, Q or S, and X₂ is A, E or S.

39. A method of treating and/or preventing a disease or condition in an individual in need thereof, comprising administering to the individual an effective amount of an antibody or antigen-binding fragment specifically binding to MASP3 and an antibody or antigen-binding fragment specifically binding to MASP2, or a pharmaceutical composition comprising an antibody or antigen-binding fragment specifically binding to MASP3 and an antibody or antigen-binding fragment specifically binding to MASP2, wherein the antibody or antigen-binding fragment specifically binding to MASP2 comprises:
a V_{H} comprising an HC-CDR1 comprising SDYAWN (SEQ ID NO: 23); an HC-CDR2 comprising YISYSGRTSYNPSLKS (SEQ ID NO: 24); and an HC-CDR3 comprising HYGDY (SEQ ID NO: 25); and a V_{L} comprising an LC-CDR 1 comprising KASQNVGTNVA (SEQ ID NO: 26); an LC-CDR2 comprising SASYRYS (SEQ ID NO: 27), and an LC-CDR3 comprising HQYX₁X₂NPLT
(SEQ ID NO: 33), wherein X₁ is N, Q or S, and X₂ is A, E or S.

40. The pharmaceutical composition of claim 38 or the method of claim 39, wherein the antibody or antigen-binding fragment specifically binding to MASP2 comprises:
(i) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 28;
(ii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 29;
(iii) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 30;
(iv) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 31; or
(v) a V_{H} comprising an HC-CDR1 comprising the amino acid sequence of SEQ ID NO: 23; an HC-CDR2 comprising the amino acid sequence of SEQ ID NO: 24; and an HC-CDR3 comprising the amino acid sequence of SEQ ID NO: 25; and a V_{L} comprising an LC-CDR1 comprising the amino acid sequence of SEQ ID NO: 26; an LC-CDR2 comprising the amino acid sequence of SEQ ID NO: 27; and an LC-CDR3 comprising the amino acid sequence of SEQ ID NO: 32.

41. The pharmaceutical composition or the method of claim 40, wherein the antibody or antigen-binding fragment specifically binding to MASP2 comprises:
(i) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 36, or a variant thereof having at least about 80% sequence identity to the amino acid sequence of SEQ ID NO: 36;
(ii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 37, or a variant thereof having at least about 80% sequence identity to the amino acid sequence of SEQ ID NO: 37;
(iii) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 38, or a variant thereof having at least about 80% sequence identity to the amino acid sequence of SEQ ID NO: 38;
(iv) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 39, or a variant thereof having at least about 80% sequence identity to the amino acid sequence of SEQ ID NO: 39;
(v) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 40, or a variant thereof having at least about 80% sequence identity to the amino acid sequence of SEQ ID NO: 40; or
(vi) a V_{H} comprising the amino acid sequence of SEQ ID NO: 34, or a variant thereof having at least about 80% sequence identity to the amino acid sequence of SEQ ID NO: 34; and a V_{L} comprising the amino acid sequence of SEQ ID NO: 41, or a variant thereof having at least about 80% sequence identity to the amino acid sequence of SEQ ID NO: 41.

42. The method of any one of claims 32-37, and 39-41, wherein the antibody or antigen-binding fragment specifically binding to MASP3 and the antibody or antigen-binding fragment specifically binding to MASP2 are administered to the individual concurrently or administered to the individual consecutively.

43. The method of any one of claims 30, 32-37, and 39-41, wherein the disease or condition is autoimmune disease, transplantation-related disease, inflammatory disease, hemopathy, coagulation disease, angiogenesis-dependent diseases and/or viral infectious disease associated with complement dysregulation.

44. The method of claim 43, wherein the disease or condition is selected from the group consisting of ischemia reperfusion injury, atherosclerosis, mesangial proliferative glomerulonephritis, membranous glomerulonephritis, membranoproliferative glomerulonephritis, acute post infection glomerulonephritis, cryoglobulinemic glomerulonephritis, lupus nephritis, systemic lupus erythematosus (SLE), Henoch Schonlein purpura nephritis, IgA nephropathy, ischemic shock, hemolytic anemia, autoimmune thrombotic thrombocytopenic purpura (TTP), hemolytic uremic syndrome (HUS), atypical hemolytic uremic syndrome (aHUS), paroxysmal nocturnal hemoglobinuria (PNH), transplant-associated TMA, Upshaw-Schulman syndrome, arthritis, traumatic brain injury, aspiration pneumonia, neuromyelitis optica, multiple sclerosis, amyotrophic lateral sclerosis (ALS), chronic obstructive pulmonary disease (COPD), C3 glomerulopathy, transplant rejection, graft-versus-host disease (GVHD), sepsis, systemic inflammatory response syndrome (SIRS) Acute respiratory distress syndrome (ARDS), ANCA vasculitis, antiphospholipid syndrome, myasthenia gravis, Degos disease, disseminated intravascular coagulation (DIC), Angiogenesis dependent cancer, age-related macular degeneration, retinopathy, proliferative diabetes, retinopathy secondary vitreous hemorrhage, neovascular glaucoma, corneal neovascularization, retinopathy of prematurity and respiratory distress syndrome or pneumonia caused by coronavirus infection.
